(19) Europäisches Patentamt
European Patent Office
Office européen des brevets

(11) EP 1 479 682 A1

(12) **EUROPEAN PATENT APPLICATION**

(43) Date of publication:
**24.11.2004 Bulletin 2004/48**

(51) Int Cl.[7]: **C07D 475/00**, C07D 475/02, A61K 31/519

(21) Application number: **03079183.4**

(22) Date of filing: **24.12.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **23.05.2003 US 444158**

(71) Applicant: **4 AZA Bioscience nv**
**3000 Leuven (BE)**

(72) Inventors:
- **Waer, Mark Jozef Albert**
**3001 Heverlee (BE)**
- **Herdewijn, Piet André Maurits Maria**
**3111 Rotselaar/Wezemaal (BE)**
- **Pfleiderer, Wolfgang Eugen**
**78464 Konstanz (DE)**

(74) Representative: **Bird, William Edward et al**
**Bird Goen & Co.,**
**Klein Dalenstraat 42A**
**3020 Winksele (BE)**

(54) **Immunosuppressive effects of pteridine derivatives**

(57) Novel poly-substituted pteridinediones (lumazines), and mono- or polysubstituted 2-thiolumazines, 4-thiolumazines or 2,4-dithiolumazines, having disclosed substituents in positions 1, 3, 6 and 7 of the pteridine ring, and pharmaceutically acceptable salts thereof, are useful as biologically active ingredients in preparing pharmaceutical compositions especially for the treatment or prevention of a CNS disorder, a cell proliferative disorder, a viral infection, an immune or auto-immune disorder or a transplant rejection. Combinations of the pteridine derivatives of the invention with an immunosuppressant or immunomodulator drug, an antineoplastic drug or an antiviral agent, providing potential synergistic effects, are also disclosed.

EP 1 479 682 A1

## Description

The invention relates to a class of novel poly-substituted pteridine-2,4-diones (lumazines), as well as novel mono- and polysubstituted 2-thiolumazines, 4-thiolumazines and 2,4-dithiolumazines. The invention further relates to pharmaceutical compositions including a broad class of poly-substituted pteridine-2,4-diones (lumazines), as well as mono- and polysubstituted 2-thiolumazines, 4-thiolumazines and 2,4-dithiolumazines especially for the prevention and/or the treatment of pathologic conditions such as, but not limited to, immune and autoimmune disorders, organ and cells transplant rejections, cell proliferative disorders, cardiovascular disorders, disorders of the central nervous system and viral diseases.

The invention further relates to combined pharmaceutical preparations comprising one or more polysubstituted pteridine-2,4-diones (lumazines), as well as mono- and polysubstituted 2-thiolumazines, 4-thiolumazines and 2,4-dithiolumazines and one or more known immunosuppressant drugs or antineoplastic drugs or anti-viral drugs.

This invention also relates to a method for the prevention and/or treatment of pathologic conditions such as, but not limited to, immune and autoimmune disorders, organ and cells transplant rejections, cell proliferative disorders, cardiovascular disorders, disorders of the central nervous system and viral diseases by the administration of an effective amount of a polysubstituted pteridine-2,4-dione (lumazine), or a mono- or polysubstituted 2-thiolumazine, 4-thiolumazine or 2,4-dithio-lumazine optionally combined with one or more known immunosuppressant drugs or antineoplastic drugs or anti-viral drugs. Finally the invention relates to a method for selecting or classifying biologically active polysubstituted pteridine-2,4-diones (lumazines), as well as mono- and polysubstituted 2-thiolumazines, 4-thiolumazines and 2,4-dithiolumazines based on the determination of two or more *in vitro* tests such as TNF-$\alpha$ and IL-1 $\beta$ assays.

## BACKGROUND OF THE INVENTION

2,4-dioxo-1,2,3,4-tetrahydropteridine is well known in the art under the name lumazine. Gabriel and Sonn first disclosed in *Ber. Deut. Chem. Ges.* (1907) 40:4850 making lumazine from pyrazin-bicarboxamide. Timmis in *Nature* (1949) 164:139 disclosed the synthesis of 1,3-dimethyl-6-phenyllumazine and 1,3-dimethyl-7-phenyl-lumazine by condensing a 6-amino-5-nitroso-pyrimidine with benzaldehyde or methylphenylketone respectively. Zondler et al. in *J. Heterocyclic Chem.* (1967) 4:124 and Taylor et al. in *Heterocycles* (1978) 10:37 disclosed 1,3,6-trimethyllumazine and 1,3-dimethyl-6-ethyllumazine. Yoneda and Higuchi in *J. Chem. Soc. Perkin* (1977) 1336 disclosed the preparation of various 1,3-dimethyl-6-aryllumazines starting from 6-amino-1,3-dimethyl-5-aryliden-aminouracil. Kang et al. in *J. Heterocycl. Chem.* (1987) 24:597-601 disclosed reacting 5,6-diamino-1,3-dimethyluracil either with propanetrione-1,3-dioxime followed by cyclization to form 1,3-dimethyllumazine-6-carboxaldoxime, or with oximinoacetone followed by cyclization to form 1,3,6-trimethyllumazine, or else with methylglyoxal to form 1,3,7-trimethyllumazine. Both latter compounds may easily, through acid hydrolysis in the presence of formaldehyde, be converted into the corresponding 1,3-dimethyllumazine-carboxaldehydes which, due to their high carbonyl reactivity, may in turn be converted into other lumazine derivatives. Blicke et al. in *J.A.C.S* (1954) 76:2798-2800 disclosed 1,3-dimethyl-7-aminolumazine, 1,3,6,7-tetramethyllumazine, 1,3-dimethyl-6,7-dihydroxy-lumazine and 1,3-dimethyl-6,7-diphenylumazine; Pfleiderer in *Chem. Ber.* (1957) 90:2588 disclosed 1,3-dimethyl-6-hydroxylumazine and 1,3-dimethyl-7-hydroxylumazine; Pfleiderer et al. in *Chem. Ber.* (1973) 106:3149-3174 disclosed 1,3-dimethyl-6-hydroxy-7-phenyllumazine, 1,3-dimethyl-6-phenyl-7-hydroxy-lumazine and 1,3-dimethyl-6,7-diisopropylumazine; Hutzenlaub et al. in *Chem. Ber.* (1973) 106:3203-3215 disclosed 1,3-dimethyl-7-methoxylumazine, 1,3,6-trimethyl-7-hydroxy-lumazine and 1,3,6-trimethyl-7-methoxylumazine; Steppan et al. in *Liebigs Ann. Chem.* (1982) 2135-2145 disclosed 1,3-dimethyl-6-aminolumazine, 1,3-dimethyl-6-chlorolumazine, 1,3-dimethyl-7-chlorolumazine and 1,3-dimethyl-7-methylaminolumazine; Kasimierczuk et al. in *Chem. Ber.* (1979) 112:1499-1513 disclosed 1,3-dimethyl-7-mercaptolumazine and 1,3-dimethyl-7-methylthio-lumazine as well as a few substituted 2- or 4-thiolumazines and 2,4-dithiolumazines, starting from substituted 6-amino-2-thiouracil or 6-amino-2,4-dithiouracil; Eisele et al. in *Pteridines* (1993) 4:178-186 disclosed 1,3,6-trimethyllumazine-7-carboxylic acid and its methyl and ethyl esters. Perez-Rubalcaba et al. in *Liebigs Ann. Chem.* (1983) 852-859 disclosed substituted 3-methyllumazines wherein one of the 6- and 7-substituents is phenyl whereas the other is chloro. Finally, Weisenfeldt (1987) disclosed a series of tetra-substituted lumazines wherein the 1- and 3-substituents are methyl and one of the 6- and 7-substituents is chloro. Further, Fink et al. in *Chem. Berichte* (1963) 96:2950-2963, as well as Pfleiderer, Perez-Rubalcaba and Eisele (all cited *supra)* disclosed bi- and tri-substituted lumazines wherein only one of the 1- and 3-nitrogen atoms is substituted. Interestingly, none of the above-cited substituted lumazines, 2-thiolumazines and 2,4-dithiolumazines was ever said to have any kind of biological activity.

A few other substituted pteridine-2,4-diones (lumazines) are already known in the art as being useful in the preparation of medicines. For instance, U.S. Patent No. 3,071,587 teaches cyanoethylpteridinediones having central nervous system (hereinafter referred as CNS) activity and anti-depressant properties. WO 94/06431 teaches a 1-methyl-3-(10,11-epoxyundecyl) pteridinedione being able to inhibit IL-1 receptors, decrease proliferation of tumor & other

cells, stimulate hematopoeisis, suppress T-cell activation, secretion of antibodies by B-cells and activation of macrophage or endothelial cells by endotoxins, lumor necrosis factor (hereinafter TNF), IL-1 or GM-CSF and enhance resistance of mesenchymal cells to TNF. WO 94/11001 teaches 1-methyl-3-(hydroxy- and dihydroxy-$C_{9-25}$ alkyl) pteridinediones being able to inhibit lysophosphatidic acid transferase as well as immune or cellular response to stimuli, and therefore can be used to treat tumor progression or invasion, autoimmune diseases, acute allergic reactions mediated by TNF or IL-1, rheumatoid arthritis, osteoarthritis, multiple sclerosis, diabetes, atherosclerosis, restenosis, stroke, HIV infection, inflammatory response, septic shock, CNS and bone diseases. Cottam et al. in *J. Med. Chem.* (1996) 39 : 2-9 and WO 98/52948 both disclose a 1-methyl-3-n-hexyl-6-carboxymethyl-7-carboxymethyl pteridinedione which, although included in a biological evaluation study of inhibitors of TNF-$\alpha$, was not tested for TNF-$\alpha$ activity. WO 96/20710 teaches substituted pteridinediones which inhibit cellular responses to ceramide metabolites of the sphingomyelin signal transduction pathway, inhibit inflammatory response associated with TNF-$\alpha$ and fibroblast proliferation or UV-induced cutaneous immune suppression and therefore can be used to treat cirrhosis, cell senescence and apoptosis.

WO 00/45800 discloses the immunosuppressive effects of pharmaceutical compositions for the treatment of autoimmuno disorders and/or for the treatment or prevention of transplant rejections comprising a pteridine derivative of general formula:

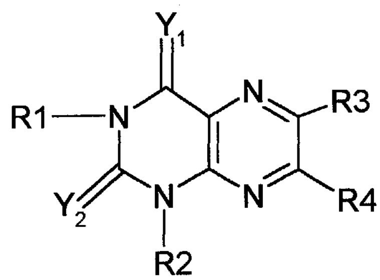

wherein:

$R_1$ and $R_2$ are independently hydrogen; aliphatic saturated or unsaturated, straight or branched carbon chains with 1 to 7 carbon atoms; substituted or unsubstituted aryl or alkylaryl substituents, whereby the carbon atoms may be oxidized represented by alcohol or carbonyl function or carboxylic acids and their esters;

$R_3$ and $R_4$ are independently hydrogen, hydroxyl, halogen, alkyl, haloalkyl, alkoxy, wherein the alkyl group may be branched or straight and contains one or four carbon atoms, formyl and derivatives such as hydroxylamino conjugates and acetals, cyano, carboxylic acids and carboxyl acid derivatives such as esters and amides, sulfhydryl, amino, alkylamino, cycloalkylamino, alkenylamino, alkynylamino, benzylamino, hydroxylalkylamino, morpholinoalkylamino, phenylhydrazino, morpholino, piperidino, mercaptobenzyl, mercaptoalkyl, cysteinyl ester, styryl, aromatic ring; aromatic or heterocyclic substituent substituted with an aliphatic spacer between the pteridine ring and the aromatic substituent of 1 to 4 carbon atoms, whereby said spacer may contain an alcohol function, carbonyl function, halogen, ether, and may be saturated or unsaturated; branched or straight, saturated or unsaturated aliphatic chain of 1 to 7 carbon atoms which may contain one or more functions chosen from the group comprising carbonyl, alcohol, ether, carboxyester nitro, thioalkyl, halogen; and

$Y_1$ and $Y_2$ are both oxygen, or a pharmaceutical salt thereof, and a pharmaceutically acceptable carrier.

WO 00/45800 also discloses a compound, 1,3-dimethyl-6-benzoyl-7-(4-methoxyphenyl)lumazine, which has poor results in a Mixed Lymphocyte Reaction test as well as in CD3 and CD 28 assays.

WO 03/067257 discloses making 3-methyl-6-iminouracil in a three-steps procedure starting from O-methylisourea hydrochloride and methylcyanoacetate with a combined yield of 29%, followed by conversion into 5,6-diamino-3-methyluracil. C. Müller et al. in *J. Med. Chem.* (2002) 45:3440-3450 discloses 5,6-diamino-1-benzyluracil and a procedure for making it.

Nevertheless, there still is a need in the art for specific and highly therapeutically active compounds, such as, but not limited to, drugs for treating immune and autoimmune disorders, organ and cells transplant rejections, cell proliferative disorders, cardiovascular disorders, disorders of the central nervous system and viral diseases. In particular, there is a need in the art to provide immunosuppressive compounds or antineoplastic drugs or anti-viral drugs which are active in a minor dose in order to replace existing drugs having significant side effects and to decrease treatment costs.

Currently used immunosuppressive drugs include antiproliferative agents, such as methotrexate, azathioprine, and cyclophosphamide. Since these drugs affect mitosis and cell division, they have severe toxic effects on normal cells with high turn-over rate such as bone marrow cells and the gastrointestinal tract lining. Accordingly, marrow depression and liver damage are common side effects.

Anti-inflammatory compounds used to induce immunosuppression include adrenocortical steroids such as dexamethasone and prednisolone. The common side effects observed with the use of these compounds are frequent infections, abnormal metabolism, hypertension, and diabetes.

Other immunosuppressive compounds currently used to inhibit lymphocyte activation and subsequent proliferation include cyclosporine, tacrolimus and rapamycin. Cyclosporine and its relatives are among the most commonly used immunosuppressant drugs. Cyclosporine is typically used for preventing or treating organ rejection in kidney, liver, heart, pancreas, bone marrow, and heart-lung transplants, as well as for the treatment of autoimmune and inflammatory diseases such as Crohn's disease, aplastic anemia, multiple sclerosis, myasthenia gravis, uveitis, biliary cirrhosis, etc. However, cyclosporines suffer from a small therapeutic dose window and severe toxic effects including nephrotoxicity, hepatotoxicity, hypertension, hirsutism, cancer, and neurotoxicity. Another such drug, mitoxantrone, is known to induce heart and kidney toxicity.

Additionally, monoclonal antibodies with immunosuppressant properties, such as OKT3, have been used to prevent and/or treat graft rejection. Introduction of such monoclonal antibodies into a patient, as with many biological materials, induces several side-effects, such as dyspnea. Within the context of many life-threatening diseases, organ transplantation is considered a standard treatment and, in many cases, the only alternative to death. The immune response to foreign cell surface antigens on the graft, encoded by the major histo-compatibility complex (hereinafter referred as MHC) and present on all cells, generally precludes successful transplantation of tissues and organs unless the transplant tissues come from a compatible donor and the normal immune response is suppressed. Other than identical twins, the best compatibility and thus, long term rates of engraftment, are achieved using MHC identical sibling donors or MHC identical unrelated cadaver donors. However, such ideal matches are difficult to achieve. Further, with the increasing need of donor organs an increasing shortage of transplanted organs currently exists. Accordingly, xenotransplantation has emerged as an area of intensive study, but faces many hurdles with regard to rejection within the recipient organism.

The host response to an organ allograft involves a complex series of cellular interactions among T and B lymphocytes as well as macrophages or dendritic cells that recognize and are activated by foreign antigen. Costimulatory factors, primarily cytokines, and specific cell-cell interactions, provided by activated accessory cells such as macrophages or dendritic cells are essential for T-cell proliferation. These macrophages and dendritic cells either directly adhere to T-cells through specific adhesion proteins or secrete cytokines that stimulate T-cells, such as IL-12 and IL-15. Accessory cell-derived co-stimulatory signals stimulate activation of interleukin-2 (IL-2) gene transcription and expression of high affinity IL-2 receptors in T-cells. IL-2 is secreted by T lymphocytes upon antigen stimulation and is required for normal immune responsiveness. IL-2 stimulates lymphoid cells to proliferate and differentiate by binding to IL-2 specific cell surface receptors (IL-2R). IL-2 also initiates helper T-cell activation of cytotoxic T-cells and stimulates secretion of interferon-$\gamma$ which in turn activates cytodestructive properties of macrophages. Furthermore, IFN-$\gamma$ and IL-4 are also important activators of MHC class II expression in the transplanted organ, thereby further expanding the rejection cascade by enhancing the immunogenicity of the grafted organ The current model of a T-cell mediated response suggests that T-cells are primed in the T-cell zone of secondary lymphoid organs, primarily by dendritic cells. The initial interaction requires cell to cell contact between antigen-loaded MHC molecules on antigen-presenting cells (hereinafter referred as APC) and the T-cell receptor/CD3 complex on T-cells. Engagement of the TCR/CD3 complex induces CD154 expression predominantly on CD4 T-cells that in turn activate the APC through CD40 engagement, leading to improved antigen presentation. This is caused partly by upregulation of CD80 and CD86 expression on the APC, both of which are ligands for the important CD28 co-stimulatory molecule on T-cells. However, engagement of CD40 also leads to prolonged surface expression of MHC-antigen complexes, expression of ligands for 4-1 BB and OX-40 (potent co-stimulatory molecules expressed on activated T-cells). Furthermore, CD40 engagement leads to secretion of various cytokines (e.g., IL-12, IL-15, TNF-$\alpha$, IL-1, IL-6, and IL-8) and chemokines, all of which have important effects on both APC and T-cell activation and maturation. Similar mechanisms are involved in the development of auto-immune disease, such as type I diabetes. In humans and non-obese diabetic mice, insulin-dependent diabetes mellitus results from a spontaneous T-cell dependent auto-immune destruction of insulin-producing pancreatic .beta. cells that intensifies with age. The process is preceded by infiltration of the islets with mononuclear cells (insulitis), primarily composed of T lymphocytes. A delicate balance between auto-aggressive T-cells and suppressor-type immune phenomena determine whether expression of auto-immunity is limited to insulitis or not. Therapeutic strategies that target T-cells have been successful in preventing further progress of the auto-immune disease. These include neonatal thymectomy, administration of cyclosporine, and infusion of anti-pan T-cell, anti-CD4, or anti-CD25 (IL-2R) monoclonal antibodies. The aim of all rejection prevention and auto-immunity reversal strategies is to suppress the patient's immune reactivity to the antigenic tissue or agent, with a minimum of morbidity and mortality. Accordingly, a number of drugs are currently being used or investigated for their immunosuppressive properties. As

discussed above, the most commonly used immunosuppressant is cyclosporine, which however has numerous side effects. Accordingly, in view of the relatively few choices for agents effective at immunosuppression with low toxicity profiles and manageable side effects, there exists a need in the art for identification of alternative immunosuppressive agents and for agents acting as complement to calcineurin inhibition.

There is also a need in the art to improve therapeutic efficiency by providing pharmaceutical compositions or combined preparations exhibiting a synergistic effect as a result of combining two or more immunosuppressant drugs, or antineoplastic drugs or anti-viral drugs.

SUMMARY OF THE INVENTION

In a first embodiment, the present invention relates to a group of novel poly-substituted pteridine-2,4-diones (lumazines), as well as novel mono- and poly-substituted 2-thiolumazines, 4-thiolumazines and 2,4-dithiolumazines and

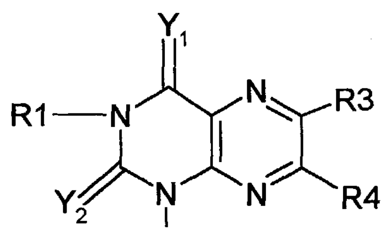

their pharmaceutically acceptable salts and enantiomers. These compounds may be represented by the general formula (I):
wherein:

a) if $Y_1$ and $Y_2$ are both oxygen and $R_4$ is hydrogen, then:

- $R_1$ is a radical selected from the group consisting of hydrogen; $C_{1-5}$ alkyl; $C_{2-7}$ alkenyl; aryl; alkylaryl; ω-hydroxy $C_{1-7}$ alkyl; ω-epoxy $C_{1-7}$ alkyl; ω-carboxy $C_{1-7}$ alkyl (wherein the carboxy group may be acid, ester, thioester, acid halide or amide); ω-cyano $C_{1-7}$ alkyl; arylalkyl; arylalkenyl; heterocyclic-substituted alkyl; heterocyclic-substituted alkenyl; groups having the formula -S-R (i.e.

wherein a sulfur atom is attached to the nitrogen atom of the pteridine ring)
wherein R is a monovalent group selected from the group consisting of $C_{1-7}$ alkyl, aryl and $C_{3-10}$ cycloalkyl and wherein the said monovalent group is optionally substituted with one or more substituents selected from the group consisting of amino, amino-acid, alkylamino, arylamino, cycloalkylamino, carboxylic acid, carboxylic ester, sulfonic acid and phosphonic acid; and optionally substituted heterocyclic radicals preferably selected from the group consisting of diazepinyl, oxadiazinyl, thiadiazinyl, dithiazinyl, triazolonyl, diazepinonyl, triazepinyl, triazepinonyl, tetrazepinonyl, benzoquinolinyl, benzothiazinyl, benzothiazinonyl, benzoxathiinyl, benzodioxinyl, benzodithiinyl, benzoxazepinyl, benzothiazepinyl, benzodiazepinyl, benzodioxepinyl, benzodithiepinyl, benzoxazocinyl, benzothiazocinyl, benzodiazocinyl, benzoxathiocinyl, benzodioxocinyl, benzotrioxepinyl, benzoxathiazepinyl, benzoxadiazepinyl, benzothiadiazepinyl, benzotriazepinyl, benzoxathiepinyl, benzotriazinonyl, benzoxazolinonyl, azetidinonyl, azaspiroundecyl, dithiaspirodecyl, selenazinyl, selenazolyl, selenophenyl, hypoxanthinyl, azahypoxanthinyl, bipyrazinyl, bipyridinyl, oxazolidinyl, diselenopyrimidinyl, benzodioxocinyl, benzopyrenyl, benzopyranonyl, benzophenazinyl, benzoquinolizinyl, dibenzocarbazolyl, dibenzoacridinyl, dibenzophenazinyl, dibenzothiepinyl, dibenzooxepinyl, dibenzopyranonyl, dibenzoquinoxalinyl, dibenzothiazepinyl, dibenzoisoquinolinyl, tetraazaadamantyl, thiatetraazaadamantyl, oxauracil, oxazinyl, dibenzothiophenyl, dibenzofuranyl, oxazolinyl, oxazolonyl, azaindolyl, azolonyl, thiazolinyl, thiazolonyl, thiazolidinyl, thiazanyl, pyrimidonyl, thiopyrimidonyl, thiamorpholinyl, azlactonyl, naphtindazolyl, naphtindolyl, naphtothiazolyl, naphtothioxolyl, naphtoxindolyl, naphtotriazolyl, naphtopyranyl, indolinyl, indolizidinyl, oxabicycloheptyl, azabenzimidazolyl, azacycloheptyl, azacyclooctyl, azacyclononyl, azabicyclononyl, tetrahydropyranyl, tetrahydropyronyl, tetrahydroquinoleinyl, tetrahydrothienyl and dioxide thereof, dihydrothienyl dioxide, dioxindolyl, dioxinyl, dioxenyl, dioxazinyl, thioxanyl, thioxolyl, thio-urazolyl, thiotriazolyl, thiopyranyl, thiopyronyl, coumarinyl, quinoleinyl, oxyquinoleinyl, quinuclidinyl, xanthinyl, dihydropyranyl, benzodihydrofuryl, benzothiopyronyl, benzothiopyranyl, benzoxazinyl, benzoxazolyl, benzodioxolyl, benzodioxanyl, benzothiadiazolyl, benzotriazinyl, benzothiazolyl, benzoxazolyl, phenothioxinyl, phenothiazolyl, phenothienyl, phenopyronyl, phenoxazolyl, pyridinyl, dihydropyridinyl, tetrahydropyridinyl, piperidinyl, morpholinyl, thiomorpholinyl, pyrazinyl, pyrimidinyl, pyridazinyl, triazinyl, tetrazinyl, triazolyl, benzotriazolyl, tetrazolyl, imidazolyl, pyrazolyl, thiazolyl, thiadiazolyl, isothiazolyl, oxazolyl, oxadiazolyl, pyrrolyl, furyl, dihydrofuryl, furoyl, hydantoinyl, dioxolanyl, dioxolyl, dithianyl, dithienyl, dithiinyl, thienyl, indolyl, indazolyl, benzofuryl, benzothienyl, quinolyl, quinazolinyl, quinoxalinyl, carbazolyl, phenoxazinyl, phenothiazinyl, xanthenyl, purinyl, benzothienyl, naphtothienyl, thianthrenyl, pyranyl, pyronyl, benzopyronyl, isobenzofuranyl, chromenyl, phenoxathiinyl, indolizinyl, quinolizinyl, isoquinolyl, phthalazinyl, naphthiridinyl, cinnolinyl, pteridinyl, carbolinyl, acridinyl, perimidinyl, phenanthrolinyl, phenazinyl, phenothiazinyl, imidazolinyl, imidazolidinyl, benzimidazolyl, pyrazolinyl, pyrazolidinyl, pyrrolinyl, pyrrolidinyl, piperazinyl, uridinyl, thymidinyl,cytidinyl, azirinyl, aziridinyl, diazirinyl, diaziridinyl, oxiranyl, oxaziridinyl, dioxiranyl, thiiranyl, azetyl, dihydroazetyl, azetidinyl, oxetyl, oxetanyl, thietyl and thietanyl;

- $R_2$ is a radical selected from the group consisting of hydrogen; $C_{1-5}$ alkyl; $C_{2-7}$ alkenyl; aryl; alkylaryl; ω-hydroxy $C_{1-7}$ alkyl; ω-epoxy $C_{1-7}$ alkyl; ω-carboxy $C_{1-7}$ alkyl (wherein the carboxy group may be acid, ester, thioester, acid halide or amide); ω-cyano $C_{1-7}$ alkyl; arylalkyl; arylalkenyl; heterocyclic-substituted alkyl; heterocyclic-substituted alkenyl; groups having the formula -S-R (i.e.

  wherein a sulfur atom is attached to the nitrogen atom of the pteridine ring)

wherein R is a monovalent group selected from the group consisting of $C_{1-7}$ alkyl, aryl and $C_{3-10}$ cycloalkyl and wherein the said monovalent group is optionally substituted with one or more substituents selected from the group consisting of amino, amino-acid, alkylamino, arylamino, cycloalkylamino, carboxylic acid, carboxylic ester, sulfonic acid and phosphonic acid; and optionally substituted heterocyclic radicals preferably other than tetrahydrofuryl, i. e. preferably selected from the group consisting of diazepinyl, oxadiazinyl, thiadiazinyl, dithiazinyl, triazolonyl, diazepinonyl, triazepinyl, triazepinonyl, tetrazepinonyl, benzoquinolinyl, benzothiazinyl, benzothiazinonyl, benzoxathiinyl, benzodioxinyl, benzodithiinyl, benzoxazepinyl, benzothiazepinyl, benzodiazepinyl, benzodioxepinyl, benzodithiepinyl, benzoxazocinyl, benzothiazocinyl, benzodiazocinyl, benzoxathiocinyl, benzodioxocinyl, benzotrioxepinyl, benzoxathiazepinyl, benzoxadiazepinyl, benzothiadiazepinyl, benzotriazepinyl, benzoxathiepinyl, benzotriazinonyl, benzoxazolinonyl, azetidinonyl, azaspiroundecyl, dithiaspirodecyl, selenazinyl, selenazolyl, selenophenyl, hypoxanthinyl, azahypoxanthinyl, bipyrazinyl, bipyridinyl, oxazolidinyl, diselenopyrimidinyl, benzodioxocinyl, benzopyrenyl, benzopyranonyl, benzophenazinyl, benzoquinolizinyl, dibenzocarbazolyl, dibenzoacridinyl, dibenzophenazinyl, dibenzothiepinyl, dibenzooxepinyl, dibenzopyranonyl, dibenzoquinoxalinyl, dibenzothiazepinyl, dibenzoisoquinolinyl, tetraazaadamantyl, thiatetraazaadamantyl, oxauracil, oxazinyl, dibenzothiophenyl, dibenzofuranyl, oxazolinyl, oxazolonyl, azaindolyl, azolonyl, thiazolinyl, thiazolonyl, thiazolidinyl, thiazanyl, pyrimidonyl, thiopyrimidonyl, thiamorpholinyl, azlactonyl, naphtindazolyl, naphtindolyl, naphtothiazolyl, naphtothioxolyl, naphtoxindolyl, naphtotriazolyl, naphtopyranyl, indolinyl, indolizidinyl, oxabicycloheptyl, azabenzimidazolyl, azacycloheptyl, azacyclooctyl, azacyclononyl, azabicyclononyl, tetrahydropyranyl, tetrahydropyronyl, tetrahydroquinoleinyl, tetrahydrothienyl and dioxide thereof, dihydrothienyl dioxide, dioxindolyl, dioxinyl, dioxenyl, dioxazinyl, thioxanyl, thioxolyl, thio-urazolyl, thiotriazolyl, thiopyranyl, thiopyronyl, coumarinyl, quinoleinyl, oxyquinoleinyl, quinuclidinyl, xanthinyl, dihydropyranyl, benzodihydrofuryl, benzothiopyronyl, benzothiopyranyl, benzoxazinyl, benzoxazolyl, benzodioxolyl, benzodioxanyl, benzothiadiazolyl, benzotriazinyl, benzothiazolyl, benzoxazolyl, phenothioxinyl, phenothiazolyl, phenothienyl, phenopyronyl, phenoxazolyl, pyridinyl, dihydropyridinyl, tetrahydropyridinyl, piperidinyl, morpholinyl, thiomorpholinyl, pyrazinyl, pyrimidinyl, pyridazinyl, triazinyl, tetrazinyl, triazolyl, benzotriazolyl, tetrazolyl, imidazolyl, pyrazolyl, thiazolyl, thiadiazolyl, isothiazolyl, oxazolyl, oxadiazolyl, pyrrolyl, furyl, dihydrofuryl, furoyl, hydantoinyl, dioxolanyl, dioxolyl, dithianyl, dithienyl, dithiinyl, thienyl, indolyl, indazolyl, benzofuryl, benzothienyl, quinolyl, quinazolinyl, quinoxalinyl, carbazolyl, phenoxazinyl, phenothiazinyl, xanthenyl, purinyl, benzothienyl, naphtothienyl, thianthrenyl, pyranyl, pyronyl, benzopyronyl, isobenzofuranyl, chromenyl, phenoxathiinyl, indolizinyl, quinolizinyl, isoquinolyl, phthalazinyl, naphthiridinyl, cinnolinyl, pteridinyl, carbolinyl, acridinyl, perimidinyl, phenanthrolinyl, phenazinyl, phenothiazinyl, imidazolinyl, imidazolidinyl, benzimidazolyl, pyrazolinyl, pyrazolidinyl, pyrrolinyl, pyrrolidinyl, piperazinyl, uridinyl, thymidinyl,cytidinyl, azirinyl, aziridinyl, diazirinyl, diaziridinyl, oxiranyl, oxaziridinyl, dioxiranyl, thiiranyl, azetyl, dihydroazetyl, azetidinyl, oxetyl, oxetanyl, thietyl and thietanyl;

- at most one of $R_1$ and $R_2$ is hydrogen; and
- $R_3$ is an atom or radical selected from the group consisting of fluorine; iodine; $C_{3-4}$ alkyl; $C_{2-7}$ alkenyl; $C_{2-7}$ alkynyl; $C_3$-$_4$ haloalkyl; $C_{1-2}$ haloalkyl wherein halo is fluoro or chloro; $C_{1-4}$ alkoxy; $C_{3-10}$ cycloalkoxy; aryloxy; arylalkyloxy; oxyheterocyclic; heterocyclic-substituted alkyloxy; thio $C_{1-7}$ alkyl; thio $C_{3-10}$ cycloalkyl; thioaryl; thioheterocyclic; arylalkylthio; heterocyclic-substituted alkylthio; hydroxylamino; acetal; carboxylic acid esters, thioesters and amides; thiocarboxylic acid; thiocarboxylic acid esters, thioesters and amides; sulfhydryl; $C_{2-7}$

alkylamino; cycloalkylamino; alkenylamino; cycloalkenylamino; alkynylamino; arylamino; arylalkylamino; hydroxyalkylamino; mercaptoalkyl-amino; heterocyclic amino; heterocyclic-substituted alkylamino; oximino; alkyloximino; hydrazino; alkylhydrazino; phenylhydrazino; cysteinyl acid, esters or amides; aryl substituted with one or more substituents selected from the group consisting of halogen, $C_{1-4}$ alkyl, $C_{3-7}$ alkenyl, $C_{2-7}$ alkynyl, $C_{1-4}$ haloalkyl, $C_{2-4}$ alkoxy, hydroxyl, sulfhydryl, amino, $C_{3-10}$ cycloalkoxy, aryloxy, arylalkyloxy, oxyheterocyclic, heterocyclic-substituted alkyloxy, thio $C_{1-7}$ alkyl, thio $C_{3-10}$ cycloalkyl, thioaryl, thioheterocyclic, arylalkylthio, heterocyclic-substituted alkylthio, formyl, hydroxylamino, cyano, carboxylic acid or esters or thioesters or amides thereof, thiocarboxylic acid or esters or thioesters or amides thereof, $C_{1-7}$ alkylamino, cycloalkylamino, alkenylamino, cycloalkenylamino, alkynylamino, arylamino, arylalkylamino, hydroxylalkylamino, mercaptoalkylamino, heterocyclic amino, hydrazino, alkylhydrazino and phenylhydrazino; optionally substituted heterocyclic radicals; aryl or heterocyclic radicals substituted with an aliphatic spacer (linking group) between the pteridine ring and the aryl or heterocyclic radical, whereby said aliphatic spacer is a branched or straight, saturated or unsaturated aliphatic chain of 2 to 4 carbon atoms which may contain one or more functions, atoms or radicals selected from the group consisting of carbonyl (oxo), alcohol (hydroxyl), ether, acetal, amino, imino, oximino, alkyloximino, amino-acid, cyano, carboxylic acid or ester or thioester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxyalkyl-amino, mercaptoalkylamino, heterocyclic amino, hydrazino, alkylhydrazino, phenylhydrazino, sulfonyl, sulfonamido and halogen, or whereby said aliphatic spacer is a methylene group containing a function, atom or radical chosen from the group consisting of carbonyl (oxo), alcohol (hydroxyl), ether, acetal, amino, imino, oximino, alkyloximino, amino-acid, cyano, carboxylic acid or ester or thioester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxyalkylamino, mercaptoalkylamino, heterocyclic amino, hydrazino, alkylhydrazino, phenylhydrazino, sulfonyl, sulfonamido and halogen; branched or straight, saturated or unsaturated aliphatic chain of 3 to 7 carbon atoms containing one or more functions selected from the group consisting of carbonyl (oxo), alcohol (hydroxyl), ether, acetal, amino, imino, oximino, alkyloximino, amino-acid, cyano, carboxylic acid ester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxylalkylamino, mercaptoalkyl-amino, heterocyclic amino, hydrazino, alkylhydrazino, phenylhydrazino, sulfonyl, sulfonamido and halogen; hydroxyethyl; oximinoethyl; alkyloximinoethyl; and methyl or ethyl or ethenyl containing one or more atoms, functions or radicals selected from the group consisting of ether, acetal, amino, imino, amino-acid, cyano, carboxylic acid or ester or thioester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylalkyl-amino, hydroxyalkylamino, mercaptoalkylamino, heterocyclic amino, hydrazino, alkylhydrazino, phenylhydrazino, sulfonyl, sulfonamido, fluoro and chloro;

b) if $Y_1$ and $Y_2$ are both oxygen and $R_3$ is hydrogen, then:

- $R_1$ is a radical selected from the group consisting of hydrogen; $C_{1-5}$ alkyl; $C_{2-7}$ alkenyl; aryl; alkylaryl; ω-hydroxy $C_{1-5}$ alkyl; ω-epoxy $C_{1-5}$ alkyl; ω-carboxy $C_{1-5}$ alkyl (wherein the carboxy group may be acid, ester, thioester, acid halide or amide); ω-cyano $C_{1-7}$ alkyl; arylalkyl; arylalkenyl; heterocyclic-substituted alkyl; heterocyclic-substituted alkenyl; groups having the formula -S-R (i.e.

wherein a sulfur atom is attached to the nitrogen atom of the pteridine ring)

wherein R is a monovalent group selected from the group consisting of $C_{1-7}$ alkyl, aryl and $C_{3-10}$ cycloalkyl and wherein the said monovalent group is optionally substituted with one or more substituents selected from the group consisting of amino, amino-acid, alkylamino, arylamino, cycloalkylamino, carboxylic acid, carboxylic ester, sulfonic acid and phosphonic acid; and optionally substituted heterocyclic radicals preferably selected from the group consisting of diazepinyl, oxadiazinyl, thiadiazinyl, dithiazinyl, triazolonyl, diazepinonyl, triazepinyl, triazepinonyl, tetrazepinonyl, benzoquinolinyl, benzothiazinyl, benzothiazinonyl, benzoxathiinyl, benzodioxinyl, benzodithiinyl, benzoxazepinyl, benzothiazepinyl, benzodiazepinyl, benzodioxepinyl, benzodithiepinyl, benzoxazocinyl, benzothiazocinyl, benzodiazocinyl, benzoxathiocinyl, benzodioxocinyl, benzotrioxepinyl, benzoxathiazepinyl, benzoxadiazepinyl, benzothiadiazepinyl, benzotriazepinyl, benzoxathiepinyl, benzotriazinonyl, benzoxazolinonyl, azetidinonyl, azaspiroundecyl, dithiaspirodecyl, selenazinyl, selenazolyl, selenophenyl, hypoxanthinyl, azahypoxanthinyl, bipyrazinyl, bipyridinyl, oxazolidinyl, diselenopyrimidinyl, benzodioxocinyl, benzopyrenyl, benzopyranonyl, benzophenazinyl, benzoquinolizinyl, dibenzocarbazolyl, dibenzoacridinyl, dibenzophenazinyl, dibenzothiepinyl, dibenzooxepinyl, dibenzopyranonyl, dibenzoquinoxalinyl, dibenzothiazepinyl, dibenzoisoquinolinyl, tetraazaadamantyl, thiatetraazaadamantyl, oxauracil, oxazinyl, dibenzothiophenyl, dibenzofuranyl, oxazolinyl, oxazolonyl, azaindolyl, azolonyl, thiazolinyl, thiazolonyl, thiazolidinyl, thiazanyl, pyrimidonyl, thiopyrimidonyl, thiamorpholinyl, azlactonyl, naphtindazolyl, naphtindolyl, naphtothiazolyl, naphtothioxolyl, naphtoxindolyl, naphtotriazolyl, naphtopyranyl, indolinyl, indolizidinyl, oxabicycloheptyl, azabenzimidazolyl, azacycloheptyl, azacyclooctyl, azacyclononyl, azabicyclononyl, tetrahydropyranyl, tetrahydropyronyl, tetrahydroquinoleinyl, tetrahydrothienyl and dioxide thereof, dihydrothienyl dioxide, dioxindolyl, dioxinyl, dioxenyl, dioxazinyl, thioxanyl, thioxolyl, thio-urazolyl, thiotriazolyl, thiopyranyl, thiopyronyl, coumarinyl, quinoleinyl, oxyquinoleinyl, quinuclidinyl, xanthinyl, dihydropyranyl, benzodihydrofuryl, benzothiopyronyl, benzothiopyranyl, benzoxazinyl, benzoxazolyl, benzodioxolyl, benzodioxanyl, benzothiadiazolyl, benzotriazinyl, benzothiazolyl, benzoxazolyl, phenothioxinyl, phenothiazolyl, phenothienyl, phenopyronyl, phenoxazolyl, pyridinyl, dihydropyridinyl, tetrahydropyridinyl, piperidinyl, morpholinyl, thiomorpholinyl, pyrazinyl, pyrimidinyl, pyridazinyl, triazinyl, tetrazinyl, triazolyl, benzotriazolyl, tetrazolyl, imidazolyl, pyrazolyl, thiazolyl, thiadiazolyl, isothiazolyl, oxazolyl, oxadiazolyl, pyrrolyl, furyl, dihydrofuryl, furoyl, hydantoinyl, dioxolanyl, dioxolyl, dithianyl, dithienyl, dithiinyl, thienyl, indolyl, indazolyl, benzofuryl, benzothienyl, quinolyl, quinazolinyl, quinoxalinyl, carbazolyl, phenoxazinyl, phenothiazinyl, xanthenyl, purinyl, benzothienyl, naphtothienyl, thianthrenyl, pyranyl, pyronyl, benzopyronyl, isobenzofuranyl, chromenyl, phenoxathiinyl, indolizinyl, quinolizinyl, isoquinolyl, phthalazinyl, naphthiridinyl, cinnolinyl, pteridinyl, carbolinyl, acridinyl, perimidinyl, phenanthrolinyl, phenazinyl, phenothiazinyl, imidazolinyl, imidazolidinyl, benzimidazolyl, pyrazolinyl, pyrazolidinyl, pyrrolinyl, pyrrolidinyl, piperazinyl, uridinyl, thymidinyl,cytidinyl, azirinyl, aziridinyl, diazirinyl, diaziridinyl, oxiranyl, oxaziridinyl, dioxiranyl, thiiranyl, azetyl, dihydroazetyl, azetidinyl, oxetyl, oxetanyl, thietyl and thietanyl;

- $R_2$ is a radical selected from the group consisting of hydrogen; $C_{1-5}$ alkyl; $C_{2-7}$ alkenyl; aryl; alkylaryl; ω-hydroxy $C_{1-7}$ alkyl; ω-epoxy $C_{1-7}$ alkyl; ω-carboxy $C_{1-7}$ alkyl (wherein the carboxy group may be acid, ester, thioester, acid halide or amide); ω-cyano $C_{1-7}$ alkyl; arylalkyl; arylalkenyl; heterocyclic-substituted alkyl; heterocyclic-substituted alkenyl; groups having the formula -S-R (i.e.

wherein a sulfur atom is attached to the nitrogen atom of the pteridine ring)

wherein R is a monovalent group selected from the group consisting of $C_{1-7}$ alkyl, aryl and $C_{3-10}$ cycloalkyl and wherein the said monovalent group is optionally substituted with one or more substituents selected from the group consisting of amino, amino-acid, alkylamino, arylamino, cycloalkylamino, carboxylic acid, carboxylic ester, sulfonic acid and phosphonic acid; and optionally substituted heterocyclic radicals preferably selected from the group consisting of diazepinyl, oxadiazinyl, thiadiazinyl, dithiazinyl, triazolonyl, diazepinonyl, triazepinyl, triazepinonyl, tetrazepinonyl, benzoquinolinyl, benzothiazinyl, benzothiazinonyl, benzoxathiinyl, benzodioxinyl, benzodithiinyl, benzoxazepinyl, benzothiazepinyl, benzodiazepinyl, benzodioxepinyl, benzodithiepinyl, benzoxazocinyl, benzothiazocinyl, benzodiazocinyl, benzoxathiocinyl, benzodioxocinyl, benzotrioxepinyl, benzoxathiazepinyl, benzoxadiazepinyl, benzothiadiazepinyl, benzotriazepinyl, benzoxathiepinyl, benzotriazinonyl, benzoxazolinonyl, azetidinonyl, azaspiroundecyl, dithiaspirodecyl, selenazinyl, selenazolyl, selenophenyl, hypoxanthinyl, azahypoxanthinyl, bipyrazinyl, bipyridinyl, oxazolidinyl, diselenopyrimidinyl, benzodioxocinyl, benzopyrenyl, benzopyranonyl, benzophenazinyl, benzoquinolizinyl, dibenzocarbazolyl, dibenzoacridinyl, dibenzophenazinyl, dibenzothiepinyl, dibenzooxepinyl, dibenzopyranonyl, dibenzoquinoxalinyl, dibenzothiazepinyl, dibenzoisoquinolinyl, tetraazaadamantyl, thiatetraazaadamantyl, oxauracil, oxazinyl, dibenzothiophenyl, dibenzofuranyl, oxazolinyl, oxazolonyl, azaindolyl, azolonyl, thiazolinyl, thiazolonyl, thiazolidinyl, thiazanyl, pyrimidonyl, thiopyrimidonyl, thiamorpholinyl, azlactonyl, naphtindazolyl, naphtindolyl, naphtothiazolyl, naphtothioxolyl, naphtoxindolyl, naphtotriazolyl, naphtopyranyl, indolinyl, indolizidinyl, oxabicycloheptyl, azabenzimidazolyl, azacycloheptyl, azacyclooctyl, azacyclononyl, azabicyclononyl, tetrahydropyranyl, tetrahydropyronyl, tetrahydroquinoleinyl, tetrahydrothienyl and dioxide thereof, dihydrothienyl dioxide, dioxindolyl, dioxinyl, dioxenyl, dioxazinyl, thioxanyl, thioxolyl, thio-urazolyl, thiotriazolyl, thiopyranyl, thiopyronyl, coumarinyl, quinoleinyl, oxyquinoleinyl, quinuclidinyl, xanthinyl, dihydropyranyl, benzodihydrofuryl, benzothiopyronyl, benzothiopyranyl, benzoxazinyl, benzoxazolyl, benzodioxolyl, benzodioxanyl, benzothiadiazolyl, benzotriazinyl, benzothiazolyl, benzoxazolyl, phenothioxinyl, phenothiazolyl, phenothienyl, phenopyronyl, phenoxazolyl, pyridinyl, dihydropyridinyl, tetrahydropyridinyl, piperidinyl, morpholinyl, thiomorpholinyl, pyrazinyl, pyrimidinyl, pyridazinyl, triazinyl, tetrazinyl, triazolyl, benzotriazolyl, tetrazolyl, imidazolyl, pyrazolyl, thiazolyl, thiadiazolyl, isothiazolyl, oxazolyl, oxadiazolyl, pyrrolyl, furyl, dihydrofuryl, furoyl, hydantoinyl, dioxolanyl, dioxolyl, dithianyl, dithienyl, dithiinyl, thienyl, indolyl, indazolyl, benzofuryl, benzothienyl, quinolyl, quinazolinyl, quinoxalinyl, carbazolyl, phenoxazinyl, phenothiazinyl, xanthenyl, purinyl, benzothienyl, naphtothienyl, thianthrenyl, pyranyl, pyronyl, benzopyronyl, isobenzofuranyl, chromenyl, phenoxathiinyl, indolizinyl, quinolizinyl, isoquinolyl, phthalazinyl, naphthiridinyl, cinnolinyl, pteridinyl, carbolinyl, acridinyl, perimidinyl, phenanthrolinyl, phenazinyl, phenothiazinyl, imidazolinyl, imidazolidinyl, benzimidazolyl, pyrazolinyl, pyrazolidinyl, pyrrolinyl, pyrrolidinyl, piperazinyl, uridinyl, thymidinyl,cytidinyl, azirinyl, aziridinyl, diazirinyl, diaziridinyl, oxiranyl, oxaziridinyl, dioxiranyl, thiiranyl, azetyl, dihydroazetyl, azetidinyl, oxetyl, oxetanyl, thietyl and thietanyl;;

- at most one of $R_1$ and $R_2$ is hydrogen; and

- $R_4$ is an atom or radical selected from the group consisting of fluoro; iodo; $C_{3-4}$ alkyl; $C_{2-7}$ alkenyl; $C_{2-7}$ alkynyl; halo $C_{3-4}$ alkyl; halo $C_{1-2}$ alkyl wherein halo is fluoro or chloro; $C_{1-4}$ alkoxy; $C_{3-10}$ cycloalkoxy; aryloxy; arylalkyloxy; oxyheterocyclic; heterocyclic-substituted alkyloxy; thio $C_{1-7}$ alkyl; thio $C_{3-10}$ cycloalkyl; thioaryl; thioheterocyclic; arylalkylthio; heterocyclic-substituted alkylthio; hydroxylamino; acetal; carboxylic acid esters, thioesters and amides; thiocarboxylic acid; thiocarboxylic acid esters, thioesters and amides; sulfhydryl; $C_{2-7}$ alkylamino; cycloalkylamino; alkenylamino; cycloalkenylamino; alkynylamino; arylamino; arylalkylamino; hydroxyalkylamino; mercaptoalkyl-amino; heterocyclic amino; heterocyclic-substituted alkylamino; oximino; alkyloximino; hydrazino; alkylhydrazino; phenylhydrazino; cysteinyl acid, esters or amides; aryl substituted with one or more substituents selected from the group consisting of halogen, nitro, $C_{1-7}$ alkyl, $C_{2-7}$ alkenyl, $C_{2-7}$ alkynyl, halo $C_{1-7}$ alkyl, methoxy, $C_{2-7}$ alkoxy, hydroxyl, sulfhydryl, amino, $C_{3-10}$ cycloalkoxy, aryloxy, arylalkyloxy, oxyheterocyclic, heterocyclic-substituted alkyloxy, thio $C_{1-7}$ alkyl, thio $C_{3-10}$ cycloalkyl, thioaryl, thioheterocyclic, arylalkylthio, heterocyclic-substituted alkylthio, formyl, hydroxylamino, cyano, carboxylic acid or esters or thioesters or amides thereof, thiocarboxylic acid or esters or thioesters or amides thereof, $C_{1-7}$ alkylamino, cycloalkylamino, alkenylamino, cycloalkenylamino, alkynylamino, arylamino, arylalkylamino, hydroxylalkylamino, mercaptoalkylamino, heterocyclic amino, hydrazino, alkylhydrazino and phenylhydrazino; optionally substituted heterocyclic radicals preferably other than morpholino and piperidino, i.e. preferably selected from the group consisting of diazepinyl, oxadiazinyl, thiadiazinyl, dithiazinyl, triazolonyl, diazepinonyl, triazepinyl, triazepinonyl, tetrazepinonyl, benzoquinolinyl, benzothiazinyl, benzothiazinonyl, benzoxathiinyl, benzodioxinyl, benzodithiinyl, benzoxazepinyl, benzothiazepinyl, benzodiazepinyl, benzodioxepinyl, benzodithiepinyl, benzoxazocinyl, benzothiazocinyl, benzodiazocinyl, benzoxathiocinyl, benzodioxocinyl, benzotrioxepinyl, benzoxathiazepinyl, benzoxadiazepinyl, benzothiadiazepinyl, benzotriazepinyl, benzoxathiepinyl, benzotriazinonyl, benzoxazolinonyl, azetidinonyl, azaspiroundecyl, dithiaspirodecyl, selenazinyl, selenazolyl, selenophenyl, hypoxanthinyl, azahypoxanthinyl, bipyrazinyl, bipyridinyl, oxazolidinyl, diselenopyrimidinyl, benzodioxocinyl, benzopyrenyl, benzopyranonyl, benzophenazinyl, benzoquinolizinyl, dibenzocarbazolyl, dibenzoacridinyl, dibenzophenazinyl, dibenzothiepinyl, dibenzooxepinyl, dibenzopyranonyl, dibenzoquinoxalinyl, dibenzothiazepinyl, dibenzoisoquinolinyl, tetraazaadamantyl, thiatetraazaadamantyl, oxauracil, oxazinyl, dibenzothiophenyl, dibenzofuranyl, oxazolinyl, oxazolonyl, azaindolyl, azolonyl, thiazolinyl, thiazolonyl, thiazolidinyl, thiazanyl, pyrimidonyl, thiopyrimidonyl, thiamorpholinyl, azlactonyl, naphtindazolyl, naphtindolyl, naphtothiazolyl, naphtothioxolyl, naphtoxindolyl, naphtotriazolyl, naphtopyranyl, indolinyl, indolizidinyl, oxabicycloheptyl, azabenzimidazolyl, azacycloheptyl, azacyclooctyl, azacyclononyl, azabicyclononyl, tetrahydrofuryl, tetrahydropyranyl, tetrahydropyronyl, tetrahydroquinoleinyl, tetrahydrothienyl and dioxide thereof, dihydrothienyl dioxide, dioxindolyl, dioxinyl, dioxenyl, dioxazinyl, thioxanyl, thioxolyl, thio-urazolyl, thiotriazolyl, thiopyranyl, thiopyronyl, coumarinyl, quinoleinyl, oxyquinoleinyl, quinuclidinyl, xanthinyl, dihydropyranyl, benzodihydrofuryl, benzothiopyronyl, benzothiopyranyl, benzoxazinyl, benzoxazolyl, benzodioxolyl, benzodioxanyl, benzothiadiazolyl, benzotriazinyl, benzothiazolyl, benzoxazolyl, phenothioxinyl, phenothiazolyl, phenothienyl, phenopyronyl, phenoxazolyl, pyridinyl, dihydropyridinyl, tetrahydropyridinyl, piperidinyl, morpholinyl, thiomorpholinyl, pyrazinyl, pyrimidinyl, pyridazinyl, triazinyl, tetrazinyl, triazolyl, benzotriazolyl, imidazolyl, pyrazolyl, thiazolyl, thiadiazolyl, isothiazolyl, oxazolyl, oxadiazolyl, pyrrolyl, furyl, dihydrofuryl, furoyl, dioxolyl, dithienyl, dithiinyl, thienyl, indolyl, indazolyl, benzofuryl, benzothienyl, quinolyl, quinazolinyl, quinoxalinyl, carbazolyl, phenoxazinyl, phenothiazinyl, xanthenyl, purinyl, benzothienyl, naphtothienyl, thianthrenyl, pyranyl, pyronyl, benzopyronyl, isobenzofuranyl, chromenyl, phenoxathiinyl, indolizinyl, quinolizinyl, isoquinolyl, phthalazinyl, naphthiridinyl, cinnolinyl, pteridinyl, carbolinyl, acridinyl, perimidinyl, phenanthrolinyl, phenazinyl, phenothiazinyl, imidazolinyl, benzimidazolyl, pyrazolinyl, pyrazolidinyl, pyrrolinyl, pyrrolidinyl, piperazinyl, uridinyl, thymidinyl, cytidinyl, azirinyl, aziridinyl, diazirinyl, diaziridinyl, oxiranyl, oxaziridinyl, dioxiranyl, thiiranyl, azetyl, dihydroazetyl, azetidinyl, oxetyl, oxetanyl, thietyl and thietanyl; aryl or heterocyclic radicals substituted with an aliphatic spacer between the pteridine ring and the aryl or heterocyclic radical, whereby said aliphatic spacer is a branched or straight, saturated or unsaturated aliphatic chain of 2 to 4 carbon atoms which may contain one or more functions, atoms or radicals selected from the group consisting of carbonyl (oxo), alcohol (hydroxyl), ether, acetal, amino, imino, oximino, alkyloximino, amino-acid, cyano, carboxylic acid or ester or thioester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxyalkyl-amino, mercaptoalkylamino, heterocyclic amino, hydrazino, alkylhydrazino, phenylhydrazino, sulfonyl, sulfonamido and halogen, or whereby said aliphatic spacer is a methylene group containing a function, atom or radical chosen from the group consisting of carbonyl (oxo), alcohol (hydroxyl), ether, acetal, amino, imino, oximino, alkyloximino, amino-acid, cyano, carboxylic acid or ester or thioester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxyalkylamino, mercaptoalkylamino, heterocyclic amino, hydrazino, alkylhydrazino, phenylhydrazino, sulfonyl, sulfonamido, fluoro and chloro; branched or straight, saturated or unsaturated aliphatic chain of 3 to 7 carbon atoms

containing one or more functions selected from the group consisting of carbonyl (oxo), alcohol (hydroxyl), ether, acetal, amino, imino, oximino, alkyloximino, aminoacid, cyano, carboxylic acid ester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxyalkylamino, mercaptoalkylamino, heterocyclic amino, hydrazino, alkylhydrazino, phenylhydrazino, sulfonyl, sulfonamido and halogen; hydroxyethyl; oximinoethyl; alkyloximinoethyl; and methyl or ethyl or ethenyl containing one or more functions, atoms or radicals selected from the group consisting of ether, acetal, amino, imino, amino-acid, cyano, carboxylic acid or ester or thioester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylalkyl-amino, hydroxyalkylamino, mercaptoalkylamino, heterocyclic amino, hydrazino, alkylhydrazino, phenylhydrazino, sulfonyl, sulfonamido, fluoro and chloro;

c) if one or more of $Y_1$ and $Y_2$ is sulfur and at most one of $Y_1$ and $Y_2$ is oxygen, then:

- each of $R_1$ and $R_2$ is a radical independently selected from the group consisting of hydrogen; $C_{1-7}$ alkyl; $C_{2-7}$ alkenyl; aryl; alkylaryl; ω-hydroxy $C_{1-7}$ alkyl; ω-epoxy $C_{1-7}$ alkyl; ω-carboxy $C_{1-7}$ alkyl (wherein the carboxy group may be acid, ester, thioester, acid halide or amide); ω-cyano $C_{1-7}$ alkyl; arylalkyl; arylalkenyl; heterocyclic-substituted alkyl; heterocyclic-substituted alkenyl; groups having the formula -S-R (i.e. wherein a sulfur atom is attached to the nitrogen atom of the pteridine ring) wherein R is a monovalent group selected from the group consisting of $C_{1-7}$ alkyl, aryl and $C_{3-10}$ cycloalkyl and wherein the said monovalent group is optionally substituted with one or more substituents selected from the group consisting of amino, amino-acid, alkylamino, arylamino, cycloalkylamino, carboxylic acid, carboxylic ester, sulfonic acid and phosphonic acid; and optionally substituted heterocyclic radicals;
- each of $R_3$ and $R_4$ is an atom or radical independently selected from the group consisting of hydrogen; halogen; $C_{2-4}$ alkyl; $C_{2-7}$ alkenyl; $C_{2-7}$ alkynyl; halo $C_{1-4}$ alkyl; $C_{2-4}$ alkoxy; $C_{3-10}$ cycloalkoxy; aryloxy; arylalkyloxy; oxyheterocyclic; heterocyclic-substituted alkyloxy; thio $C_{2-7}$ alkyl; thio $C_{3-10}$ cycloalkyl; thioaryl; thioheterocyclic; arylalkylthio; heterocyclic-substituted alkylthio; hydroxylamino; acetal; formyl; cyano; carboxylic acid; carboxylic acid esters, thioesters and amides; thiocarboxylic acid; thiocarboxylic acid esters, thioesters and amides; amino; alkylamino; cycloalkylamino; alkenylamino; cycloalkenylamino; alkynylamino; arylamino; arylalkylamino; hydroxyalkylamino; mercaptoalkylamino; heterocyclic amino; heterocyclic-substituted alkylamino; oximino; alkyloximino; hydrazino; alkylhydrazino; phenylhydrazino; cysteinyl acid, esters or amides; aryl substituted with one or more substituents selected from the group consisting of halogen, $C_{1-4}$ alkyl, $C_{2-7}$ alkenyl, $C_{2-7}$ alkynyl, halo $C_{1-4}$ alkyl, $C_{1-4}$ alkoxy, hydroxyl, sulfhydryl, amino, $C_{3-10}$ cycloalkoxy, aryloxy, arylalkyloxy, oxyheterocyclic, heterocyclic-substituted alkyloxy, thio $C_{1-7}$ alkyl, thio $C_{3-10}$ cycloalkyl, thioaryl, thioheterocyclic, arylalkylthio, heterocyclic-substituted alkylthio, formyl, hydroxylamino, cyano, carboxylic acid or esters or thioesters or amides thereof, thiocarboxylic acid or esters or thioesters or amides thereof, $C_{1-7}$ alkylamino, cycloalkylamino, alkenylamino, cycloalkenylamino, alkynylamino, arylamino, arylalkylamino, hydroxylalkylamino, mercaptoalkylamino, heterocyclic amino, hydrazino, alkylhydrazino and phenylhydrazino; optionally substituted heterocyclic radicals; aryl or heterocyclic radicals substituted with an aliphatic spacer (linking group) between the pteridine ring and the aryl or heterocyclic radical, whereby said aliphatic spacer is a branched or straight, saturated or unsaturated aliphatic chain of 1 to 4 carbon atoms (such as a $C_{1-4}$ alkylene) which may contain one or more functions, atoms or radicals selected from the group consisting of carbonyl (oxo), alcohol (hydroxyl), ether, acetal, amino, imino, oximino, alkyloximino, amino-acid, cyano, carboxylic acid or ester or thioester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxyalkylamino, mercaptoalkylamino, heterocyclic amino, hydrazino, alkylhydrazino, phenylhydrazino, sulfonyl, sulfonamido and halogen; branched or straight, saturated or unsaturated aliphatic chain of 1 to 7 carbon atoms containing one or more functions selected from the group consisting of carbonyl (oxo), alcohol (hydroxyl), ether, acetal, amino, imino, oximino, alkyloximino, amino-acid, cyano, carboxylic acid ester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxylalkylamino, mercaptoalkyl-amino, heterocyclic amino, hydrazino, alkylhydrazino, phenylhydrazino, sulfonyl, sulfonamido and halogen; or $R_4$ and $R_3$ together form an aryl radical being optionally substituted with one or more substituents $R_a$ each independently selected from the group consisting of amino, alkylamino, cycloalkylamino, alkenylamino, cycloalkenylamino, alkynylamino, arylamino, arylalkylamino, hydroxyalkylamino, mercaptoalkylamino, heterocyclic amino and heterocyclic-substituted alkylamino, wherein each substituent $R_a$ may further comprise one or more functions selected from the group consisting of carbonyl, amino and carboxyl, and wherein two adjacent substituents $R_a$ may together form an heterocyclic radical; and
- at most one of $R_1$, $R_2$, $R_3$ and $R_4$ is hydrogen;

d) if $Y_1$ and $Y_2$ are both oxygen and none of $R_3$ and $R_4$ is hydrogen, then:

- $R_2$ is a radical selected from the group consisting of $C_{1-7}$ alkyl; $C_{2-7}$ alkenyl; aryl; alkylaryl; ω-hydroxy $C_{1-7}$ alkyl; ω-epoxy $C_{1-7}$ alkyl; ω-carboxy $C_{1-7}$ alkyl (wherein the carboxy group may be acid, ester, thioester, acid halide or amide); ω-cyano $C_{1-7}$ alkyl; arylalkyl; arylalkenyl; heterocyclic-substituted alkyl; heterocyclic-substituted alkenyl; groups having the formula -S-R (i.e.

  wherein a sulfur atom is attached to the nitrogen atom of the pteridine ring)

wherein R is a monovalent group selected from the group consisting of $C_{1-7}$ alkyl, aryl and $C_{3-10}$ cycloalkyl and wherein the said monovalent group is optionally substituted with one or more substituents selected from the group consisting of amino, amino-acid, alkylamino, arylamino, cycloalkylamino, carboxylic acid, carboxylic ester, sulfonic acid and phosphonic acid; and optionally substituted heterocyclic radicals;

- $R_1$ is an atom or radical independently defined as $R_2$, or is hydrogen;
- $R_4$ is an atom or radical selected from the group consisting of halogen (preferably chloro); $C_{2-7}$ alkenyl; $C_{2-7}$ alkynyl; $C_{2-7}$ haloalkyl; fluoromethyl; $C_{2-4}$ alkoxy; $C_{3-10}$ cycloalkoxy; aryloxy; arylalkyloxy; oxyheterocyclic; heterocyclic-substituted alkyloxy; thio $C_{1-7}$ alkyl; thio $C_{3-10}$ cycloalkyl; thioaryl; thioheterocyclic; arylalkylthio; heterocyclic-substituted alkylthio; hydroxylamino; acetal; carboxylic acid thioesters and amides; thiocarboxylic acid; thiocarboxylic acid esters, thioesters and amides; sulfhydryl; $C_{2-7}$ alkylamino; cycloalkylamino; alkenylamino; cycloalkenylamino; alkynylamino; arylamino; arylalkylamino; hydroxyalkylamino; mercaptoalkylamino; heterocyclic amino; heterocyclic-substituted alkylamino; hydrazino; alkylhydrazino; phenylhydrazino; cysteinyl acid, esters or amides; aryl optionally substituted with one or more substituents selected from the group consisting of halogen, $C_{1-4}$ alkyl, $C_{2-7}$ alkenyl, $C_{2-7}$ alkynyl, halo $C_{1-7}$ alkyl, $C_{1-7}$ alkoxy, hydroxyl, sulfhydryl, amino, $C_{3-10}$ cycloalkoxy, aryloxy, arylalkyloxy, oxyheterocyclic, heterocyclic-substituted alkyloxy, thio $C_{1-7}$ alkyl, thio $C_{3-10}$ cycloalkyl, thioaryl, thioheterocyclic, arylalkylthio, heterocyclic-substituted alkylthio, formyl, hydroxylamino, cyano, carboxylic acid or esters or thioesters or amides thereof, thiocarboxylic acid or esters or thioesters or amides thereof, $C_{1-7}$ alkylamino, cycloalkylamino, alkenylamino, cycloalkenylamino, alkynylamino, arylamino, arylalkylamino, hydroxylalkylamino, mercaptoalkylamino, heterocyclic amino, hydrazino, alkylhydrazino and phenylhydrazino; optionally substituted heterocyclic radicals preferably selected from the group consisting of diazepinyl, oxadiazinyl, thiadiazinyl, dithiazinyl, triazolonyl, diazepinonyl, triazepinyl, triazepinonyl, tetrazepinonyl, benzoquinolinyl, benzothiazinyl, benzothiazinonyl, benzoxathiinyl, benzodioxinyl, benzodithiinyl, benzoxazepinyl, benzothiazepinyl, benzodiazepinyl, benzodioxepinyl, benzodithiepinyl, benzoxazocinyl, benzothiazocinyl, benzodiazocinyl, benzoxathiocinyl, benzodioxocinyl, benzotrioxepinyl, benzoxathiazepinyl, benzoxadiazepinyl, benzothiadiazepinyl, benzotriazepinyl, benzoxathiepinyl, benzotriazinonyl, benzoxazolinonyl, azetidinonyl, azaspiroundecyl, dithiaspirodecyl, selenazinyl, selenazolyl, selenophenyl, hypoxanthinyl, azahypoxanthinyl, bipyrazinyl, bipyridinyl, oxazolidinyl, diselenopyrimidinyl, benzodioxocinyl, benzopyrenyl, benzopyranonyl, benzophenazinyl, benzoquinolizinyl, dibenzocarbazolyl, dibenzoacridinyl, dibenzophenazinyl, dibenzothiepinyl, dibenzooxepinyl, dibenzopyranonyl, dibenzoquinoxalinyl, dibenzothiazepinyl, dibenzoisoquinolinyl, tetraazaadamantyl, thiatetraazaadamantyl, oxauracil, oxazinyl, dibenzothiophenyl, dibenzofuranyl, oxazolinyl, oxazolonyl, azaindolyl, azolonyl, thiazolinyl, thiazolonyl, thiazolidinyl, thiazanyl, pyrimidonyl, thiopyrimidonyl, thiamorpholinyl, azlactonyl, naphtindazolyl, naphtindolyl, naphtothiazolyl, naphtothioxolyl, naphtoxindolyl, naphtotriazolyl, naphtopyranyl, indolinyl, indolizidinyl, tetrahydrofuryl, oxabicycloheptyl, azabenzimidazolyl, azacycloheptyl, azacyclooctyl, azacyclononyl, azabicyclononyl, tetrahydropyranyl, tetra-hydropyronyl, tetrahydroquinoleinyl, tetrahydrothienyl and dioxide thereof, dihydrothienyl dioxide, dioxindolyl, dioxinyl, dioxenyl, dioxazinyl, thioxanyl, thioxolyl, thio-urazolyl, thiotriazolyl, thiopyranyl, thiopyronyl, coumarinyl, quinoleinyl, oxyquinoleinyl, quinuclidinyl, xanthinyl, dihydropyranyl, benzodihydrofuryl, benzothiopyronyl, benzothiopyranyl, benzoxazinyl, benzoxazolyl, benzodioxolyl, benzodioxanyl, benzothiadiazolyl, benzotriazinyl, benzothiazolyl, benzoxazolyl, phenothioxinyl, phenothiazolyl, phenothienyl, phenopyronyl, phenoxazolyl, pyridinyl, dihydropyridinyl, tetrahydropyridinyl, piperidinyl, morpholinyl, thiomorpholinyl, pyrazinyl, pyrimidinyl, pyridazinyl, triazinyl, tetrazinyl, triazolyl, benzotriazolyl, tetrazolyl, imidazolyl, pyrazolyl, thiazolyl, thiadiazolyl, isothiazolyl, oxazolyl, oxadiazolyl, pyrrolyl, furyl, dihydrofuryl, furoyl, hydantoinyl, dioxolyl, dithianyl, dithienyl, dithiinyl, thienyl, indolyl, indazolyl, benzofuryl, benzothienyl, quinolyl, quinazolinyl, quinoxalinyl, carbazolyl, phenoxazinyl, phenothiazinyl, xanthenyl, purinyl, benzothienyl, naphtothienyl, thianthrenyl, pyranyl, pyronyl, benzopyronyl, isobenzofuranyl, chromenyl, phenoxathiinyl, indolizinyl, quinolizinyl, isoquinolyl, phthalazinyl, naphthiridinyl, cinnolinyl, pteridinyl, carbolinyl, acridinyl, perimidinyl, phenanthrolinyl, phenazinyl, phenothiazinyl, imidazolinyl, imidazolidinyl, benzimidazolyl, pyrazolinyl, pyrazolidinyl, pyrrolinyl, pyrrolidinyl, piperazinyl, uridinyl, thymidinyl,cytidinyl, azirinyl, aziridinyl, diazirinyl, diaziridinyl, oxiranyl, oxaziridinyl, dioxiranyl, thiiranyl, azetyl, dihydroazetyl, azetidinyl, oxetyl, oxetanyl, thietyl and thietanyl; aryl or

heterocyclic radicals substituted with an aliphatic spacer between the pteridine ring and the aryl or heterocyclic radical, whereby said aliphatic spacer is a branched or straight, saturated or unsaturated aliphatic chain of 1 to 4 carbon atoms which may contain one or more functions, atoms or radicals selected from the group consisting of carbonyl (oxo), alcohol (hydroxyl), ether, acetal, amino, imino, oximino, alkyloximino, amino-acid, cyano, carboxylic acid or ester or thioester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxyalkylamino, mercaptoalkylamino, heterocyclic amino, hydrazino, alkylhydrazino, phenylhydrazino, sulfonyl, sulfonamido and halogen; branched or straight, saturated or unsaturated aliphatic chain of 2 to 7 carbon atoms containing one or more atoms, functions or radicals selected from the group consisting of carbonyl (oxo), alcohol (hydroxyl), ether, acetal, amino, imino, oximino, alkyloximino, amino-acid, cyano, carboxylic acid ester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxylalkylamino, mercaptoalkyl-amino, heterocyclic amino, hydrazino, alkylhydrazino, phenylhydrazino, sulfonyl, sulfonamido and halogen; hydroxyethyl; and

- $R_3$ is an atom or radical independently defined as $R_4$, or is amino or methoxy, or $R_4$ and $R_3$ together form an aryl radical being optionally substituted with one or more substituents $R_a$ each independently selected from the group consisting of amino, alkylamino, cycloalkylamino, alkenylamino, cycloalkenylamino, alkynylamino, arylamino, arylalkylamino, hydroxyalkylamino, mercaptoalkylamino, heterocyclic amino and heterocyclic-substituted alkylamino, wherein each substituent $R_a$ may further comprise one or more functions selected from the group consisting of carbonyl, amino and carboxyl, and wherein two adjacent substituents $R_a$ may together form an heterocyclic radical;

e) if $Y_1$ and $Y_2$ are both oxygen and none of $R_3$ and $R_4$ is hydrogen, then:

- $R_1$ is a radical selected from the group consisting of $C_{2-7}$ alkyl; $C_{2-7}$ alkenyl; aryl; alkylaryl; ω-hydroxy $C_{1-7}$ alkyl; ω-epoxy $C_{1-7}$ alkyl; ω-carboxy $C_{1-7}$ alkyl (wherein the carboxy group may be acid, ester, thioester, acid halide or amide); ω-cyano $C_{1-7}$ alkyl; arylalkyl; arylalkenyl; heterocyclic-substituted alkyl; heterocyclic-substituted alkenyl; groups having the formula -S-R (i.e.

wherein a sulfur atom is attached to the nitrogen atom of the pteridine ring)

wherein R is a monovalent group selected from the group consisting of $C_{1-7}$ alkyl, aryl and $C_{3-10}$ cycloalkyl and wherein the said monovalent group is optionally substituted with one or more substituents selected from the group consisting of amino, amino-acid, alkylamino, arylamino, cycloalkylamino, carboxylic acid, carboxylic ester, sulfonic acid and phosphonic acid; and optionally substituted heterocyclic radicals;

- $R_2$ is hydrogen;
- $R_3$ is an atom or radical selected from the group consisting of chloro, fluoro; bromo; iodo; $C_{2-7}$alkyl; $C_{2-7}$ alkenyl; $C_{2-7}$ alkynyl; $C_{1-7}$ haloalkyl; $C_{1-4}$ alkoxy; $C_3$-$_{10}$ cycloalkoxy; aryloxy; arylalkyloxy; oxyheterocyclic; heterocyclic-substituted alkyloxy; thio $C_{1-7}$ alkyl; thio $C_{3-10}$ cycloalkyl; thioaryl; thioheterocyclic; arylalkylthio; heterocyclic-substituted alkylthio; hydroxylamino; acetal; carboxylic acid esters, thioesters and amides; thiocarboxylic acid; thiocarboxylic acid esters, thioesters and amides; sulfhydryl; amino; alkylamino; cycloalkylamino; alkenylamino; cycloalkenyl-amino; alkynylamino; arylamino; arylalkylamino; hydroxyalkylamino; mercaptoalkylamino; heterocyclic amino; heterocyclic-substituted alkylamino; hydrazino; alkylhydrazino; phenylhydrazino; cysteinyl acid, esters or amides; aryl substituted with one or more substituents selected from the group consisting of halogen, $C_{1-4}$alkyl, $C_{2-7}$ alkenyl, $C_{2-7}$ alkynyl, $C_{1-4}$ haloalkyl, $C_{1-4}$ alkoxy, hydroxyl, sulfhydryl, amino, $C_{3-10}$ cycloalkoxy, aryloxy, arylalkyloxy, oxyheterocyclic, heterocyclic-substituted alkyloxy, thio $C_{1-7}$ alkyl, thio $C_{3-10}$ cycloalkyl, thioaryl, thioheterocyclic, arylalkylthio, heterocyclic-substituted alkylthio, formyl, hydroxylamino, cyano, carboxylic acid or esters or thioesters or amides thereof, thiocarboxylic acid or esters or thioesters or amides thereof, $C_{1-7}$ alkylamino, cycloalkylamino, alkenylamino, cycloalkenylamino, alkynylamino, arylamino, arylalkylamino, hydroxyalkylamino, mercaptoalkylamino, heterocyclic amino, hydrazino, alkylhydrazino and phenylhydrazino; optionally substituted heterocyclic radicals preferably selected from the group consisting of diazepinyl, oxadiazinyl, thiadiazinyl, dithiazinyl, triazolonyl, diazepinonyl, triazepinyl, triazepinonyl, tetrazepinonyl, benzoquinolinyl, benzothiazinyl, benzothiazinonyl, benzoxathiinyl, benzodioxinyl, benzodithiinyl, benzoxazepinyl, benzothiazepinyl, benzodiazepinyl, benzodioxepinyl, benzodithiepinyl, benzoxazocinyl, benzothiazocinyl, benzodiazocinyl, benzoxathiocinyl, benzodioxocinyl, benzotrioxepinyl, benzoxathiazepinyl, benzoxadiazepinyl, benzothiadiazepinyl, benzotriazepinyl, benzoxathiepinyl, benzotriazinonyl, benzoxazolinonyl, azetidinonyl, azaspiroundecyl, dithiaspirodecyl, selenazinyl, selenazolyl, selenophenyl, hypoxanthinyl, azahypoxanthinyl, bipyrazinyl, bipyridinyl, oxazolidinyl, diselenopyrimidinyl, benzodioxocinyl, benzopyrenyl, benzopyranonyl, benzophenazinyl, benzoquinolizinyl, dibenzocarbazolyl, dibenzoacridinyl, dibenzophenazinyl,

dibenzothiepinyl, dibenzooxepinyl, dibenzopyranonyl, dibenzoquinoxalinyl, dibenzothiazepinyl, dibenzoisoquinolinyl, tetraazaadamantyl, thiatetraazaadamantyl, oxauracil, oxazinyl, dibenzothiophenyl, dibenzofuranyl, oxazolinyl, oxazolonyl, azaindolyl, azolonyl, thiazolinyl, thiazolonyl, thiazolidinyl, thiazanyl, pyrimidonyl, thiopyrimidonyl, thiamorpholinyl, azlactonyl, naphtindazolyl, naphtindolyl, naphtothiazolyl, naphtothioxolyl, naphtoxindolyl, naphtotriazolyl, naphtopyranyl, indolinyl, indolizidinyl, tetrahydrofuryl, oxabicycloheptyl, azabenzimidazolyl, azacycloheptyl, azacyclooctyl, azacyclononyl, azabicyclononyl, tetrahydropyranyl, tetra-hydropyronyl, tetrahydroquinoleinyl, tetrahydrothienyl and dioxide thereof, dihydrothienyl dioxide, dioxindolyl, dioxinyl, dioxenyl, dioxazinyl, thioxanyl, thioxolyl, thio-urazolyl, thiotriazolyl, thiopyranyl, thiopyronyl, coumarinyl, quinoleinyl, oxyquinoleinyl, quinuclidinyl, xanthinyl, dihydropyranyl, benzodihydrofuryl, benzothiopyronyl, benzothiopyranyl, benzoxazinyl, benzoxazolyl, benzodioxolyl, benzodioxanyl, benzothiadiazolyl, benzotriazinyl, benzothiazolyl, benzoxazolyl, phenothioxinyl, phenothiazolyl, phenothienyl, phenopyronyl, phenoxazolyl, pyridinyl, dihydropyridinyl, tetrahydropyridinyl, piperidinyl, morpholinyl, thiomorpholinyl, pyrazinyl, pyrimidinyl, pyridazinyl, triazinyl, tetrazinyl, triazolyl, benzotriazolyl, tetrazolyl, imidazolyl, pyrazolyl, thiazolyl, thiadiazolyl, isothiazolyl, oxazolyl, oxadiazolyl, pyrrolyl, furyl, dihydrofuryl, furoyl, hydantoinyl, dioxolyl, dithianyl, dithienyl, dithiinyl, thienyl, indolyl, indazolyl, benzofuryl, benzothienyl, quinolyl, quinazolinyl, quinoxalinyl, carbazolyl, phenoxazinyl, phenothiazinyl, xanthenyl, purinyl, benzothienyl, naphtothienyl, thianthrenyl, pyranyl, pyronyl, benzopyronyl, isobenzofuranyl, chromenyl, phenoxathiinyl, indolizinyl, quinolizinyl, isoquinolyl, phthalazinyl, naphthiridinyl, cinnolinyl, pteridinyl, carbolinyl, acridinyl, perimidinyl, phenanthrolinyl, phenazinyl, phenothiazinyl, imidazolinyl, imidazolidinyl, benzimidazolyl, pyrazolinyl, pyrazolidinyl, pyrrolinyl, pyrrolidinyl, piperazinyl, uridinyl, thymidinyl,cytidinyl, azirinyl, aziridinyl, diazirinyl, diaziridinyl, oxiranyl, oxaziridinyl, dioxiranyl, thiiranyl, azetyl, dihydroazetyl, azetidinyl, oxetyl, oxetanyl, thietyl and thietanyl; aryl or heterocyclic radicals substituted with an aliphatic spacer between the pteridine ring and the aryl or heterocyclic radical, whereby said aliphatic spacer is a branched or straight, saturated or unsaturated aliphatic chain of 1 to 4 carbon atoms (e.g. $C_{1-4}$ alkylene) which may contain one or more functions, atoms or radicals selected from the group consisting of carbonyl (oxo), alcohol (hydroxyl), ether, acetal, amino, imino, oximino, alkyloximino, amino-acid, cyano, carboxylic acid or ester or thioester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxyalkylamino, mercaptoalkylamino, heterocyclic amino, hydrazino, alkylhydrazino, phenylhydrazino, sulfonyl, sulfonamido and halogen; branched or straight, saturated or unsaturated aliphatic chain of 1 to 7 carbon atoms containing one or more atoms, functions or radicals selected from the group consisting of carbonyl (oxo), alcohol (hydroxyl), ether, acetal, amino, imino, oximino, alkyloximino, amino-acid, cyano, carboxylic acid ester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxylalkylamino, mercaptoalkyl-amino, heterocyclic amino, hydrazino, alkylhydrazino, phenylhydrazino, sulfonyl, sulfonamido and halogen; hydroxyethyl; and

- $R_4$ is an atom or radical independently defined as $R_3$, or is chloro, or $R_4$ and $R_3$ together form an aryl radical being optionally substituted with one or more substituents $R_a$ each independently selected from the group consisting of amino, alkylamino, cycloalkylamino, alkenylamino, cycloalkenylamino, alkynylamino, arylamino, arylalkylamino, hydroxyalkylamino, mercaptoalkylamino, heterocyclic amino and heterocyclic-substituted alkylamino, wherein each substituent $R_a$ may further comprise one or more functions selected from the group consisting of carbonyl, amino and carboxyl, and wherein two adjacent substituents $R_a$ may together form an heterocyclic radical;

f) if $Y_1$ and $Y_2$ are both oxygen and $R_2$ and $R_3$ are both hydrogen, then:

- $R_1$ is a radical selected from the group consisting of $C_{1-7}$ alkyl; $C_{2-7}$ alkenyl; aryl; alkylaryl; ω-hydroxy $C_{1-7}$ alkyl; ω-epoxy $C_{1-7}$ alkyl; ω-carboxy $C_{1-7}$ alkyl (wherein the carboxy group may be acid, ester, thioester, acid halide or amide); ω-cyano $C_{1-7}$ alkyl; arylalkyl; arylalkenyl; heterocyclic-substituted alkyl; heterocyclic-substituted alkenyl; groups having the formula -S-R (i.e.

wherein a sulfur atom is attached to the nitrogen atom of the pteridine ring)

wherein R is a monovalent group selected from the group consisting of $C_{1-7}$ alkyl, aryl and $C_{3-10}$ cycloalkyl and wherein the said monovalent group is optionally substituted with one or more substituents selected from the group consisting of amino, amino-acid, alkylamino, arylamino, cycloalkylamino, carboxylic acid, carboxylic ester, sulfonic acid and phosphonic acid; and optionally substituted heterocyclic radicals;

- $R_4$ is an atom or a radical selected from the group consisting of halogen (preferably chloro), cyano, amino, alkylamino, cycloalkylamino, alkenylamino, cycloalkenylamino, alkynylamino, arylamino, arylalkylamino, hydroxyalkylamino, mercaptoalkylamino, heterocyclic amino and heterocyclic-substituted alkylamino; or a pharmaceutically acceptable salt or an enantiomer thereof.

Within the scope of this invention, the term " substituted heterocyclic radicals" include such groups represented by the general formula:

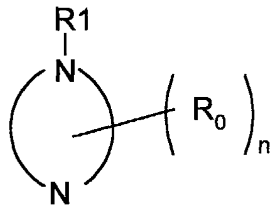 (II)

wherein:

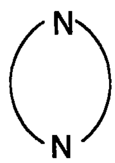 (III)

schematically represents a saturated or partly unsaturated heterocyclic ring with at least two nitrogen atoms in the said heterocyclic ring and with a total of 5 to 7 atoms in the said heterocyclic ring, and optionally with one or more other heteroatoms (e.g. oxygen or sulfur) in the said heterocyclic ring or attached to one or more carbon atoms of said heterocyclic ring (for instance in the form of a carbonyl or thiocarbonyl group), wherein one of said at least two nitrogen atoms in the heterocyclic ring is attached to a carbon atom of the pteridine ring at any of positions 2, 4, 6 or 7 of the pteridine ring, wherein the said heterocyclic ring may be fused to one or more aromatic hydrocarbon rings, and wherein:

- $R_0$ is a group independently selected from the group consisting of halogen, nitro, $C_{1-7}$ alkyl (optionally containing one or more functions or radicals selected from the group consisting of halogen, carbonyl, thiocarbonyl, hydroxyl, sulfhydryl, $C_{1-7}$ alkoxy, thio $C_{1-7}$ alkyl, thio $C_{3-10}$ cycloalkyl, acetal, thioacetal, imino, oximino, alkyloximino, amino-acid, cyano, (thio)carboxylic acid, (thio)carboxylic acid ester or amide, nitro, amino, $C_{1-7}$ alkylamino, cycloalkylamino, alkenylamino, cycloalkenylamino, alkynyl-amino, arylamino, arylalkylamino, hydroxyalkylamino, mercapto-alkylamino, heterocyclic amino, hydrazino, alkylhydrazino, phenyl-hydrazino, sulfonyl and sulfonamido), $C_{2-7}$ alkenyl, $C_{2-7}$ alkynyl, halo $C_{1-7}$ alkyl, $C_{3-10}$ cycloalkyl, aryl, arylalkyl, alkylaryl, alkylacyl, arylacyl , hydroxyl, sulfhydryl, amino, $C_{1-7}$ alkylamino, cycloalkylamino, alkenylamino, cyclo-alkenylamino, alkynylamino, arylamino, arylalkylamino, hydroxyalkylamino, mercaptoalkylamino, heterocyclic amino, hydrazino, alkylhydrazino, phenylhydrazino, $C_{1-7}$ alkoxy, $C_{3-10}$ cycloalkoxy, aryloxy, arylalkyloxy, oxyheterocyclic, heterocyclic-substituted alkyloxy, thio $C_{1-7}$ alkyl, thio $C_{3-10}$ cycloalkyl, thioaryl, thioheterocyclic, arylalkylthio, heterocyclic-substituted alkylthio, formyl, hydroxylamino, cyano, (thio)carboxylic acid or esters or thioesters or amides or thioamides thereof;
- n is an integer from 0 to 6; and
- $R^1$ is a group selected from the group consisting of formyl, acyl, thio-acyl, amide, thioamide, sulfonyl, sulfinyl, carboxylate, thiocarboxylate and amino-substituted acyl.

  For easiness of understanding the set of compounds of general formula (I) may be sub-divided into six subsets of compounds, depending upon (i) the nature of $Y_1$ and $Y_2$, and (ii) when $Y_1$ and $Y_2$ are both oxygen, upon the number and location of substituents on the pteridine ring. More specifically, these six sub-sets of compounds of general formula (I) are as follows:

  a) trisubstituted lumazines wherein substitutions are in positions 1, 3 and 6 on the pteridine ring,
  b) trisubstituted lumazines wherein substitutions are in positions 1, 3 and 7 on the pteridine ring,
  c) mono- and poly-substituted 2-thiolumazines, 4-thiolumazines and 2,4-dithiolumazines,
  d) tetrasubstituted lumazines,
  e) trisubstituted lumazines wherein substitutions are in positions 3, 6 and 7 on the pteridine ring, and
  f) disubstituted lumazines wherein substitutions are in positions 1 and 7 on the pteridine ring. It should be

noted, however, that these sub-sets of novel compounds have in common the structural features present in the general formula (I). They also have a potential specific biological activity profile and consequent usefulness in medicinal chemistry.

In a second embodiment, the present invention relates to the unexpected finding that at least one desirable biological property such as, but not limited to, the ability to decrease the proliferation of lymphocytes, or to decrease T-cell activation, or to decrease B-cell or monocytes or macrophages activation, or to inhibit TNF-$\alpha$ and IL-1 release, is a common feature which is not only present in the group of novel compounds defined in the general formula (I), but also in a group of polysubstituted pteridine-2,4-diones (lumazines), as well as mono- and poly-substituted 2-thiolumazines, 4-thiolumazines and 2,4-dithiolumazines which is broader than the said group of novel compounds, specifically in a group of compounds represented by the general formula (IV):

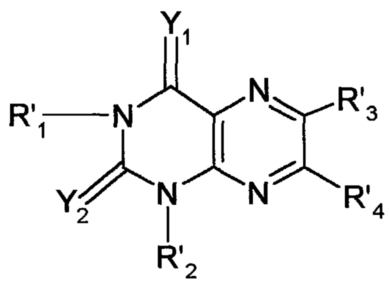

wherein:

- each of $Y_1$ and $Y_2$ is independently selected from sulfur and oxygen;
- each of $R'_1$ and $R'_2$ is a radical independently selected from the group consisting of hydrogen; $C_{1-7}$ alkyl; $C_{2-7}$ alkenyl; aryl; alkylaryl; $\omega$-hydroxy $C_{1-7}$ alkyl; $\omega$-epoxy $C_{1-7}$ alkyl; $\omega$-carboxy $C_{1-7}$ alkyl (wherein the carboxy group may be acid, ester, thioester, acid halide or amide); $\omega$-cyano $C_{1-7}$ alkyl; arylalkyl; arylalkenyl; heterocyclic-substituted alkyl; heterocyclic-substituted alkenyl; groups having the formula -S-R (i.e. wherein a sulfur atom is attached to the nitrogen atom of the pteridine ring) wherein R is a monovalent group selected from the group consisting of $C_{1-7}$ alkyl, aryl and $C_{3-10}$ cycloalkyl and wherein the said monovalent group is optionally substituted with one or more substituents selected from the group consisting of amino, amino-acid, alkylamino, arylamino, cycloalkylamino, carboxylic acid, carboxylic ester, sulfonic acid and phosphonic acid; and optionally substituted heterocyclic radicals;
- at most one of $R_1'$, $R'_2$, $R'_3$ and $R'_4$ is hydrogen when one or more of $Y_1$ and $Y_2$ is sulfur;
- at most one of $R'_1$ and $R'_2$ is hydrogen when both $Y_1$ and $Y_2$ are oxygen;
- each of $R'_3$ and $R'_4$ is an atom or radical independently selected from the group consisting of hydrogen; halogen; $C_{1-4}$ alkyl; $C_{2-7}$ alkenyl; $C_{2-7}$ alkynyl; halo $C_{1-4}$ alkyl; $C_{1-4}$ alkoxy; $C_{3-10}$ cycloalkoxy; aryloxy; arylalkyloxy; oxyheterocyclic; heterocyclic-substituted alkyloxy; thio $C_{1-7}$ alkyl; thio $C_{3-10}$ cycloalkyl; thioaryl; thioheterocyclic; arylalkylthio; heterocyclic-substituted alkylthio; hydroxylamino; acetal; formyl; cyano; carboxylic acid; carboxylic acid esters, thioesters and amides; thiocarboxylic acid; thiocarboxylic acid esters, thioesters and amides; amino; $C_{2-7}$ alkylamino; cycloalkylamino; alkenylamino; cycloalkenylamino; alkynylamino; arylamino; arylalkylamino; hydroxyalkylamino; mercaptoalkylamino; heterocyclic amino; heterocyclic-substituted alkylamino; oximino; alkyloximino; hydrazino; alkylhydrazino; phenylhydrazino; cysteinyl acid, esters or amides; aryl optionally substituted with one or more substituents selected from the group consisting of halogen, $C_{1-7}$ alkyl, $C_{2-7}$ alkenyl, $C_{2-7}$ alkynyl, halo $C_{1-7}$ alkyl, $C_{1-7}$ alkoxy, hydroxyl, sulfhydryl, amino, $C_{3-10}$ cycloalkoxy, aryloxy, arylalkyloxy, oxyheterocyclic, heterocyclic-substituted alkyloxy, thio $C_{1-7}$ alkyl, thio $C_{3-10}$ cycloalkyl, thioaryl, thioheterocyclic, arylalkylthio, heterocyclic-substituted alkylthio, formyl, hydroxylamino, cyano, carboxylic acid or esters or thioesters or amides thereof, thiocarboxylic acid or esters or thioesters or amides thereof, $C_{1-7}$ alkylamino, cycloalkylamino, alkenylamino, cycloalkenylamino, alkynylamino, arylamino, arylalkylamino, hydroxyalkylamino, mercaptoalkylamino, heterocyclic amino, hydrazino, alkylhydrazino and phenylhydrazino; optionally substituted heterocyclic radicals; aryl or heterocyclic radicals substituted with an aliphatic spacer between the pteridine ring and the aryl or heterocyclic radical, whereby said aliphatic spacer (linking group) is a branched or straight, saturated or unsaturated aliphatic chain of 1 to 4 carbon atoms (e.g. a $C_{1-4}$ alkylene) which may contain one or more functions, atoms or radicals selected from the group consisting of carbonyl (oxo), alcohol (hydroxyl), ether, acetal, amino, imino, oximino, alkyloximino, amino-acid, cyano, carboxylic acid or ester or thioester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxyalkylamino,

mercaptoalkylamino, hetero-cyclic amino, hydrazino, alkylhydrazino, phenylhydrazino, sulfonyl, sulfonamido and halogen; branched or straight, saturated or unsaturated aliphatic chain of 1 to 7 carbon atoms containing one or more atoms, functions or radicals selected from the group consisting of carbonyl (oxo), alcohol (hydroxyl), ether, acetal, amino, imino, oximino, alkyloximino, amino-acid, cyano, carboxylic acid ester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxyalkylamino, mercaptoalkylamino, heterocyclic amino, hydrazino, alkylhydrazino, phenylhydrazino, sulfonyl, sulfonamido and halogen, and

- $R'_4$ and $R'_3$ may together form an aryl radical being optionally substituted with one or more substituents $R_a$ each independently selected from the group consisting of amino, alkylamino, cycloalkylamino, alkenylamino, cycloalkenylamino, alkynylamino, arylamino, arylalkylamino, hydroxyalkylamino, mercaptoalkylamino, heterocyclic amino and heterocyclic-substituted alkylamino, wherein each substituent $R_a$ may further comprise one or more functions selected from the group consisting of carbonyl, amino and carboxyl, and wherein two adjacent substituents $R_a$ may together form an heterocyclic radical.

Compounds of formula (IV) are highly active immunosuppressive agents, antineoplastic agents or anti-viral agents which, together with one or more pharmaceutically acceptable carriers, may be formulated into pharmaceutical compositions for the prevention or treatment of pathologic conditions such as, but not limited to, immune and autoimmune disorders, organ and cells transplant rejections, cell proliferative disorders, cardiovascular disorders, disorders of the central nervous system and viral diseases.

In a further embodiment, the present invention relates to combined preparations containing at least one compound of formula (IV) and one or more drugs such as immunosuppressant and/or immunomodulator drugs, antineoplastic drugs, or antiviral agents. In a further embodiment, the present invention relates to the prevention or treatment of the above-cited pathologic conditions by administering to the patient in need thereof an effective amount of a compound of general formula (IV), optionally in the form of a pharmaceutical composition or combined preparation with another drug.

In a still further embodiment, the present invention relates to processes and methods for making the novel poly-substituted pteridine-2,4-diones (lumazines), as well as the novel mono- and poly-substituted 2-thiolumazines, 4-thiolumazines and 2,4-dithiolumazines defined in general formula (I) and their pharmaceutically acceptable salts and enantiomers.

## BRIEF DESCRIPTION OF THE DRAWINGS

Figure 1 represents a scheme for replacing a methyl substituent $R_3$ with an unsaturated partly aliphatic chain or spacer in the 6-position of the pteridine ring of a substituted lumazine.

Figure 2 represents a scheme for replacing a carboxylic substituent $R_3$ in a substituted lumazine.

Figure 3 represents a scheme for replacing a hydroxyl group with an aryl substituent $R_4$ in the 7-position of the pteridine ring of a substituted lumazine.

Figure 4 represents a scheme for performing various alterations of a substituent $R_4$ on a substituted lumazine wherein the said $R_4$ is either methyl or formyl.

Figure 5 represents a scheme for performing various alterations of a substituent $R_3$ starting from a substituted 6-formyllumazine.

Figure 6 represents a scheme for preparing a polysubstituted lumazine
wherein the substituent $R_4$ is either alkylaryl or hydroxyl and then performing various alterations thereon.

Figure 7 represents a scheme for successively introducing substituent $R_2$ and $R_1$ substituents into a poly-substituted lumazine.

Figure 8 represents a scheme for preparing 1-substituted-5,6-diaminouracil derivatives.

Figure 9 represents a scheme for preparing a 1,3,6-trisubstituted lumazine with a halogen at position 7 of the pteridine ring.

Figure 10 represents a scheme for introducing a substituent $R_2$ or $R_1$ onto a polysubstituted lumazine.

Figure 11 represents a scheme for altering the side chain at position 3 of the pteridine ring of a polysubstituted lumazine.

## DEFINITIONS

As used herein, the terms " lumazine " and " pteridine-2,4-dione " are interchangeable and designate any of the tautomeric forms of 2,4-dioxopteridine or 2,4-dihydroxypteridine; the term " 2-thiolumazine " designate any of the

tautomeric forms of 2-thioxo-4-oxo-pteridine; the term " 4-thiolumazine " designate any of the tautomeric forms of 2-oxo-4-thioxo-pteridine; the term " 2,4-dithiolumazine " designate any of the tautomeric forms of 2,4-dithioxo-pteridine; the term " (thio)lumazine " is a generic abbreviation for all sub-sets of compounds designated herein-above. Unless otherwise stated herein, the term " polysubstituted " means that two, three or four of the carbon atoms (thus respectively " disubstituted ", " trisubstituted or " tetrasubstituted ") being in any of positions 1, 3, 6 and 7 (according to standard atom numbering for the pteridine ring) are substituted with an atom or group other than hydrogen. The term "monosubstituted " means that only one of the carbon atoms being in positions 1, 3, 6 or 7 is substituted with an atom or group other than hydrogen.

As used herein with respect to a substituting group and unless otherwise stated, the term " $C_{1-7}$ alkyl" means straight and branched chain saturated acyclic hydrocarbon monovalent radicals or groups having from 1 to 7 carbon atoms such as, for example, methyl, ethyl, propyl, n-butyl, 1-methylethyl (isopropyl), 2-methylpropyl (isobutyl), 1,1-dimethylethyl (ter-butyl), 2-methylbutyl, n-pentyl, dimethylpropyl, n-hexyl, 2-methylpentyl, 3-methylpentyl, n-heptyl and the like; optionally the carbon chain length of such group may be extended to 20 carbon atoms; the term " $C_{1-4}$ alkyl " designate the corresponding radicals or groups with only 1 to 4 carbon atoms, and so on.

As used herein with respect to a linking group and unless otherwise stated, the term " $C_{1-7}$ alkylene " means the divalent hydrocarbon radical corresponding to the above defined $C_{1-7}$ alkyl, such as methylene, bis(methylene), tris(methylene), tetramethylene, hexamethylene and the like.

As used herein with respect to a substituting group and unless otherwise stated, the term " $C_{3-10}$ cycloalkyl" means a mono- or polycyclic saturated hydrocarbon monovalent radical having from 3 to 10 carbon atoms, such as for instance cyclopropyl, cyclobutyl, cyclopentyl, cyclohexyl, cycloheptyl, cyclooctyl and the like, or a $C_{7-10}$ polycyclic saturated hydrocarbon monovalent radical having from 7 to 10 carbon atoms such as, for instance, norbornyl, fenchyl, trimethyltricycloheptyl or adamantyl; the term " $C_{5-8}$ cycloalkyl " designate the corresponding radicals or groups with 5 to 8 carbon atoms, and so on.

As used herein with respect to a linking group and unless otherwise stated, the term " $C_{3-10}$ cycloalkylene " means the divalent hydrocarbon radical corresponding to the above defined $C_{3-10}$ cycloalkyl, such as 1,2-cyclohexylene and 1,4-cyclohexylene.

As used herein with respect to a substituting group and unless otherwise stated, the term " aryl " designate any mono- or polyaromatic monovalent hydrocarbon radical having from 6 up to 30 carbon atoms such as but not limited to phenyl, naphthyl, anthracenyl, phenantracyl, fluoranthenyl, chrysenyl, pyrenyl, biphenylyl, terphenyl, picenyl, indenyl, indacenyl, benzocyclobutenyl, benzocyclooctenyl and the like, including fused benzo - $C_{5-8}$ cycloalkyl radicals (the latter being as defined above) such as, for instance, indanyl, tetrahydronaphtyl, fluorenyl and the like, all of the said radicals being optionally substituted with one or more substituents selected from the group consisting of halogen, amino, halo $C_{1-7}$ alkyl, hydroxyl, sulfhydryl and nitro, such as for instance 4-fluorophenyl, 4-chlorophenyl, 3,4-dichlorophenyl, 2,6-diisopropyl-4-bromophenyl, pentafluorophenyl, 4-trifluoromethylphenyl and 4-cyanophenyl.

As used herein with respect to a linking group, and unless otherwise stated, the term "arylene" means the divalent hydrocarbon radical corresponding to the above defined aryl, such as phenylene, naphtylene and the like.

As used herein with respect to a substituting group and unless otherwise stated, the term " heterocyclic " means a mono- or polycyclic, saturated or monounsaturated or polyunsaturated monovalent hydrocarbon radical having from 2 up to 15 carbon atoms and including one or more heteroatoms in one or more heterocyclic rings, each of said rings having from 3 to 10 atoms (and optionally further including one or more heteroatoms attached to one or more carbon atoms of said ring, for instance in the form of a carbonyl or thiocarbonyl or selenocarbonyl group and/or to one or more heteroatoms of said ring, for instance in the form of a sulfone, sulfoxide, N-oxide, phosphate, phosphonate or selenium oxide group), each of said heteroatoms being independently selected from the group consisting of nitrogen, oxygen, sulfur, selenium and phosphorus, also including radicals wherein a heterocyclic ring is fused to one or more aromatic hydrocarbon rings for instance in the form of benzo-fused, dibenzo-fused and naphto-fused heterocyclic radicals; within this definition are included heterocyclic radicals such as, but not limited to, diazepinyl, oxadiazinyl, thiadiazinyl, dithiazinyl, triazolonyl, diazepinonyl, triazepinyl, triazepinonyl, tetrazepinonyl, benzoquinolinyl, benzothiazinyl, benzothiazinonyl, benzoxathiinyl, benzodioxinyl, benzodithiinyl, benzoxazepinyl, benzothiazepinyl, benzodiazepinyl, benzodioxepinyl, benzodithiepinyl, benzoxazocinyl, benzothiazocinyl, benzodiazocinyl, benzoxathiocinyl, benzodioxocinyl, benzotrioxepinyl, benzoxathiazepinyl, benzoxadiazepinyl, benzothiadiazepinyl, benzotriazepinyl, benzoxathiepinyl, benzotriazinonyl, benzoxazolinonyl, azetidinonyl, azaspiroundecyl, dithiaspirodecyl, selenazinyl, selenazolyl, selenophenyl, hypoxanthinyl, azahypoxanthinyl, bipyrazinyl, bipyridinyl, oxazolidinyl, diselenopyrimidinyl, benzodioxocinyl, benzopyrenyl, benzopyranonyl, benzophenazinyl, benzoquinolizinyl, dibenzocarbazolyl, dibenzoacridinyl, dibenzophenazinyl, dibenzothiepinyl, dibenzooxepinyl, dibenzopyranonyl, dibenzoquinoxalinyl, dibenzothiazepinyl, dibenzoisoquinolinyl, tetraazaadamantyl, thiatetraazaadamantyl, oxauracil, oxazinyl, dibenzothiophenyl, dibenzofuranyl, oxazolinyl, oxazolonyl, azaindolyl, azolonyl, thiazolinyl, thiazolonyl, thiazolidinyl, thiazanyl, pyrimidonyl, thiopyrimidonyl, thiamorpholinyl, azlactonyl, naphtindazolyl, naphtindolyl, naphtothiazolyl, naphtothioxolyl, naphtoxindolyl, naphtotriazolyl, naphtopyranyl, indolinyl, indolizidinyl, oxabicycloheptyl, azabenzimidazolyl, azacycloheptyl, azacyclooctyl, azacyclononyl, azabicyclononyl, tetrahydrofuryl, tetrahydropyranyl, tetrahydropyronyl, tetrahydroquinoleinyl, tetrahydrothienyl and dioxide thereof, dihydrothienyl dioxide, dioxindolyl, dioxinyl, dioxenyl, dioxazinyl, thioxanyl, thioxolyl, thio-urazolyl, thiotriazolyl, thiopyranyl, thiopyronyl, coumarinyl, quinoleinyl, oxyquinoleinyl, quinuclidinyl, xanthinyl, dihydropyranyl, benzodihydrofuryl, benzothiopyronyl, benzothiopyranyl, benzoxazinyl, benzoxazolyl, benzodioxolyl, benzodioxanyl, benzothiadiazolyl, benzotriazinyl, benzothiazolyl, benzoxazolyl, phenothioxinyl, phenothiazolyl, phenothienyl, phenopyronyl, phenoxazolyl, pyridinyl, dihydropyridinyl, tetrahydropyridinyl, piperidinyl, morpholinyl, thiomorpholinyl, pyrazinyl, pyrimidinyl, pyridazinyl, triazinyl, tetrazinyl, triazolyl, benzotriazolyl, tetrazolyl, imidazolyl, pyrazolyl, thiazolyl, thiadiazolyl, isothiazolyl, oxazolyl, oxadiazolyl, pyrrolyl, furyl, dihydrofuryl, furoyl, hydantoinyl, dioxolanyl, dioxolyl, dithianyl, dithienyl, dithiinyl, thienyl, indolyl, indazolyl, benzofuryl, quinolyl, quinazolinyl, quinoxalinyl, carbazolyl, phenoxazinyl, phenothiazinyl, xanthenyl, purinyl, benzothienyl, naphtothienyl, thianthrenyl, pyranyl, pyronyl, benzopyronyl, isobenzofuranyl, chromenyl, phenoxathiinyl, indolizinyl, quinolizinyl, isoquinolyl, phthalazinyl, naphthiridinyl, cinnolinyl, pteridinyl, carbolinyl, acridinyl, perimidinyl, phenanthrolinyl, phenazinyl, phenothiazinyl, imidazolinyl, imidazolidinyl, benzimidazolyl, pyrazolinyl, pyrazolidinyl, pyrrolinyl, pyrrolidinyl, piperazinyl, uridinyl, thymidinyl, cytidinyl, azirinyl, aziridinyl, diazirinyl, diaziridinyl, oxiranyl, oxaziridinyl, dioxiranyl, thiiranyl, azetyl, dihydroazetyl, azetidinyl, oxetyl, oxetanyl, thietyl, thietanyl and the like, including all possible isomeric forms thereof, wherein each carbon atom of the said heterocyclic ring may be independently substituted with a substituent selected from the group consisting of halogen, nitro, $C_{1-7}$ alkyl (optionally containing one or more functions or radicals selected from the group consisting of carbonyl (oxo), alcohol (hydroxyl), ether (alkoxy), acetal, amino, imino, oximino, alkyloximino, amino-acid, cyano, carboxylic acid ester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxylalkylamino, mercapto-alkylamino, heterocyclic amino, hydrazino, alkylhydrazino, phenyl-hydrazino, sulfonyl, sulfonamido and halogen), $C_{3-7}$ alkenyl, $C_{2-7}$ alkynyl, halo $C_{1-7}$ alkyl, $C_{3-10}$ cycloalkyl, aryl, arylalkyl, alkylaryl, alkylacyl, arylacyl, hydroxyl, amino, $C_{1-7}$ alkylamino, cycloalkylamino, alkenylamino, cyclo-alkenylamino, alkynylamino, arylamino, arylalkylamino, hydroxyalkylamino, mercaptoalkylamino, heterocyclic amino, hydrazino, alkylhydrazino, phenylhydrazino, sulfhydryl, $C_{1-7}$ alkoxy, $C_{3-10}$ cycloalkoxy, aryloxy, arylalkyloxy, oxyheterocyclic, heterocyclic-substituted alkyloxy; thio $C_{1-7}$ alkyl, thio $C_{3-10}$ cycloalkyl, thioaryl, thioheterocyclic, arylalkylthio, heterocyclic-substituted alkylthio, formyl, hydroxylamino, cyano, carboxylic acid or esters or thioesters or amides thereof, thiocarboxylic acid or esters or thioesters or amides thereof; depending upon the number of unsaturations in the heterocyclic ring having 3 to 10 atoms, heterocyclic radicals may be sub-divided into heteroaromatic (or " heteroaryl ") radicals and non-aromatic heterocyclic radicals; when a heteroatom of the said non-aromatic heterocyclic radical is nitrogen, the latter may be substituted with a substituent selected from the group consisting of $C_{1-7}$ alkyl, $C_{3-10}$ cycloalkyl, aryl, arylalkyl, alkylaryl, alkylacyl and arylacyl.

As used herein with respect to a substituting group and unless otherwise stated, the terms " $C_{1-7}$ alkoxy ", " $C_{2-7}$ alkenyloxy ", " $C_{2-7}$ alkynyloxy", " $C_{3-10}$ cycloalkoxy ", " aryloxy", "arylalkyloxy ", " oxyheterocyclic ", "thio $C_{1-7}$ alkyl", " thio $C_{3-10}$ cycloalkyl ", " arylthio ", " arylalkylthio " and "thioheterocyclic" refer to substituents wherein a $C_{1-7}$ alkyl, $C_{2-7}$ alkenyl or $C_{2-7}$ alkynyl (optionally the carbon chain length of such group may be extended to 20 carbon atoms), respectively a $C_{3-10}$ cycloalkyl, aryl, arylalkyl or heterocyclic radical (each of them such as defined herein), are attached to an oxygen atom or a divalent sulfur atom through a single bond, such as but not limited to methoxy, ethoxy, propoxy, butoxy, pentoxy, isopropoxy, sec-butoxy, tert-butoxy, isopentoxy, cyclopropyloxy, cyclobutyloxy, cyclopentyloxy, thiomethyl, thioethyl, thiopropyl, thiobutyl, thiopentyl, thiocyclopropyl, thiocyclobutyl, thiocyclopentyl, thiophenyl, cresoxy, phenyloxy, benzyloxy, mercaptobenzyl and the like.

As used herein with respect to a substituting atom and unless otherwise stated, the term halogen means any atom selected from the group consisting of fluorine, chlorine, bromine and iodine.

As used herein with respect to a substituting group and unless otherwise stated, the term " halo $C_{1-7}$ alkyl " means a $C_{1-7}$ alkyl radical (such as above defined, i.e. optionally the carbon chain length of such group may be extended to 20 carbon atoms) in which one or more hydrogen atoms are independently replaced by one or more halogens (preferably fluorine, chlorine or bromine), such as but not limited to difluoromethyl, trifluoromethyl, trifluoroethyl, octafluoropentyl, dodecafluoroheptyl, dichloromethyl and the like; the term " halo $C_{1-4}$ alkyl " designate the corresponding radical with only 1 to 4 carbon atoms, and so on.

As used herein with respect to a substituting group and unless otherwise stated, the terms " $C_{2-7}$ alkenyl " and " aliphatic unsaturated hydrocarbon radical with 2 to 7 carbon atoms " are interchangeable and designate a straight or branched acyclic hydrocarbon monovalent radical having one or more ethylenical unsaturations and having from 2 to 7 carbon atoms such as, for example, vinyl, 2-propenyl, 3-butenyl, 2-butenyl, 2-pentenyl, 3-pentenyl, 3-methyl-2-butenyl, 3-hexenyl, 2-hexenyl, 2-heptenyl, butadienyl, pentadienyl, hexadienyl, heptadienyl, heptatrienyl and the like, including all possible isomers thereof; optionally the carbon chain length of such group may be extended to 20 carbon atoms; the term " $C_{3-7}$ alkenyl " designate the corresponding radical with only 3 to 7 carbon atoms, and so on.

As used herein with respect to a substituting group and unless otherwise stated, the terms " $C_{3-10}$ cycloalkenyl" or " cycloaliphatic unsaturated hydrocarbon radicals with 3 to 10 carbon atoms " means a monocyclic mono- or polyunsaturated hydrocarbon monovalent radical having from 3 to 8 carbon atoms, such as for instance cyclopropenyl,

cyclobutenyl, cyclopentenyl, cyclopentadienyl, cyclohexenyl, cyclohexadienyl, cycloheptenyl, cycloheptadienyl, cycloheptatrienyl, cyclooctenyl, cyclooctadienyl, cyclooctatrienyl, 1,3,5,7-cyclooctatetraenyl and the like, or a $C_{7-10}$ polycyclic mono- or polyunsaturated hydrocarbon mono-valent radical having from 7 to 10 carbon atoms such as dicyclopentadienyl, fenchenyl (including all isomers thereof, such as $\alpha$-pinolenyl), bicyclo[2.2.1 ]hept-2-enyl, bicyclo[2.2.1 ] hepta-2,5-dienyl, cyclo-fenchenyl and the like.

As used herein with respect to a substituting group and unless otherwise stated, the term " $C_{2-7}$ alkynyl " defines straight and branched chain hydrocarbon radicals containing one or more triple bonds and having from 2 to 20 carbon atoms such as, for example, acetylenyl, 2-propynyl, 3-butynyl, 2-butynyl, 2-pentynyl, 3-pentynyl, 3-methyl-2-butynyl, 3-hexynyl, 2-hexynyl and the like and all possible isomers thereof; optionally the carbon chain length of such group may be extended to 20 carbon atoms.

As used herein with respect to a substituting group and unless otherwise stated, the terms " arylalkyl ", "arylalkenyl ", "heterocyclic-substituted alkyl" and "heterocyclic-substituted alkenyl" refer to an aliphatic saturated or ethylenically unsaturated hydrocarbon monovalent radical (preferably a $C_{1-7}$ alkyl or $C_{3-10}$ cycloalkyl or $C_{2-7}$ alkenyl or $C_{3-10}$ cycloalkenyl such as defined above, i.e. optionally the carbon chain length of such group may be extended to 20 carbon atoms), onto which an aryl radical or respectively a heterocyclic radical (such as defined above) is already bonded, such as but not limited to benzyl, 2-phenylethyl, 2-phenylethenyl, pyridylmethyl, pyridylethyl, 2-(2-pyridyl) isopropyl, oxazolylbutyl, 2-thienylmethyl and 2-furylmethyl.

As used herein with respect to a substituting group and unless otherwise stated, the terms " alkylcycloalkyl ", "alkenyl(hetero)aryl ", "alkyl(hetero)aryl " , and "alkyl-substituted heterocyclic " refer respectively to an aryl, heteroaryl, cycloalkyl or heterocyclic radical (such as defined above) onto which are already bonded one or more aliphatic saturated or unsaturated hydrocarbon monovalent radicals, preferably one or more $C_{1-7}$ alkyl, $C_{2-7}$ alkenyl or $C_{3-10}$ cycloalkyl radicals as defined above, such as, but not limited to, o-toluyl, m-toluyl, p-toluyl, 2,3-xylyl, 2,4-xylyl, 3,4-xylyl, o-cumenyl, m-cumenyl, p-cumenyl, o-cymenyl, m-cymenyl, p-cymenyl, mesityl, lutidinyl (i.e. dimethylpyridyl), 2-methylaziridinyl, methylbenzimidazolyl, methylbenzofuranyl, methylbenzothiazolyl, methylbenzotriazolyl, methylbenzoxazolyl, methylcyclohexyl and menthyl.

As used herein with respect to a substituting group and unless otherwise stated, the terms " alkylamino ", "cycloalkylamino ", "alkenylamino " , " cycloalkenylamino " , " arylamino ", "arylalkylamino", "heterocyclic amino " , " hydroxyalkylamino ", "mercaptoalkyl-amino " and " alkynylamino" mean that one or even two $C_{1-7}$ alkyl, $C_{3-10}$ cycloalkyl, $C_{2-7}$ alkenyl, $C_{3-10}$ cycloalkenyl, aryl, arylalkyl, heterocyclic, hydroxy $C_{1-7}$ alkyl, mercapto $C_{1-7}$ alkyl or $C_{2-7}$ alkynyl radicals (each of them as defined herein, respectively) are attached to a nitrogen atom through a single bond or, in the case of heterocyclic, include a nitrogen atom, such as but not limited to, anilino, benzylamino, methylamino, dimethylamino, ethylamino, isopropylamino, propenylamino, n-butylamino, terbutylamino, dibutylamino, morpholino-alkylamino, morpholinyl, piperidinyl, piperazinyl, hydroxymethylamino and ethynylamino; this definition also includes mixed amino radicals wherein the nitrogen atom is attached to two such radicals belonging to two different sub-set of radicals, e.g. an alkyl radical and an alkenyl radical.

As used herein with respect to a substituting group and unless otherwise stated, the terms " (thio)carboxylic acid ester " , " (thio)carboxylic acid thioester " and " (thio)carboxylic acid amide" refer to radicals wherein the carboxyl or thiocarboxyl group is directly attached to the pteridine ring (e.g. in the 6- and/or 7-position) and wherein said carboxyl or thiocarboxyl group is bonded to the hydrocarbonyl residue of an alcohol, a thiol, a polyol, a phenol, a thiophenol, a primary or secondary amine, a polyamine, an amino-alcohol or ammonia, the said hydrocarbonyl residue being selected from the group consisting of alkyl, alkenyl, alkynyl, cycloalkyl, cycloalkenyl, aryl, arylalkyl, alkylaryl, alkylamino, cycloalkylamino, alkenylamino, cycloalkenylamino, arylamino, arylalkylamino, heterocyclic amino, hydroxyalkylamino, mercaptoalkylamino or alkynylamino (such as above defined, respectively).

As used herein with respect to a substituting group and unless otherwise stated, the term " amino-acid " refers to a radical derived from a molecule having the chemical formula $H_2N-CHR-COOH$, wherein R is the side group of atoms characterizing the amino-acid type; said molecule may be one of the 20 naturally-occurring amino-acids or any non naturally-occurring amino-acid.

As used herein and unless otherwise stated, the term " enantiomer " means each individual optically active form of a compound of the invention, having an optical purity or enantiomeric excess (as determined by methods standard in the art) of at least 80% (i.e. at least 90% of one enantiomer and at most 10% of the other enantiomer), preferably at least 90% and more preferably at least 98%.

DETAILED DESCRIPTION OF THE INVENTION

In one aspect, the present invention requires a 3-alkyl-6-aminouracil or a 3-alkenyl-6-uracil as a starting material. Since the procedure of WO 03/067257 achieves 3-methyl-6-aminouracil only with a poor yield, one aspect of the invention comprises a method of making a 3-alkyl-6-aminouracil or a 3-alkenyl-6-uracil in high yield by reacting, in the presence of a catalytic amount of ammonium sulfate, 6-aminouracil with a molar excess of an alkyl halide or alkenyl

halide (wherein the halide is preferably iodine, and the alkyl or alkenyl group may have from 1 to 7 carbon atoms) at reflux temperature in the presence of 1,1,1,3,3,3-hexamethyldisilazane as a solvent, and then separating the precipitate formed after quenching the reaction e.g. with sodium bicarbonate.

In the first embodiment of the invention, the novel poly-substituted pteridine-2,4-diones (lumazines), as well as the novel mono- and poly-substituted 2-thiolumazines, 4-thiolumazines and 2,4-dithiolumazines are as defined in the general formula (I), wherein each of the substituents $Y_1$, $Y_2$, $R_1$, $R_2$, $R_3$ and $R_4$, and optionally any of n, $R_0$ and $R^1$ , may correspond to any of the definitions given herein, in particular with any of the individual meanings (such as illustrated above) of generic terms such as but not limited to "$C_{1-7}$ alkyl", " $C_{2-7}$ alkenyl ", " $C_{2-7}$ alkynyl ", " aryl ", " heterocyclic ", " alkylaryl ", "arylalkyl ", "alkylamino", " cycloalkylamino ", " alkenylamino ", "alkynylamino", "arylamino", " arylalkylamino ", " $C_{1-7}$ alkoxy", " $C_{3-10}$ cycloalkoxy ", "thio $C_{1-7}$ alkyl ", " thio $C_{3-10}$ cycloalkyl ", " halo $C_{1-7}$ alkyl ", " amino-acid " and the like.

When a mixture of enantiomers of the pteridinediones (lumazines), 2-thiolumazines, 4-thiolumazines or 2,4-dithiolumazines having the general formula (I) according to the invention is obtained during their synthesis, the said mixture may be separated by means and methods standard in the art, e.g. liquid chromatography using one or more suitable chiral stationary phases. The latter include, for example, polysaccharides, in particular cellulose or amylose derivatives. Commercially available polysaccharide based chiral stationary phases are ChiralCel™ CA, OA, OB, OC, OD, OF, OG, OJ and OK, and Chiralpak™ AD, AS, OP(+) and OT(+). Appropriate eluents or mobile phases for use in combination with said polysaccharide chiral stationary phases are hydrocarbons such as hexane and the like, optionally admixed with an alcohol such as ethanol, isopropanol and the like. The above mixture of enantiomers may alternatively be separated by forming diastereoisomers, followed by separation of the diastereoisomers, e.g. by differential crystallization or chromatography. The resolving agent may be cleaved from the separated diastereoisomers, e. g. by treatment with acids or bases, to generate the pure enantiomers of the compounds of the invention.

Some preferred polysubstituted pteridine-2,4-diones (lumazines), as well as mono- and polysubstituted 2-thiolumazines, 4-thiolumazines and 2,4-dithiolumazines having the general formula (I) according to the invention are more specifically illustrated in the following examples and defined in the following claims. For instance, useful species include those wherein:

- $R_1$ and $R_2$ are independently benzyl, phenyl, 2-phenylethyl, butyric acid or ester, butyronitrile, 2-hydroxyethyl, ethyl,methyl, and/or
- $R_4$ is chloro, hydroxyl, phenoxy, and/or
- $R_3$ is substituted phenyl.

The present invention further provides processes and methods for making the novel polysubstituted pteridine-2,4-diones (lumazines), as well as the novel mono- and polysubstituted 2-thiolumazines, 4-thiolumazines and 2,4-dithiolumazines having the general formula (I). As a general rule, the preparation of these (thio)lumazines is based on the principle that, starting from a lumazine or (di)thiolumazine or a lumazine or (di)thiolumazine precursor, e.g. an adequately substituted uracil or thiouracil, each of the substituents $R_1$, $R_2$, $R_3$ and $R_4$ may be introduced separately without adversely influencing the presence of a substituent already introduced or the capacity to introduce further substituents later on. Therefore a process for making a (thio)lumazine having the general formula (I) usually consists of one (e.g. in the case of mono-substituted thiolumazines) or more (in the case of polysubstituted lumazines and thiolumazines) reaction steps for successively introducing one or more of the substituents $R_1$, $R_2$, $R_3$ and $R_4$ into a compound selected from the group consisting of lumazine, 2-thiolumazine, 4-thiolumazine, 2,4-dithiolumazine and known substituted (thio) lumazines, or starting from suitable (thio)lumazine precursors (such as uracils and thiouracils) already bearing the desired substituents, each reaction step being optionally, if needed, sub-divided into one or more sub-steps involving intermediate substituted (thio)lumazines.

A limited number of methods are already known in the art for introducing a substituent $R_1$, $R_2$, $R_3$ or $R_4$ to form a (substituted) lumazine or a substituted (thio)lumazine precursor, and a still more limited number of methods are known in the art for introducing a substituent $R_1$, $R_2$, $R_3$ or $R_4$ into a 2-thiolumazine, 4-thiolumazine or 2,4- dithiolumazine, all of these methods being disclosed in the prior documents referred to in the background of the invention. These methods may be applied successfully to the preparation of compounds having the general formula (I). Other methods have been developed by the present inventors, which may be used alternatively or may be combined with the former methods (depending upon the targeted final compound) and will now be explained by reference to the appended figures 1 to 7 relating to substituted lumazines
wherein, unless otherwise stated hereinafter, $R_1$, $R_2$, $R_3$ or $R_4$ are as defined in the summary of the invention. The same methods may be applied starting from the few substituted thiolumazines or dithiolumazines which are already known in the art. In the description of the reaction steps involved in each figure, reference may be made to the use of certain catalysts and/or certain types of solvents. It should be understood that each catalyst mentioned should be used in a catalytic amount well known to the skilled person with respect to the type of reaction involved. Solvents that may

be used in the following reaction steps include various kinds of organic solvents such as protic solvents, polar aprotic solvents and non-polar solvents as well as aqueous solvents which are inert under the relevant reaction conditions. More specific examples include aromatic hydrocarbons, chlorinated hydrocarbons, ethers, aliphatic hydrocarbons, alcohols, esters, ketones, amides, water or mixtures thereof, as well as supercritical solvents such as carbon dioxide (while performing the reaction under supercritical conditions). The suitable reaction temperature and pressure conditions applicable to each kind of reaction step will not be detailed herein but do not depart from the relevant conditions already known to the skilled person with respect to the type of reaction involved.

Figure 1 represents a scheme for (i) replacing a methyl substituent $R_3$ with an unsaturated partly aliphatic chain or spacer in the 6-position of the pteridine ring and optionally further (ii) inserting an oxygen atom between the $R_4$ substituent and the pteridine ring in the 7-position thereof. The $R_3$ substituent replacement occurs via a succession of reaction steps as follows. In reaction step (a), a 6-methyl substituted lumazine is reacted:

- either with an halogen, preferably chlorine or bromine, in the presence of a protic solvent, or
- with an N-halosuccinimide in the presence of a protic or aprotic solvent. Then in reaction step (b), the reaction product of step (a) is reacted, in the presence of a non-polar solvent, preferably toluene, xylene or nitro-methane, with a phosphine selected from the group consisting of trialkylphosphines ($alkyl_3P$), triarylphosphines ($aryl_3P$), tricycloalkylphosphines ($cylcoalkyl_3P$) and trialkylphosphites ($alkoxy_3P$), or the corresponding arsines, thus resulting in an intermediate compound wherein $R_5$ is selected from the group consisting of $(aryl)_3\ P^+X^-$, $(alkyl)_3\ P^+X^-$, $(cycloalkyl)_3\ P^+X^-$, $(alkyloxy)_2PO$, $(aryl)_3\ As^+X^-$, $(alkyl)_3\ As^+X^-$ and $(cycloalkyl)_3As^+X^-$.

Then in the combined reaction steps (c) and (d), this intermediate compound is reacted, in the presence of a catalyst, with alkyl- or aryl- or alkylaryl- or heterocyclic- or alkoxycarbonyl-aldehyde or ketone. Suitable aliphatic, aromatic or heteroaromatic aldehydes and ketones include (in the following non-exhaustive list, use of the plural is meant to include all possible isomers) benzaldehyde, mono- and polyhalogenated benzaldehydes, cyanobenzaldehydes, substituted or non-substituted amino-benzaldehydes, mono- and dinitrobenzaldehydes, mono- and polyalkoxybenzaldehydes, mono- and polyalkylated benzaldehydes, carboxylated (esters and amides) benzaldehydes, aryloxybenzaldehydes, 2-fluorene-carboxaldehyde, naphthaldehydes, alkoxynaphthaldehydes, N-ethyl-3-carbazole-carboxaldehyde, 4-formylcinnamic acid, alkylthiobenzaldehydes, 2-formylbenzenesulfonic acid, methylformylbenzoate, acetaminobenzaldehyde, aryloxyalkylbenzaldehydes, acetamidobenzaldehyde, alkylsulfonylbenzaldehyde, propionaldehyde, butyraldehyde and the corresponding ketones. The catalyst used in the combined reaction steps (c) and (d) may be selected from the group consisting of alkoxy-alkaline metals (e.g.
wherein the metal is Li, Na or K), DBU (1,8-diazabicyclo[5.4.0]undec-7-ene), DBN (1,5-diazabicyclo[4.3.0]non-5-ene), guanidine and strong bases such as butyllithium. The combined reaction steps (c) and (d) result in a compound
wherein $R_6$ is selected from the group consisting of alkyl, aryl, alkylaryl, heterocyclic and alkoxycarbonyl and wherein, depending on whether an aldehyde or a ketone was reacted,$R_7$ is selected from the group consisting of hydrogen, alkyl and aryl.

Then in reaction step (e), this compound is reacted with an halogen, preferably chlorine or bromine, in the presence of a chlorinated solvent such as carbon tetrachloride or chloroform. Finally in reaction step (f), the replacement of the $R_4$ substituent is performed by reacting the product from step (e) with an alkoxy-alkaline metal (e.g. wherein the metal is Li, Na or K), thus resulting in a compound wherein $R_8$ is alkyl.

Figure 2 represents a scheme for replacing a carboxylic substituent $R_3$ in a substituted lumazine with a group having the formula $COCHZ_1Z_2$, wherein each of $Z_1$ and $Z_2$ may be independently selected from the group consisting of alkylcarbonyl, arylcarbonyl, alkoxycarbonyl, aryloxycarbonyl and cyano. In a first reaction step (a), a 6-carboxylic acid substituted lumazine is reacted with:

- either a compound selected from the group consisting of $SOX_2$, $(COX)_2$, $PX_3$ and $PX_5$ (preferably wherein X is Cl or Br) in the presence of a solvent,
- or triphenylphosphine in the presence of carbon tetrachloride. In a second reaction step (b), the halocarbonyl product from step (a) is reacted with a methylene $Z_1Z_2$ ethoxy-magnesium salt or alkaline metal salt (preferably

wherein the metal is Na, K or Li) in the presence of an aprotic solvent.

Figure 3 represents a scheme for replacing a hydroxyl group with an aryl substituent $R_4$ in the 7-position of the pteridine ring of a substituted lumazine. In a first reaction step (a), a 7-hydroxyl, 6-carboxyaryl substituted lumazine is reacted with an aromatic hydrocarbon in the presence of a catalyst such as aluminum trichloride and a chlorinated solvent, then in a second step (b) the product of step (a) is reacted with $KMnO_4$ in the presence of a solvent such as dioxane.

Figure 4 represents a scheme for performing various alterations of a substituent $R_4$ starting from a known substituted lumazine wherein the said $R_4$ is either methyl or formyl. In reaction step (a), a 7-methyl substituted lumazine

is reacted:

- either with an halogen, preferably chlorine or bromine, in the presence of a protic solvent, or
- with an N-halosuccinimide in the presence of a protic or aprotic solvent. Then in reaction step (b), the reaction product of step (a) is reacted, in the presence of a non-polar solvent, preferably toluene, xylene or nitro-methane, with a phosphine selected from the group consisting of trialkylphosphines ($alkyl_3P$), triarylphosphines ($aryl_3P$), tricycloalkyl-phosphines ($cylcoalkyl_3P$) and trialkoxyphosphines ($alkoxy_3P$), or the corresponding arsines, thus resulting in an intermediate compound wherein $R_5$ is selected from the group consisting of $(aryl)_3\ P^+X^-$, $(alkyl)_3\ P^+X^-$, $(cycloalkyl)_3\ P^+X^-$, $(alkyloxy)_2PO$, $(aryl)_3\ As^+X^-$, $(alkyl)_3\ As^+X^-$ and $(cycloalkyl)_3As^+X^-$.

Then in the combined reaction steps (c) and (d), this intermediate compound is reacted, in the presence of a catalyst, with an alkyl- or aryl- or alkylaryl- or heterocyclic- or alkoxycarbonyl-aldehyde or ketone which, alike the catalyst, may be as defined herein-above with reference to the description of figure 1. The combined reaction steps (c) and (d) result in a compound wherein $R_6$ is selected from the group consisting of alkyl, aryl, alkylaryl, heterocyclic and alkoxycarbonyl and wherein, depending on whether an aldehyde or a ketone was reacted, $R_7$ is selected from the group consisting of hydrogen, alkyl and aryl. This same compound may also be prepared from a 7-formyl substituted lumazine through the combined reaction steps (g) and (h), using first an alkyl-, alkylaryl-, alkylheterocyclic-, alkoxycarbonylalkyl-, aryloxycarbonylalkyl-$R_9$ wherein $R_9$ is selected from the group consisting of triphenylphosphonium halides, trialkylphosphonium halides, tricycloalkylphosphonium halides or alkyl-phosphonates, and secondly a catalyst which may be selected from the group consisting of alkoxy-alkaline metals (e.g. wherein the metal is Li, Na or K), DBU, DBN and guanidine.

Then in reaction step (e), this compound is reacted with an halogen, preferably chlorine or bromine, in the presence of a chlorinated solvent such as carbon tetrachloride or chloroform. Finally in reaction step (f), the product of step (e) is reacted with a catalyst which may be selected from the group consisting of alkoxy-alkaline metals (e.g. wherein the metal is Li, Na or K), DBU, DBN and guanidine.

Figure 5 represents a scheme for performing various alterations of a substituent $R_3$ starting from a known substituted lumazine wherein the said $R_3$ is either formyl. In reaction step (a), a 6-formyl substituted lumazine is reacted with a compound having the formula $R_5CH_2NO_2$ in the presence of a base (such as a tertiary amine) and a protic or aprotic solvent. Then in step (b), the product of step (a) may be reacted with acetic anhydride in the presence of pyridine and a base in an aprotic solvent. Then in step (e), the product of step (b) may be reacted with hydrogen sulfide or a thiol $R_6SH$ wherein $R_6$ may be alkyl or aryl in a protic solvent. Alternatively in step (c), the starting 6-formyl substituted lumazine is reacted with a compound selected from the group consisting of $R_7$-oxycarbonyl-methyltriphenylphosphonium halides, $R_7$-oxycarbonyl-methyltrialkyl-phosphonium halides, $R_7$oxycarbonylmethylalkyl-phosphonates and the corresponding arsenium compounds in the presence of a base. Then in step (d), the product of step (c) may be reacted with an halogen, preferably chlorine or bromine, or an N-halosuccinimide in the presence of a protic solvent. Then in step (f), the product of step (d) may be reacted with a catalyst such as DBU, DBN or guanidine in an aprotic solvent in order to produce one or more halogenated isomers wherein the halogen atom X may be on one or the other side of the double bond. Then in step (g), the product of step (f) may be reacted with a compound having the formula $R_8OM$ (wherein M is a metal such as Na, K or Li and $R_8$ may be an alkoxy, alkoxylaryl or alkoxyheterocyclic radical).

Figure 6 represents a scheme for preparing a substituted lumazine wherein the said $R_4$ is either arylalkyl or hydroxyl and performing various alterations of a substituent $R_4$ starting from the said 7-hydroxyl substituted lumazine. In step (a), a 6-amino-5-nitroso-pyrimidin-2,4-dione being optionally substituted in the 1-position (with substituent $R_2$) and/or in the 3-position (with substituent $R_1$) is reacted with:

- either hydrogen in the presence of a hydrogenation catalyst (such as $PtO_2$) in the presence of a protic solvent,
- or sodium dithionite in the presence of water, in order to obtain an optionally substituted 5,6-diamino-uracil.

In step (b), the said optionally substituted 5,6-diamino-uracil is reacted with an arylmethylglyoxal in the presence of a protic solvent in order to obtain a 7-arylmethyl lumazine being optionally further substituted in positions 1 and/or 3. In step (c), the latter is further modified into the corresponding 7-acyl lumazine (e.g. a 7-benzoyllumazine) through an oxidation reaction by means of $KMnO_4$ in the presence of water.

Alternatively, the optionally substituted 5,6-diamino-uracil from step (a) is transformed into a 7-hydroxy lumazine being substituted in the 6-position with substituent $R_3$ through the combined steps (d) and (e) of :

- reaction with an alkyl arylglyoxylate or alkyl alkylglyoxylate or alkyl heterocyclic glyoxylate or alkyl arylalkylglyoxylate or alkyl heterocyclic alkylglyoxylate in the presence of a protic solvent, and
- acidification by means of an acid (e.g. nitric, sulfuric, chlorhydric acids and the like) in the presence of water. The hydroxyl substituent of the lumazine from step (e) may then be replaced by a thiol, mercapto, alkoxy, chloro or

amino substituent by any of steps (f), (g), (h) and (I) or any combination of such steps. For instance, in step (f) the lumazine from step (e) is reacted with $P_4S_{10}$ in the presence of pyridine. In step (f) the lumazine from step (e) or thiol analogue from step (f) is reacted with either a dialkylsulfate or a compound having the formula $XR_5$ wherein X may be halogen, tosyl, mesyl and the like, and $R_5$ may be alkyl, arylalkyl, heterocycloalkyl, alkoxycarbonylalkyl or aryloxycarbonyl-alkyl. In step (h), the lumazine from step (e) is reacted with $POCl_3$ in the presence of ammonium chloride. In step (i) the 7-chloro lumazine from step (h) is reacted, in the presence of an aprotic solvent, with an amine, alcohol, phenol, thiol or thiophenol having the formula $HR_6$ (wherein $R_6$ may be selected from the group consisting of amino, alkylamino, cycloalkylamino, arylamino, heterocycloalkylamino, alkoxy, aryloxy, alkylaryloxy, arylalkyloxy, heterocycloalkyloxy, thio, alkylthio, arylthio, arylalkylthio, alkylarylthio, heterocycloalkylthio) in the further presence of CsF and a catalyst such as a crown ether (e.g. 18-crown-6).

Figure 7 represents a general scheme for preparing first a poly-substituted lumazine bearing either a multiplet of substituents ($R_1$, $R_3$, $R_4$) or a multiplet of substituents ($R_2$, $R_3$, $R_4$) and then introducing a further substituent ($R_2$ or $R_1$ respectively) into the compound of the first step. The first step consists of two sub-steps (a, b) which may be performed either simultaneously or subsequently. In the first sub-step (a), an uracil bearing a substituent $R_2$ or $R_1$, wherein $R_1$ and $R_2$ may be selected from the group consisting of hydrogen, alkyl, cycloalkyl, arylalkyl and heterocycloalkyl, is reacted in a protic solvent with a reactant bearing atoms or groups $R_3$ and $R_4$, wherein $R_3$, $R_4$ may be selected from the group consisting of hydrogen, hydroxyl, alkyl, cycloalkyl, aryl, arylalkyl, alkylaryl and heterocyclic, and wherein the said reactant may be selected from the group consisting of:

- alkyl arylglyoxylates, alkyl alkylglyoxylates, alkyl heterocyclic glyoxylates, alkyl arylalkylglyoxylates, and alkyl heterocyclic alkylglyoxylates, in which case $R_4$ is hydroxyl;
- alkylglyoxals, arylglyoxals, alkylarylglyoxals, arylalkylglyoxals, heterocyclic glyoxals, and the monoximes corresponding to the said glyoxals, in which case one of $R_3$ and $R_4$ is hydrogen; and
- dialkylethylenediones, alkyl arylethylenediones, diarylethylenediones, alkyl heterocyclic ethylenediones and diheterocyclic ethylenediones, in which case $R_3$ and $R_4$ are both different from hydrogen and hydroxyl. In the second sub-step (b), reaction is completed by acidification of the reaction mixture.

After the first step (a, b) is completed, the resulting poly-substituted lumazine bearing either a multiplet of substituents ($R_1$, $R_3$, $R_4$) or a multiplet of substituents ($R_2$, $R_3$, $R_4$) is reacted, through step (c) or step (d) respectively, in the presence of a base and a polar aprotic solvent or a protic solvent, with a reactant $R_2X$ or $R_1X$ respectively (wherein $R_1$ and $R_2$ may each be selected from the group consisting of alkyl, cycloalkyl, arylalkyl and heterocycloalkyl, and X may be selected from the group consisting of chloro, bromo, iodo, tosylate, mesylate and the like).

When $R_4$ and $R_3$ together form an optionally substituted aryl radical, this is achieved by aromatic cyclization techniques well known in the art.

Further methods of preparation are shown in figures 8 to 11 and are further explained and illustrated in the following exmples.

When applicable, and depending upon the specific substituents being present, not only the novel poly-substituted pteridine-2,4-diones (lumazines), and mono- and polysubstituted 2-thiolumazines, 4-thiolumazines and 2,4-dithiolumazines having the general formula (I) but also the (thio)lumazines previously known in the art without any indication of biological activity, i.e. all of the (thio)lumazines having the general formula (II) according to this invention, may be in the form of a pharmaceutically acceptable salt. The latter include any therapeutically active non-toxic addition salts which compounds having the general formula (II) are able to form with a salt-forming agent. Such addition salts may conveniently be obtained by treating the (thio)lumazine compounds (II) of the invention with an appropriate salt-forming acid or base. Examples of such appropriate salt-forming acids include, for instance, inorganic acids resulting in forming salts such as but not limited to the hydrochloride, hydrobromide, sulfate, nitrate, phosphate, diphosphate, bicarbonate, carbonate, and the like, of (thio)lumazine compounds having the general formula (II); or organic monocarboxylic or dicarboxylic acids resulting in forming salts such as, for example, the acetate, propanoate, hydroxyacetate, 2-hydroxypropanoate, 2-oxopropanoate, lactate, pyruvate, oxalate, malonate, succinate, maleate, fumarate, malate, tartrate, citrate, methanesulfonate, ethanesulfonate, benzoate, benzenesulfonate, p-toluene-sulfonate, salicylate, p-aminosalicylate, pamoate, bitartrate, camphorsulfonate, edetate, 1,2-ethanedisulfonate, fumarate, glucoheptonate, gluconate, glutamate, hexylresorcinate, hydroxynaphtoate, hydroxyethanesulfonate, lactate, mandelate, methylsulfate, pantothenate, stearate and the like, of (thio)lumazine compounds having the general formula (II). Examples of appropriate salt-forming bases include, for instance, inorganic bases like metallic hydroxides such as but not limited to those of calcium, lithium, magnesium, potassium, sodium and zinc, resulting in the corresponding metal salt of the (thio)lumazine compounds having the general formula (II); organic bases such as but not limited to N,N'-dibenzylethylenediamine, chloroprocaine, choline, diethanolamine, ethylene-diamine, N-methylglucamine, procaine and the like.

Reaction conditions for treating the (thio)lumazine compounds (II) of this invention with an appropriate salt-forming acid or base are similar to standard conditions involving the same acid or base but different compounds to be submitted to salification. Preferably the pharmaceutically acceptable salt will be selected so as to impart greater water-solubility, lower toxicity, greater stability and/or slower dissolution rate to the compounds of this invention. The term "pharmaceutically acceptable salt " as used herein also includes any solvate which may be formed with a suitable inorganic solvent (e.g. hydrates) or organic solvent, such as but not limited to alcohols, ketones, esters and the like.

The present invention further provides the use of a poly-substituted pteridinedione (lumazine), as well as a mono- or poly-substituted 2-thiolumazine, 4-thiolumazine or 2,4-dithiolumazine represented by the general formula (II), or a pharmaceutically acceptable salt thereof, as a biologically-active ingredient, especially as a medicine or a diagnostic agent or for the manufacture of a medicament or a diagnostic kit. In particular the said medicament may be for the prevention or treatment of a pathologic condition selected from the group consisting of:

- immune disorders, in particular organ and cells transplant rejections, and autoimmune disorders,
- cardiovascular disorders,
- disorders of the central nervous system, and
- cell proliferative disorders.

The pathologic conditions and disorders concerned by the said use, and the corresponding methods of prevention or treatment, are detailed hereinbelow. Any of the uses mentioned with respect to the present invention may be restricted to a non-medical use (e.g. in a cosmetic composition), a non-therapeutic use, a non-diagnostic use, a non-human use (e.g. in a veterinary composition), or exclusively an *in-vitro* use, or a use with cells remote from an animal.

The invention further relates to a pharmaceutical composition comprising:

(a) one or more tri- or tetra-substituted pteridinediones (lumazines), 2-thiolumazines, 4-thiolumazines or 2,4-dithiolumazines represented by the general formula (II), and
(b) one or more pharmaceutically acceptable carriers.

In a third embodiment, this invention provides combinations, preferably synergistic combinations, of one or more tri- or tetra-substituted pteridinediones (lumazines), 2-thiolumazines, 4-thiolumazines or 2,4-dithiolumazines represented by the general formula (II) with one or more biologically-active drugs being preferably selected from the group consisting of immunosuppressant and/or immunomodulator drugs, antineoplastic drugs, and antiviral agents. As is conventional in the art, the evaluation of a synergistic effect in a drug combination may be made by analyzing the quantification of the interactions between individual drugs, using the median effect principle described by Chou et al. in *Adv. Enzyme Reg.* (1984) 22:27. Briefly, this principle states that interactions (synergism, additivity, antagonism) between two drugs can be quantified using the combination index (hereinafter referred as CI) defined by the following equation:

$$CI_x = \frac{ED_x^{1c}}{ED_x^{1a}} + \frac{ED_x^{2c}}{ED_x^{2a}}$$

wherein $ED_x$ is the dose of the first or respectively second drug used alone (1 a, 2a), or in combination with the second or respectively first drug (1 c, 2c), which is needed to produce a given effect. The said first and second drug have synergistic or additive or antagonistic effects depending upon CI < 1, CI = 1, or CI > 1, respectively. As will be explained in more detail herein-below, this principle may be applied to a number of desirable effects such as, but not limited to, an activity against transplant rejection, an activity against immunosuppression or immunomodulation, or an activity against cell proliferation.

For instance the present invention relates to a pharmaceutical composition or combined preparation having synergistic effects against immunosuppression or immunomodulation and containing:

(a) one or more immunosuppressant and/or immunomodulator drugs, and
(b) at least one poly-substituted pteridinedione (lumazine), or mono- or poly-substituted 2-thiolumazine, 4-thiolumazine or 2,4-dithiolumazine represented by the general formula (II), and
(c) optionally one or more pharmaceutical excipients or pharmaceutically acceptable carriers, for simultaneous, separate or sequential use in the treatment or prevention of autoimmune disorders and/or in transplant-rejections.

The advantages to associate a (thio)lumazine compound represented by the general formula (II) with one or more other immunosuppressants are that:

- the therapeutic spectrum of action of the individual components is quantitatively and qualitatively broadened, and
- it allows, by means of a dose reduction without reduced efficacy but with increased safety, the treatment of immune disorders which hitherto had no indication for immunosuppressive therapy as a result of side effects. At the same time, the therapy costs can be decreased to an appreciable extent.

Suitable immunosuppressant drugs for inclusion in the synergistic compositions or combined preparations of this invention are preferably selected from the group consisting of cyclosporin A, substituted xanthines (e.g. methylxanthines such as pentoxyfylline), tacrolimus, rapamycin (and derivatives thereof), leflunomide (or its main active metabolite A771726, or analogs thereof called malononitrilamides), mycophenolic acid and salts thereof (including the sodium salt marketed under the trade name Mofetil® ), adrenocortical steroids, azathioprine, brequinar, gusperimus, 6-mercaptopurine, mizoribine, chloroquine, hydroxychloroquine and monoclonal antibodies with immunosuppressive properties. Adrenocortical steroids within the meaning of this invention mainly include glucocorticoids such as but not limited to dexamethasone, methylprednisolone, methotrexate, prednisone, prednisolone, triamcinolone and pharmaceutically acceptable salts thereof. Rapamycin derivatives as referred herein include O-alkylated derivatives, particularly 9-deoxorapamycins, 26-dihydrorapamycins, 40-O-substituted rapamycins and 28,40-O,O-disubstituted rapamycins (as disclosed in U.S. Patent No. 5,665,772) such as 40-O-(2-hydroxy) ethyl rapamycin - also known as SDZ-RAD -, pegylated rapamycin (as disclosed in U.S. Patent No. 5,780,462), ethers of 7-desmethylrapamycin (as disclosed in U.S. Patent No. 6,440,991) and polyethylene glycol esters of SDZ-RAD (as disclosed in U.S. Patent No. 6,331,547).

Suitable immunomodulator drugs for inclusion into the synergistic immunomodulating pharmaceutical compositions or combined preparations of this invention are preferably selected from the group consisting of acemannan, amiprilose, bucillamine, ditiocarb sodium, imiquimod, Inosine Pranobex, interferon-β, interferon-γ, lentinan, levamisole, pidotimod, romurtide, platonin, procodazole, propagermanium, thymomodulin, thymopentin and ubenimex.

Synergistic activity of the pharmaceutical compositions or combined preparations of this invention against immunosuppression or immunomodulation may be readily determined by means of one or more tests such as, but not limited to, the MLR (abbreviation standing for " mixed lymphocyte reaction ") test, or a test wherein TNF-α or IL-1β inhibition and/or a test wherein the activation of a cluster of differentiation (hereinafter referred as CD) is quantified. The synergistic effect may be evaluated by the median effect analysis method described herein-before. Such tests may for instance, according to standard practice in the art, involve the use of equiment, such as flow cytometer, being able to separate and sort a number of cell subcategories at the end of the analysis, before these purified batches can be analyzed further.

Synergistic activity of the pharmaceutical compositions of this invention in the treatment of transplant rejection may be readily determined by means of one or more tests such as but not limited to the Whole Blood Assay (hereinafter referred as WBA) as described for instance by Lin et al. in *Transplantation* (1997) 63:1734-1738. WBA is a lymphoproliferation assay performed *in vitro* using lymphocytes present in the whle blood, taken from animals that were previously given test substances *in vivo.* Hence it reflects the *in vivo* effect of substances as assessed by an *in vitro* readout assay. The synergistic effect is evaluated by the median effect analysis method described herein-before.

The pharmaceutical composition or combined preparation with synergistic activity against immunosuppression or immunomodulation according to this invention may contain the compound of formula (II) over a broad content range depending on the contemplated use and the expected effect of the preparation. Generally, the (thio)lumazine content of the combined preparation is within the range of 0.1 to 99.9% by weight, preferably from 1 to 99% by weight, more preferably from 5 to 95% by weight.

The invention further relates to a composition or combined preparation having synergistic effects against cell proliferation and containing:

(a) one or more antineoplastic drugs, and
(b) at least one poly-substituted pteridinedione (lumazine), or a mono- or poly-substituted 2-thiolumazine, 4-thiolumazine or 2,4-dithiolumazine represented by the general formula (I) or (IV), and
(c) optionally one or more pharmaceutical excipients or pharmaceutically acceptable carriers, for simultaneous, separate or sequential use in the treatment or prevention of cell proliferative disorders.

Suitable antineoplastic drugs for inclusion into the synergistic antiproliferative pharmaceutical compositions or combined preparations of this invention are preferably selected from the group consisting of alkaloids, alkylating agents (including but not limited to alkyl sulfonates, aziridines, ethylenimines, methylmelamines, nitrogen mustards and nitrosoureas), antibiotics, antimetabolites (including but not limited to folic acid analogs, purine analogs and pyrimidine analogs), enzymes, interferon and platinum complexes.

Synergistic activity of the pharmaceutical compositions or combined preparations of this invention against cell proliferation may be readily determined by means of one or more tests such as, but not limited to, the measurement of the radioactivity resulting from the incorporation of $^3$H-thymidine in culture of tumor cell lines. For instance, different

tumor cell lines are selected in order to evaluate the anti-tumor effects of the test compounds, such as but not limited to:

- RPMI1788: human Peripheral Blood Leucocytes (PBL) Caucasian tumor line,
- Jurkat: human acute T cell leukemia,
- EL4: C57Bl/6 mouse lymphoma, or
- THP-1: human monocyte tumor line. Depending on the selected tumor cell line, different culture media may be used, such as for example:

- for RPMI1788 and THP-1: RPMI-1640 + 10% FCS + 1% NEAA + 1% sodium pyruvate + $5x10^{-5}$ mercapto-ethanol + antibiotics (G-418 0.45 μg/ml).
- for Jurkat and EL4: RPMI-1640 + 10% FCS + antibiotics (G-418 0.45 μg/ml). In a specific embodiment of the synergy determination test, the tumor cell lines are harvested and a suspension of $0.27x10^6$ cells/ml in whole medium is prepared. The suspensions (150 μl) are added to a microtiter plate in triplicate. Either complete medium (controls) or the test compounds at the test concentrations (50 μl) are added to the cell suspension in the microtiter plate. The cells are incubated at 37°C under 5% $CO_2$ for about 16 hours. $^3$H-thymidine is added, and the cells incubated for another 8 hours. The cells are harvested and radioactivity is measured in counts per minute (CPM) in a ß-counter. The $^3$H-thymidine cell content, and thus the measured radioactivity, is proportional to the proliferation of the cell lines. The synergistic effect is evaluated by the median effect analysis method as disclosed herein-before.

The pharmaceutical composition or combined preparation with synergistic activity against cell proliferation according to this invention may contain the (thio)lumazine compound of formula (I) or (IV) over a broad content range depending on the contemplated use and the expected effect of the preparation. Generally, the (thio)lumazine content of the combined preparation is within the range of 0.1 to 99.9% by weight, preferably from 1 to 99% by weight, more preferably from 5 to 95% by weight.

The invention further relates to a pharmaceutical composition or combined preparation having synergistic effects against a viral infection and containing:

(a) one or more anti-viral agents, and
(b) at least one poly-substituted pteridinedione (lumazine), or mono- or poly-substituted 2-thiolumazine, 4-thiolumazine or 2,4-dithiolumazine represented by the general formula (I) or (IV), and
(c) optionally one or more pharmaceutical excipients or pharmaceutically acceptable carriers, for simultaneous, separate or sequential use in the treatment or prevention of a viral infection.

Suitable anti-viral agents for inclusion into the synergistic antiviral compositions or combined preparations of this invention include, for instance, retroviral enzyme inhibitors belonging to categories well known in the art, such as HIV-1 IN inhibitors, nucleoside reverse transcriptase inhibitors (e.g. zidovudine, lamivudine, didanosine, stavudine, zalcitabine and the like), non-nucleoside reverse transcriptase inhibitors (e.g. nevirapine, delavirdine and the like), other reverse transcriptase inhibitors (e.g. foscarnet sodium and the like), and HIV-1 protease inhibitors (e.g. saquinavir, ritonavir, indinavir, nelfinavir and the like). Other suitable antiviral agents include for instance acyclovir, cidofovir, cytarabine, edoxudine, famciclovir, floxuridine, ganciclovir, idoxuridine, penciclovir, sorivudine, trifluridine, valaciclovir, vidarabine, kethoxal, methisazone, moroxydine, podophyllotoxin, ribavirine, rimantadine, stallimycine, statolon, tromantadine and xenazoic acid.

Especially relevant to this aspect of the invention is the inhibition of the replication of viruses selected from the group consisting of picorna-, toga-, bunya-, orthomyxo-, paramyxo-, rhabdo-, retro-, arena-, hepatitis B-, hepatitis C-, hepatitis D-, adeno-, vaccinia-, papilloma-, herpes-, corona-, varicella- and zoster-virus, in particular human immunodeficiency virus (HIV). Synergistic activity of the pharmaceutical compositions or combined preparations of this invention against viral infection may be readily determined by means of one or more tests such as, but not limited to, the isobologram method, as previously described by Elion et al. in *J. Biol. Chem.* (1954) 208:477-488 and by Baba et al. in *Antimicrob. Agents Chemother.* (1984) 25:515-517, using $EC_{50}$ for calculating the fractional inhibitory concentration (hereinafter referred as FIC). When the minimum FIC index corresponding to the FIC of combined compounds (e. g., $FIC_x + FIC_y$) is equal to 1.0, the combination is said to be additive; when it is beween 1.0 and 0.5, the combination is defined as subsynergistic, and when it is lower than 0.5, the combination is by defined as synergistic. When the minimum FIC index is between 1.0 and 2.0, the combination is defined as subantagonistic and, when it is higher than 2.0, the combination is defined as antagonistic.

The pharmaceutical composition or combined preparation with synergistic activity against viral infection according to this invention may contain the (thio)lumazine compound of formula (I) or (IV) over a broad content range depending on the contemplated use and the expected effect of the preparation. Generally, the (thio)lumazine content of the combined preparation is within the range of 0.1 to 99.9% by weight, preferably from 1 to 99% by weight, more

preferably from 5 to 95% by weight.

The pharmaceutical compositions and combined preparations according to this invention may be administared orally or in any other suitable fashion. Oral administration is preferred and the preparation may have the form of a tablet, aqueous dispersion, dispersable powder or granule, emulsion, hard or soft capsule, syrup, elixir or gel. The dosing forms may be prepared using any method known in the art for manufacturing these pharmaceutical compositions and may comprise as additives sweeteners, flavoring agents, coloring agents, preservatives and the like. Carrier materials and excipients are detailed hereinbelow and may include, *inter alia,* calcium carbonate, sodium carbonate, lactose, calcium phosphate or sodium phosphate; granulating and disintegrating agents, binding agents and the like. The pharmaceutical composition or combined preparation may be included in a gelatin capsule mixed with any inert solid diluent or carrier material, or has the form of a soft gelatin capsule, in which the ingredient is mixed with a water or oil medium. Aqueous dispersions may comprise the biologically active composition or combined preparation in combination with a suspending agent, dispersing agent or wetting agent. Oil dispersions may comprise suspending agents such as a vegetable oil. Rectal administration is also applicable, for instance in the form of suppositories or gels. Injection is also applicable as a mode of administration, for instance in the form of injectable solutions or dispersions.

Auto-immune disorders to be prevented or treated by the pharmaceutical compositions or combined preparations of this invention include both systemic auto-immune diseases such as but not limited to lupus erythematosus, psoriasis, vasculitis, polymyositis, scleroderma, multiple sclerosis, ankylosing spondilytis, rheumatoid arthritis and Sjögren syndrome; auto-immune endocrine disorders such as thyroiditis; and organ-specific auto-immune diseases such as but not limited to Addison disease, hemolytic or pernicious anemia, Goodpasture syndrome, Graves disease, idiopathic thrombocytopenic purpura, insulin-dependent diabetes mellitus, juvenile diabetes, uveitis, Crohn's disease, ulcerative colitis, pemphigus, atopic dermatitis, autoimmune hepatitis, primary biliary cirrhosis, autoimmune pneumonitis, auto-immune carditis, myasthenia gravis, glomerulonephritis and spontaneous infertility.

Transplant rejections to be prevented or treated by the pharmaceutical compositions or combined preparations of this invention include the rejection of transplanted or grafted organs or cells (both allografts and xenografts), such as but not limited to host versus graft reaction and, especially after bone marrow transplantation, graft versus host reaction or disease. "Organ" herein means all organs or parts of organs in mammals, in particular humans, such as but not limited to kidney, lung, bone marrow, hair, cornea, eye (vitreous), heart, heart valve, liver, pancreas, blood vessel, skin, muscle, bone, intestine or stomach. " Rejection " as used herein mean all reactions of the recipient body or of the transplanted organ which in the end lead to cell or tissue death in the transplanted organ or adversely affect the functional ability and viability of the transplanted organ or the recipient. In particular, this means acute and chronic rejection reactions. Also included in this invention is preventing or treating the rejection of cell transplants and xenotransplantation. The major hurdle for xenotransplantation is that even before the T lymphocytes, responsible for the rejection of allografts, are activated, the innate immune system, especially T-independent B lymphocytes and macrophages are activated. This provokes two types of severe and early acute rejection called hyper-acute rejection and vascular rejection, respectively. The present invention addresses the problem that conventional immunosuppressant drugs like cyclosporin A are ineffective in xenotransplantation. The ability of the compounds of this invention to suppress T-independent xeno-antibody production as well as macrophage activation may be evaluated in the ability to prevent xenograft rejection in athymic, T-deficient mice receiving xenogenic hamster-heart grafts.

Cell proliferative disorders to be prevented or treated by the pharmaceutical compositions or combined preparations of this invention include not only tumor progression or invasion or metastasis inhibition of a cancer selected from the group consisting of lung cancer, leukaemia, ovarian cancer, sarcoma, Kaposi's sarcoma, meningioma, colon cancer, lymp node tumor, glioblastoma multiforme, prostate cancer or skin carcinose, but also side effects associated with current cancer therapies, including chemotherapy or radiation therapy, such as gastrointestinal mucosal damage or radiation-induced mucositis, the treatment being based on enhancing resistance of mesenchymal cells to TNF.

CNS disorders to be prevented or treated by the pharmaceutical compositions of this invention include cognitive pathologies such as dementia, cerebral ischemia, trauma, epilepsy, schizophrenia, chronic pain and neurologic disorders such as but not limited to depression, social phobia and obsessive compulsive disorders.

Cardiovascular disorders to be prevented or treated by the pharmaceutical compositions of this invention include ischemic disorders, infarct or reperfusion damage, atherosclerosis and stroke.

The term " pharmaceutically acceptable carrier or excipient " as used herein in relation to pharmaceutical compositions and combined preparations means any material or substance with which the biologically-active ingredient (s), i.e. the (thio)lumazine of formula (I) or (IV), and optionally the immunosuppressant or immunomodulator or antineoplastic drug or antiviral agent, may be formulated in order to facilitate its application or dissemination to the locus to be treated, for instance by dissolving, dispersing or diffusing the said composition, and/or to facilitate its storage, transport or handling without impairing its effectiveness. The pharmaceutically acceptable carrier may be a solid or a liquid or a gas which has been compressed to form a liquid, i.e. the compositions of this invention can suitably be used as concentrates, emulsions, solutions, granulates, dusts, sprays, aerosols, pellets or powders.

Suitable pharmaceutical carriers for use in the said pharmaceutical compositions and their formulation are well known to those skilled in the art. There is no particular restriction to their selection within the present invention although, due to the usually low or very low water-solubility of the poly-substituted pteridinediones (lumazines), or mono- or poly-substituted 2-thiolumazines, 4-thiolumazines and 2,4-dithiolumazines of this invention, special attention will be paid to the selection of suitable carrier combinations that can assist in properly formulating them in view of the expected time release profile. Suitable pharmaceutical carriers include additives such as wetting agents, dispersing agents, stickers, adhesives, emulsifying or surface-active agents, thickening agents, complexing agents, gelling agents, solvents, coatings, antibacterial and antifungal agents (for example phenol, sorbic acid, chlorobutanol), isotonic agents (such as sugars or sodium chloride) and the like, provided the same are consistent with pharmaceutical practice, i.e. carriers and additives which do not create permanent damage to mammals. The pharmaceutical compositions of the present invention may be prepared in any known manner, for instance by homogeneously mixing, dissolving, spray-drying, coating and/or grinding the active ingredients, in a one-step or a multi-steps procedure, with the selected carrier material and, where appropriate, the other additives such as surface-active agents. may also be prepared by micronisation, for instance in view to obtain them in the form of microspheres usually having a diameter of about 1 to 10 µm, namely for the manufacture of microcapsules for controlled or sustained release of the biologically active ingredient(s).

Suitable surface-active agents to be used in the pharmaceutical compositions of the present invention are non-ionic, cationic and/or anionic materials having good emulsifying, dispersing and/or wetting properties. Suitable anionic surfactants include both water-soluble soaps and water-soluble synthetic surface-active agents. Suitable soaps are alkaline or alkaline-earth metal salts, unsubstituted or substituted ammonium salts of higher fatty acids ($C_{10}$-$C_{22}$), e.g. the sodium or potassium salts of oleic or stearic acid, or of natural fatty acid mixtures obtainable form coconut oil or tallow oil. Synthetic surfactants include sodium or calcium salts of polyacrylic acids; fatty sulphonates and sulphates; sulphonated benzimidazole derivatives and alkylarylsulphonates. Fatty sulphonates or sulphates are usually in the form of alkaline or alkaline-earth metal salts, unsubstituted ammonium salts or ammonium salts substituted with an alkyl or acyl radical having from 8 to 22 carbon atoms, e.g. the sodium or calcium salt of lignosulphonic acid or dodecylsulphonic acid or a mixture of fatty alcohol sulphates obtained from natural fatty acids, alkaline or alkaline-earth metal salts of sulphuric or sulphonic acid esters (such as sodium lauryl sulphate) and sulphonic acids of fatty alcohol/ ethylene oxide adducts. Suitable sulphonated benzimidazole derivatives preferably contain 8 to 22 carbon atoms. Examples of alkylarylsulphonates are the sodium, calcium or alcanolamine salts of dodecylbenzene sulphonic acid or dibutyl-naphtalenesulphonic acid or a naphtalene-sulphonic acid/formaldehyde condensation product. Also suitable are the corresponding phosphates, e.g. salts of phosphoric acid ester and an adduct of p-nonylphenol with ethylene and/or propylene oxide, or phospholipids. Suitable phospholipids for this purpose are the natural (originating from animal or plant cells) or synthetic phospholipids of the cephalin or lecithin type such as e.g. phosphatidylethanolamine, phosphatidylserine, phosphatidylglycerine, lysolecithin, cardiolipin, dioctanylphosphatidylcholine, dipalmitoylphoshatidylcholine and their mixtures.

Suitable non-ionic surfactants include polyethoxylated and polypropoxylated derivatives of alkylphenols, fatty alcohols, fatty acids, aliphatic amines or amides containing at least 12 carbon atoms in the molecule, alkylarenesulphonates and dialkylsulphosuccinates, such as polyglycol ether derivatives of aliphatic and cycloaliphatic alcohols, saturated and unsaturated fatty acids and alkylphenols, said derivatives preferably containing 3 to 10 glycol ether groups and 8 to 20 carbon atoms in the (aliphatic) hydrocarbon moiety and 6 to 18 carbon atoms in the alkyl moiety of the alkylphenol. Further suitable non-ionic surfactants are water-soluble adducts of polyethylene oxide with poylypropylene glycol, ethylenediaminopolypropylene glycol containing 1 to 10 carbon atoms in the alkyl chain, which adducts contain 20 to 250 ethyleneglycol ether groups and/or 10 to 100 propyleneglycol ether groups. Such compounds usually contain from 1 to 5 ethyleneglycol units per propyleneglycol unit. Representative examples of non-ionic surfactants are nonylphenolpolyethoxyethanol, castor oil polyglycolic ethers, polypropylene/ polyethylene oxide adducts, tributylphenoxypolyethoxyethanol, polyethyleneglycol and octylphenoxypolyethoxyethanol. Fatty acid esters of polyethylene sorbitan (such as polyoxyethylene sorbitan trioleate), glycerol, sorbitan, sucrose and pentaerythritol are also suitable non-ionic surfactants.

Suitable cationic surfactants include quaternary ammonium salts, preferably halides, having 4 hydrocarbon radicals optionally substituted with halo, phenyl, substituted phenyl or hydroxy; for instance quaternary ammonium salts containing as N-substituent at least one $C_8$-$C_{22}$ alkyl radical (e.g. cetyl, lauryl, palmityl, myristyl, oleyl and the like) and, as further substituents, unsubstituted or halogenated lower alkyl, benzyl and/or hydroxy-lower alkyl radicals.

A more detailed description of surface-active agents suitable for this purpose may be found for instance in "McCutcheon's Detergents and Emulsifiers Annual" (MC Publishing Crop., Ridgewood, New Jersey, 1981), "Tensid-Taschenbuch", 2nd ed. (Hanser Verlag, Vienna, 1981) and "Encyclopaedia of Surfactants (Chemical Publishing Co., New York, 1981).

Structure-forming, thickening or gel-forming agents may be included into the pharmaceutical compositions and combined preparations of the invention. Suitable such agents are in particular highly dispersed silicic acid, such as the product commercially available under the trade name Aerosil; bentonites; tetraalkyl ammonium salts of montmorillonites (e.g., products commercially available under the trade name Bentone), wherein each of the alkyl groups may contain from 1 to 20 carbon atoms; cetostearyl alcohol and modified castor oil products (e.g. the product commercially available under the trade name Antisettle).

Gelling agents which may be included into the pharmaceutical compositions and combined preparations of the present invention include, but are not limited to, cellulose derivatives such as carboxymethylcellulose, cellulose acetate and the like; natural gums such as arabic gum, xanthum gum, tragacanth gum, guar gum and the like; gelatin; silicon dioxide; synthetic polymers such as carbomers, and mixtures thereof. Gelatin and modified celluloses represent a preferred class of gelling agents.

Other optional excipients which may be included in the pharmaceutical compositions and combined preparations of the present invention include additives such as magnesium oxide; azo dyes; organic and inorganic pigments such as titanium dioxide; UV-absorbers; stabilisers; odor masking agents; viscosity enhancers; antioxidants such as, for example, ascorbyl palmitate, sodium bisulfite, sodium metabisulfite and the like, and mixtures thereof; preservatives such as, for example, potassium sorbate, sodium benzoate, sorbic acid, propyl gallate, benzylalcohol, methyl paraben, propyl paraben and the like; sequestering agents such as ethylene-diamine tetraacetic acid; flavoring agents such as natural vanillin; buffers such as citric acid and acetic acid; extenders or bulking agents such as silicates, diatomaceous earth, magnesium oxide or aluminum oxide; densification agents such as magnesium salts; and mixtures thereof.

Additional ingredients may be included in order to control the duration of action of the biologically-active ingredient in the compositions and combined preparations of the invention. Control release compositions may thus be achieved by selecting appropriate polymer carriers such as for example polyesters, polyamino-acids, polyvinyl-pyrrolidone, ethylene-vinyl acetate copolymers, methylcellulose, carboxymethylcellulose, protamine sulfate and the like. The rate of drug release and duration of action may also be controlled by incorporating the active ingredient into particles, e.g. microcapsules, of a polymeric substance such as hydrogels, polylactic acid, hydroxymethylcellulose, polymethyl methacrylate and the other above-described polymers. Such methods include colloid drug delivery systems like liposomes, microspheres, microemulsions, nanoparticles, nanocapsules and so on. Depending on the route of administration, the pharmaceutical composition or combined preparation of the invention may also require protective coatings.

Pharmaceutical forms suitable for injectable use include sterile aqueous solutions or dispersions and sterile powders for the extemporaneous preparation thereof. Typical carriers for this purpose therefore include biocompatible aqueous buffers, ethanol, glycerol, propylene glycol, polyethylene glycol, complexing agents such as cyclodextrins and the like, and mixtures thereof.

Since, in the case of combined preparations including the (thio)lumazine of this invention and an immunosuppressant or immunomodulator or antineoplastic drug or antiviral agent, both ingredients do not necessarily bring out their synergistic therapeutic effect directly at the same time in the patient to be treated, the said combined preparation may be in the form of a medical kit or package containing the two ingredients in separate but adjacent form. In the latter context, each ingredient may therefore be formulated in a way suitable for an administration route different from that of the other ingredient, e.g. one of them may be in the form of an oral or parenteral formulation whereas the other is in the form of an ampoule for intravenous injection or an aerosol.

The present invention is useful for preventing or treating a disease selected from the group consisting of CNS disorders, cell proliferative disorders, viral infections, immune and auto-immune disorders and transplant rejections in a subject or patient by administering to the patient in need thereof an effective amount of a (thio)lumazine compound having the general formula (I) or (IV), optionally together with an effective amount of another immunosuppressant or immunomodulator or antineoplastic drug or antiviral agent, or a pharmaceutical composition such as disclosed above in extensive details. The effective amount is usually in the range of 0.01 mg to 20 mg, preferably 0.1 mg to 5 mg, per day per kg bodyweight for humans. Depending upon the pathologic condition to be treated and the patient's condition, the said effective amount may be divided into several sub-units per day or may be administered at more than one day intervals.

The present invention further relates to the use of a composition comprising:

(a) one or more immunosuppressant and/or immunomodulator drugs, and
(b) at least one (thio)lumazine represented by the general formula (I) or (IV), in respective proportions such as to provide a synergistic effect against immunosuppression or immunomodulation in a human being. Similarly the invention relates to the use of a composition comprising:

(a) one or more immunosuppressant and/or immunomodulator drugs, and
(b) at least one (thio)lumazine represented by the general formula (I) or (IV), for the manufacture of a medicine for the treatment of an immune or autoimmune disorder in a human being, in respective proportions such as to provide a synergistic effect in the said treatment.

The present invention further relates to a method for selecting or classifying potent immunosuppressive agents, particularly agents or drugs selected from the family of (thio)lumazines represented by the general formula (I) or (IV). Various models may be used for testing an immunosuppressive effect. In vivo, for example, different transplantation models are available. They are strongly influenced by different immunogenicities, depending on the donor and recipient species used and depending on the nature of the transplanted organ. The survival time of transplanted organs can thus be used to measure the suppression of the immune response.

*In vitro*, the most used models are lymphocyte activation tests. Usually activation is measured via lymphocyte proliferation. Inhibition of proliferation thus always means immunosuppression under the experimental conditions applied. There exist different stimuli for lymphocyte activation, in particular co-culture of lymphocytes of different species (mixed lymphocyte reaction, hereinafter referred as MLR) in a so-called mixed lymphocyte culture test: lymphocytes expressing different minor and major antigens of the HLA-DR type (i.e. alloantigens) activate each other non-specifically.

The selection or classification method of this invenion is based on the determination of a set of at least two lymphocyte activation *in vitro* tests, wherein the said set includes at least the MLR test and at least one other test selected from the group consisting of a TNF-$\alpha$ assay and an IL-1 assay. Optionally the selection or classification of agents or drugs of this invention may be performed on the basis of more than two lymphocyte activation *in vitro* tests, for instance on the basis of a set further including an IL-6 assay or an IL-10 assay or an IL-12 assay or an assay for a cluster of differentiation belonging to type I transmembrane proteins such as but not limited to CD3 or CD28, or a cluster belonging to type II transmembrane proteins such as, but not limited to, CD69, CD 71 or CD134.

Tables 2 and 7 below summarize some of the compounds of the present invention that were made and tested for biological activity, in particular lymphocyte activation, according to the above-mentioned assays and test methods.

The following examples are provided only for illustration of the invention and should in no way be understood as limiting its scope.

EXAMPLES 1 to 5 - preparation of tri- and tetra-substituted 1,3-dimethyl lumazines

The following tri- and tetra-substituted 1,3-dimethyl-lumazines were prepared according to methods and procedures described hereinbefore by reference to figures 6 and 7:

- 6-[2-(p-trifluoromethylphenyl)ethenyl]- 1,3-dimethyl-lumazine (example 1),
- 6-[2-(p-trifluoromethylphenyl)ethenyl]-1,3-dimethyl-lumazine (example 2),
- 6-[2-phenylethenyl]-1,3-dimethyl-lumazine (example 3),
- 6-[2-(p-trifluoromethoxyphenyl)ethenyl]-1,3-dimethyl-lumazine (example 4), and
- 6-cyano-7-ethylmercaptoacetate-1,3-dimethyl-lumazine (example 5).

EXAMPLE 6 - preparation of 3-methyl-6-phenyl-7-chloro-lumazine

3-methyl-6-phenyl-7-chloro-lumazine was prepared according to the general methods described hereinbefore by reference to figure 6.

EXAMPLES 7 to 20 - preparation of trisubstituted lumazines

In a first step, trisubstituted lumazines having the general formula (I)
wherein $R_1$ is hydrogen, $R_4$ is hydroxyl, and $R_2$ and $R_3$ are as indicated in table 1 below for each of examples 7-13, were prepared according to the general methods described hereinbefore by reference to figure 6, in particular steps (d) and (e) thereof, and more specifically according to the following procedure:

To a suspension of a 5, 6-diamino-1-substituted uracil hydrate (10 mmole) in 200 ml water, ethyl substituted benzoylformate (12.5 mmole) was added. The resulting mixture was heated under reflux for 40 minutes. After cooling to room temperature, the precipitate was collected to yield a crude product which was recrystallized from a MeOH/water (1/1) mixture to yield yellowish crystals or powder of the desired 7-hydroxyl tri-substituted lumazine.

Table 1

| $R_2$ | $R_3$ | Example |
|---|---|---|
| methyl | phenyl | 7 |
| methyl | p-methoxyphenyl | 8 |
| methyl | p-toluyl | 9 |

Table 1 (continued)

| $R_2$ | $R_3$ | Example |
|---|---|---|
| phenyl | phenyl | 10 |
| benzyl | phenyl | 11 |
| methyl | m,p-dimethoxyphenyl | 12 |
| methyl | p-chlorophenyl | 13 |

In a second step, the following 7-chloro tri-substituted lumazines:

- 1-methyl-6-phenyl-7-chloro-lumazine (example 14),
- 1-methyl-6-(4'-methoxyphenyl)-7-chloro-lumazine (example 15),
- 1-methyl-6-(4'-methylphenyl)-7-chloro-lumazine (example 16),
- 1,6-diphenyl-7-chloro-lumazine (example 17),
- 1-benzyl-6-phenyl-7-chloro-lumazine (example 18),
- 1-methyl-6-(3',4'-dimethoxyphenyl)-7-chloro-lumazine (example 19), and
- 1-methyl-6-(4'-chlorophenyl)-7-chloro-lumazine (example 20) were prepared according to the general methods described hereinbefore by reference to figure 6, in particular step (h) thereof, and more specifically according to the following procedure:

To a suspension of 0.5 g $NH_4Cl$ in 20 ml $POCl_3$, a 7-hydroxy tri-substituted lumazine of examples 7 to 13 (5 mmole) was added. The resulted mixture was heated at 90°C till the starting material completely disappeared. The reaction mixture was concentrated under reduced pressure to a syrup and then 30 g ice was added. After 30 minutes stirring at room temperature, the precipitate was collected, washed with water and dried to yield a crude product. The latter was then purified by chromatography on silica gel (using $MeOH/CH_2Cl_2$ mixtures 1/100 to1/20) to yield the desired 7-chloro-1-substituted-6-substituted lumazine. Crystals were obtained by re-crystallization from methanol.

Each compound of examples 14 to 20 was obtained in the following yield and characterized by the following UV ($MeOH/H_2O$) and $^1$NMR (200 MHz, DMSO-d6) spectra:

Example 14: yield 54%; UV: 238.1, 274.7, 352.1 nm; NMR: 12.1 (s, 1H), 7.74 (m, 2H), 7.56 (m, 3H) and 3.46 (s, 3H) ppm.

Example 15: yield 40%; UV: 288.9, 356.9 nm; $^1$NMR : 12.1 (s, 1 H), 8.06 (d, 2H), 7.04 (d, 2H), 3.82 (s, 3H) and 3.43 (s, 3H) ppm.

Example 16: yield 58%; UV: 279.5, 355.7 nm; $^1$NMR: 12.19 (s, 1 H), 7.75 (d, 2H), 7.46 (d, 2H), 3.55 (s, 3H) and 2.50 (s, 3H) ppm.

Example 17: yield 60%; UV: 274.7, 349.7 nm; $^1$NMR : 12.25 (s, 1 H), 7.73 (m, 2H), 7.54 (m, 5H), 7.43 (m, 3H) and 3.36 (s, 3H) ppm.

Example 18: yield 48%; UV: 274.7, 350.9 nm; $^1$NMR: 12.20 (s, 1 H), 7.74 (m, 2H), 7.55 (m, 3H), 7.35 (m, 5H), 5.28 (s, 2H) and 3.45 (s, 3H) ppm.

Example 19: yield 62%; UV: 298.4, 365.0 nm; $^1$NMR: 12.07 (s, 1 H), 7.35 (d, 1 H), 7.30 (s, 1 H), 7.12 (d, H), 3.83 (s, 3H), 3.80 (s, 3H) and 3.44 (s, 3H) ppm.

Example 20: yield 71%; UV: 238.1, 278.3 and 352.1 nm; $^1$NMR: 12.13 (s, 1 H), 7.77 (d, 2H), 7.64 (d, 2H) and 3.45 (s, 3H) ppm.

EXAMPLES 21 to 25 - preparation of tetra-substituted 1,3-dimethyl-lumazines

The following tetra-substituted 1,3-dimethyl-lumazines:

- 1,3-dimethyl-6-phenyl-7-phenoxy lumazine (example 21),
- 1, 3-dimethyl-6-phenyl-7-piperidino lumazine (example 22),
- 1, 3-dimethyl-6-phenyl-7-morpholino lumazine (example 23),
- 1,3-dimethyl-6-phenyl-7-(4'-N-acetyl)piperazino lumazine (example 24), and
- 1,3-dimethyl-6-phenyl-7-isopropylaminolumazine (example 25) were prepared according to the general methods described hereinbefore by reference to figure 6, in particular step (i) thereof, and more specifically according to the following procedure:

To a mixture of 1.0 g of a 4 Angström molecular sieve, 200 mg CsF (1.2 mmole) in 4 ml THF, 40 mg 18-crown-6 (0.15 mmole) was added. The resulting mixture was stirred at room temperature for one hour. Then, 0.5 mmole of 6-phenyl-7-chloro-1,3-dimethyllumazine and 0.6 mmole of a reactant $HR_6$ (as defined in figure 6) were added respectively (this reactant is phenol in example 21, piperidine in example 22, morpholine in example 23, 4'-N-acetylpiperazine in example 24 and isopropylamine in example 25). The mixture was stirred at room temperature for another one hour, and then filtrated through a pad of Celite® (a filter agent) and rinsed with $CH_2Cl_2$. After concentration under reduced pressure, the residue was purified by chromatography on silica gel (1-5% MeOH in $CH_2Cl_2$) to yield the desired compound as a white or yellow powder.

Each compound of examples 21 to 25 was obtained in the following yield and characterized by the following UV (MeOH/$H_2O$) and $^1$NMR (200 MHz, DMSO-d6 for examples 21-23, $CDCl_3$ for examples 24 and 25) spectra:

Example 21: yield: 83%; UV: 283.0 and 348.5 nm; $^1$NMR: 8.11 (m, 2H), 7.53 (m, 5H), 7.38 (m, 3H) and 3.33 (s, 6H) ppm.
Example 22: yield: 99%; UV: 236.9, 308.0, and 374.6 nm; $^1$NMR: 7.68 (m, 2H), 7.48 (m, 3H), 3.33 (s, 6H), 3.33 (m, 4H) and 1.52 (m, 6H) ppm.
Example 23: yield: 99%; UV: 235.7, 304.4 and 367.4 nm; $^1$NMR: 7.72 (m, 2H), 7.49 (m, 3H), 3.60 (m, 4H), 3.36 (m, 4H) and 3.33 (s, 6H) ppm.
Example 24: yield: 98%; UV: 234.5, 303.2, 367.4 nm; $^1$NMR: 7.77 (m, 2H), 7.45 (m, 3H), 3.66 (s, 1 H), 3.51 (s, 3H), 3.40-3.70 (m, 8H) and 2.09 (s, 3H).
Example 25: yield: 99%; UV: 226.3, 293.7 and 354.5 nm; $^1$NMR: 7.63 (m, 2H), 7.52 (m, 3H), 5.46 (br., 1 H), 4.32 (m, 1 H), 3.67 (s, 3H), 3.50 (s, 3H), 2.17 (s, 3H) and 1.28 (d, 6H).

EXAMPLE 26 - *in vitro* lymphocyte activation tests

All reagents were dissolved in 0.5 ml dimethylsulfoxide (hereinafter referred as DMSO) and further diluted in culture medium before use for the following *in vitro* experiments. The commercially available culture medium consisted of RPMI-1640 + 10% foetal calf serum (FCS).

Compounds described in some of the present examples were tested in the following lymphocyte activation tests:

Mixed Lymphocyte Reaction

Peripheral blood mononuclear cells (hereinafter referred as PBMC) were isolated from heparinized peripheral blood by density gradient centrifugation over Lymphoprep (Nycomed, Maorstua, Norway). Allogeneic PBMC or Eppstein-Barr Virus-transformed human B cells [commercially available under the trade name RPMI1788 (ATCC name CCL156)] which strongly express B7-1 and B7-2 antigens were used as stimulator cells after irradiation with 30 Gy. MLR was performed in triplicate wells. After 5 days incubation at 37°C, 1 µCi [$^3$H]-thymidine was added to each cup. After a further 16 hours incubation, cells were harvested and counted in a ß-counter. Inhibition of proliferation by a compound (drug) described in some of the previous examples was counted using the formula:

$$\text{Percent inhibition} = \frac{\text{(cpm+drugs) - cpm Cult. Med}}{\text{(cpm-drugs) - cpm Cult. Med}} \times 100$$

wherein cpm is the thymidine count per minute.

TNF-alpha and IL-1 beta assays

Peripheral blood mononuclear cells (herein referred as PBMC), in response to stimulation by lipopolysaccharide (LPS), a gram-negative bacterial endotoxin, produce various chemokines, in particular human TNF-alpha and II-1 beta. The inhibition of the activation of PBMC can be measured by the level of suppression of the production of TNF-alpha or IL-1 beta by PBMC in response to stimulation by LPS.

Such inhibition measurement was performed as follows: PBMC were isolated from heparinized peripheral blood (Buffy coat) by density gradient centrifugation. LPS is then added to the PMBC suspension in complete medium ($10^6$ cells /ml) at a final concentration of 1 µg/ml. The compound to be tested was added at different dilution levels, and the cells were incubated at 37°C for 72 hours. The supernatants were collected, and TNF-alpha or II-1 beta concentrations were measured with respectively an anti-TNF antibody or an anti-IL-1 beta antibody in a sandwich ELISA.

The percent inhibition was calculated as : % inhibition = (pg/ml in sample - pg/ml min.) / (pg/ml max. - pg/ml min.) -1

wherein:

- min.: pg/ml in culture medium without test compound, and
- max.: pg/ml in culture medium + LPS without test compound.

Table 2 below shows the $IC_{50}$ values (expressed in μM) of the tested compounds in the MLR test and in the TNF and IL-1 assays (ND: not determined).

TABLE 2

| Ex. n° | $R_3$ | $R_4$ | MLR | TNF | IL-1 |
|---|---|---|---|---|---|
| 6 | phenyl | chloro | 4.2 | 3.0 | 0.4 |
| 14 | phenyl | chloro | 4.1 | 0.5 | 0.35 |
| 15 | p-methoxyphenyl | chloro | 3.0 | 0.8 | 0.4 |
| 16 | p-toluyl | chloro | 3.6 | 1.1 | 0.5 |
| 17 | phenyl | chloro | 3.0 | 0.8 | 0.2 |
| 18 | phenyl | chloro | 4.4 | 4.5 | ND |
| 19 | m,p-dimethoxyphenyl | chloro | 5.1 | 1.1 | ND |
| 20 | p-chlorophenyl | chloro | 3.9 | 3.8 | 0.4 |

EXAMPLE 27 - preparation of 3-methyl-6-aminouracil and 3-allyl-6-aminouracil

A suspension of 6-aminouracil (5 g, 39.3 mmole) in 1,1,1,3,3-hexamethyldisilazane (25 ml, 118 mmole) with a catalytic amount of ammonium sulfate (20 mg) was refluxed for 2 hours until a clear solution was obtained. Methyl iodide or allyl iodide (1.5 equivalent) was added and refluxed. Reaction was quenched with a saturated sodium bicarbonate solution and the precipate formed was filtered off, yielding the pure 3-methyl-6-amino-uracil with a 98% yield, or the pure 3-allyl-6-amino-uracil with a 83% yield.

EXAMPLE 28 - preparation of 5, 6-diamino-1-substituted uracils and 5, 6-diamino-3-substituted uracils

5,6-diamino-1-substituted uracils and 5,6-diamino-3-substituted uracils were prepared according to the following general method described herein by reference to Figure 8. The monosubstituted urea (wherein $R_2$ may be for instance selected from the group consisting of alkyl, cycloalkyl, alkyl, heterocycloalkyl, alkylaryl, arylalkyl and heterocycloalkyl) is reacted with cyanoacetic acid, followed by refluxing in an aqueous alkaline solution to yield a 6-aminouracil derivative. In step (b), a nitroso group is introduced at position 5 of the uracil ring by reaction with sodium nitrite under aqueous acidic conditions. In step (c), the reduction of the nitrosogroup is effected either catalytically (e.g. Pt/H2) or chemically (e.g. sodium dithionite in water or ammonium sulfide in water), yielding the 5,6-diamino-uracil derivative. The 1-substituted-6-aminouracil is reacted in step (d) with the reactant $R_1X$ (wherein $R_1$ may be for instance selected from the group consisting of alkyl, cycloalkyl and allyl, and X may be selected from the group consisting of chloro, bromo and iodo) in the presence of a base (such as potassium carbonate) and a polar aprotic solvent.

More specifically the following procedure was performed: the monosubstituted urea (40 mmole) and cyanoacetic acid (40 mmole) were dissolved in 5 ml acetic anhydride, and the resulting mixture was stirred at 60 °C for 3 hours. After cooling to room temperature, 30 ml ice water was added. The suspension was filtered and the precipitate was washed with water to yield a crude product. Recrystallization from methanol quantitatively yielded the pure intermediate as a white solid. This intermediate (40 mmole) was dissolved in an aqueous NaOH solution (33 %, 50 ml) and stirred at 80 °C for 10 minutes. After cooling to 40 °C, the mixture was neutralized with acetic acid to pH 7, diluted with water (50 ml) and stirred for another 30 minutes. The precipitate was collected by filtration to yield the 6-amino-uracil derivative as a white powder.

In the following step, the 6-amino-uracil derivative (20 mmole) was suspended in water (60 ml), then $NaNO_2$ (24 mmole) and acetic acid (1 ml) were added dropwise. The resulting mixture was stirred at room temperature for 1 hour. A pink precipitate was formed, which was filtered off and washed with water to yield the corresponding 5-nitroso-6-amino-uracil derivative.

Then, $Na_2S_2O_4$ (45 mmole) was added portionwise to a suspension of the 5-nitroso-6-amino-uracil derivative (15 mmole) in water (50 ml). The resulting mixture was stirred at room temperature for 1 hour. The precipitate was collected and washed with water to yield the corresponding 5, 6-diamino-uracil as a grey powder.

The following compounds were synthesized in this way:

5,6-diamino-1-methyluracil was obtained in a 90% yield from commercially available 1-methyl-6-amino-uracil;
5,6-diamino-3-methyluracil was obtained in a 88% yield from the 3-methyl-6-amino-uracil of example 27;
5,6-diamino-1-phenyluracil was obtained from phenylurea in a 62% yield;
5,6-diamino-1-ethyluracil was obtained from ethylurea in a 47% yield;
5,6-diamino-1-butyluracil was obtained from *n*-butylurea in a 45% yield;
5,6-diamino-1-benzyluracil was obtained from benzylurea in a 52% yield;
5,6-diamino-3-allyluracil was obtained in a 58% yield from the 3-allyl-6-aminouracil of example 27.

EXAMPLES 29 to 39 - preparation of 1,3,6-trisubstituted-7-hydroxy-lumazines

The 1,3,6-trisubstituted-7-hydroxy-lumazines (otherwise named 1,3,6-trisubstituted-2,4,7(1H, 3H, 8H)-pteridinetriones) of examples 29 to 39 were prepared from the relevant 5,6-diamino-1-substituted uracils and 5,6-diamino-3-substituted uracils of example 28 in accordance with the general method described herein with reference to figure 9. In step (a) of figure 9, an uracil bearing a substituent $R_1$ and/or $R_2$ wherein $R_1$ and $R_2$ may for instance be selected from the group consisting of hydrogen, alkyl, cycloalkyl, arylalkyl and heterocyclic is reacted in a polar protic solvent with a reactant selected from the group consisting of alkyl arylglyoxylates, alkyl alkylglyoxylates, alkyl heterocyclic glyoxylates, alkyl arylalkylglyoxylates and alkyl heterocyclic alkylglyoxylates.

More specifically the following procedure was performed: to a suspension of the appropriate 5,6-diamino-1,3-substituted uracil made in example 28 (10 mmole) in water (200 ml), the appropriate alkyl arylglyoxylate, alkyl alkylglyoxylate, alkyl heterocyclic glyoxylate, alkyl arylalkylglyoxylate or alkyl heterocyclic alkylglyoxylate (12.5 mmole) was added. The resulting mixture was heated under reflux for 40 minutes. After cooling to room temperature, the precipitate was collected. The crude product was recrystallized from a mixture (1/1) of methanol and water to yield the desired 1,3,6-trisubstituted-7-hydroxylumazine as yellowish crystals or powder. The following table 3 provides the meaning of substituents $R_1$, $R_2$ and $R_3$ for each of the 7-hydroxy lumazines of examples 29 to 39.

The synthesis of each individual compound is now briefly described by indicating the starting uracil and glyoxylate, the yield and physical form, and
characterized by nuclear magnetic resonance data (200 MHz, in DMSO-d6).

6-(4-methoxyphenyl)-1-methyl-7-hydroxy-lumazine (example 29) was obtained from 5,6-diamino-1-methyluracil and ethyl 4-methoxybenzoylformate in 87 % yield as yellow crystals. $^1$H NMR (ppm): 11.65 (s, 1 H), 8.07 (d, J=8.8Hz, 2H), 7.04 (d, J=8.8Hz, 2H), 3.82 (s, 3H), 3.43 (s, 3H).
6-(3,4-dimethoxyphenyl)-1-methyl-7-hydroxy-lumazine (example 30) was obtained from 5,6-diamino-1-methyluracil and ethyl-3,4-dimethoxybenzoylformate in 88 % yield as yellowish powder. $^1$H NMR (ppm): 12.07 (s, 1 H), 7.33 (d, 1 H), 7.30 (s, 1 H), 7.11 (d, 1 H), 3.83 (s, 3H), 3.80 (s, 1 H), 3.44 (s, 3H).
6-(4-fluorophenyl)-1-methyl-7-hydroxy-lumazine (example 31) was obtained from 5,6-diamino-1-methyluracil and ethyl-4-fluorobenzoylformate in 60 % yield as a yellow powder. $^1$H NMR (ppm): 11.68 (s, 1 H), 8.13 (m, 2H), 7.32 (t, 8.8Hz, 2H), 3.43 (s, 3H).
6-(3-methoxyphenyl)-1-methyl-7-hydroxy-lumazine (example 32) was obtained from 5,6-diamino-1-methyluracil and ethyl-3-methoxybenzoylformate in 84 % yield as a yellowish powder. $^1$H NMR (ppm): 11.68 (s, 1 H), 7.65 (m, 1 H), 7.40 (t, 1 H), 7.02 (dd, 1 H), 3.80 (s, 3H), 3.43 (s, 3H).
6-(2,6-dimethoxyphenyl)-1-methyl-7-hydroxy-lumazine (example 33) was obtained from 5,6-diamino-1-methyluracil and ethyl-3,4-dimethoxybenzoylformate in 92 % yield as a yellow powder. $^1$H NMR (ppm): 13.2 (br., 1 H), 11.64 (s, 1 H), 7.05 (m, 3H), 7.30 (s, 1 H), 3.73 (s, 3H), 3.68 (s, 1 H), 3.44 (s, 3H).
6-(2-chlorophenyl)-1-methyl-7-hydroxy-lumazine (example 34) was obtained from 5,6-diamino-1-methyluracil and ethyl-2-chlorobenzoylformate in 92 % yield as a white powder. $^1$H NMR (ppm): 11.70 (s, 1 H), 7.46 (m, 3H), 7.33 (m, 1H),3.45(s,3H).
6-(3-chlorophenyl)-1-methyl-7-hydroxy-lumazine (example 35) was obtained from 5,6-diamino-1-methyluracil and ethyl-3-chlorobenzoylformate in 56 % yield as a yellow powder. $^1$H NMR (ppm): 11.74 (s, 1 H), 8.11 (m, 2H), 7.52 (m, 2H), 3.44 (s, 1H). 6-(4-cyanophenyl)-1-methyl-7-hydroxy-lumazine (example 36) was obtained from 5,6-diamino-1-methyluracil and ethyl 4-cyanobenzoylformate in 88 % yield as yellow crystals. $^1$H NMR (ppm): 11.75 (s, 1 H), 8.28 (d, 2H), 7.95 (d, 2H), 3.44 (s, 3H).
1-methyl-6-(4-methylphenyl)-7-hydroxy-lumazine (example 37) was obtained from 5,6-diamino-1-methyluracil and ethyl 4-methylbenzoylformate in 74 % yield as yellowish crystals. $^1$H NMR (ppm): 11.66 (s, 1 H), 7.98 (d, 2H), 7.29 (d, 2H), 3.43 (s, 3H), 2.36 (s, 3H).
1-benzyl-6-phenyl-7-hydroxy-lumazine (example 38) was obtained from 1-benzyl-5,6-diamino-uracil and ethyl benzoylformate in 90 % yield as yellowish crystals. 6-(4-chlorophenyl)-1-methyl-7-hydroxy-lumazine (example 39) was

obtained from 5, 6-diamino-1-methyluracil and ethyl 4-chlorobenzoylformate in 40 % as a white powder.

Table 3

| Example | $R_2$ | $R_1$ | $R_3$ |
|---|---|---|---|
| 29 | methyl | H | 4-methoxyphenyl |
| 30 | methyl | H | 3,4- dimethoxyphenyl |
| 31 | methyl | H | 4-fluorophenyl |
| 32 | methyl | H | 3-methoxyphenyl |
| 33 | methyl | H | 2,6- dimethoxyphenyl |
| 34 | methyl | H | 2-chlorophenyl |
| 35 | methyl | H | 3-chlorophenyl |
| 36 | methyl | H | 4-cyanophenyl |
| 37 | methyl | H | 4-methylphenyl |
| 38 | benzyl | H | phenyl |
| 39 | methyl | H | 4-chlorophenyl |

EXAMPLES 40 to 54 - preparation of 1,3,6-trisubstituted-7-halo-lumazines

The 1,3,6-trisubstituted-7-halo-lumazines of examples 40 to 54 were prepared from the corresponding 1,3,6-trisubstituted-7-hydroxy-lumazines such as those of examples 29 to 39 in accordance with the method described herein with reference to step (b) of figure 9. The relevant 1,3,6-trisubstituted-7-hydroxylumazine (5 mmole) was added to a suspension of 0.5 g NaX (wherein X is chloro or bromo) in 20 ml $POX_3$ (wherein X is chloro or bromo). The resulting mixture was heated at 90°C until the starting material completely disappeared. The reaction mixture was concentrated under reduced pressure to a syrup and then 30 g ice was added. After 30 minutes stirring at room temperature, the precipitate was collected, washed with water, dried naturally to yield a crude product. Chromatography on silica gel (using a mixture $MeOH/CH_2Cl_2$ in a ratio ranging from 1/100 to 1/20) yielded the desired 7-halo-1,3,6-trisubstituted lumazine. Crystals could be obtained by recrystallization from MeOH.

The synthesis of each individual compound is now briefly described by indicating the starting compound, the yield, purity (determined by high performance liquid chrmoatography) and physical form, and characterized by nuclear magnetic resonance data (200 MHz, in DMSO-d6).

7-chloro-6-(4-methoxyphenyl)-1-methyl-lumazine (example 40) was obtained from the compound of example 29 in 40 % yield as yellowish crystals. Purity: 93.1%. $^1$H NMR (ppm): 12.07 (s, 1H), 7.73 (d, 2H), 7.10 (d, 2H), 3.85 (s, 3H), 3.45 (s, 3H).

7-chloro-6-(3,4-dimethoxyphenyl)-1-methyl-lumazine (example 41) was obtained from the compound of example 30 in 62 % yield as white crystals. Purity: 98.8%. $^1$H NMR (ppm): 12.07 (s, 1 H), 7.35 (d, 1 H), 7.30 (s, 1 H), 7.12 (d, H), 3.83 (s, 3H), 3.80 (s, 3H), 3.44 (s, 3H).

7-chloro-6-(4-fluorophenyl)-1-methyl-lumazine (example 42) was obtained from the compound of example 31 in 48 % yield as yellowish crystals. Purity (HPLC): 95.2%. $^1$H NMR (ppm): 12.12 (s, 1 H), 7.81 (m, 2H), 7.40 (m, 2H), 3.45 (s, 3H).

7-chloro-6-(3-methoxyphenyl)-1-methyl-lumazine (example 43) was obtained from the compound of example 32 in 64 % yield as white crystals. Purity: 94.1 %; $^1$H NMR (ppm): 12.11 (s, 1 H), 7.48 (t, 1 H), 7.26 (m, 2H), 7.11 (m, 1 H), 3.82 (s, 3H), 3.45 (s, 3H).

7-chloro-(2,6-dimethoxyphenyl)-1-methyl-lumazine (example 44) was obtained from the compound of example 33 in 75 % yield as white crystals. Purity: 98.3%. $^1$H NMR (ppm): 12.06 (s, 1 H), 7.10 (m, 2H), 6.88 (m, 1H), 3.75 (s, 3H), 3.72 (s, 3H), 3.44 (s, 3H).

7-chloro-(2-chlorophenyl)-1-methyl-lumazine (example 45) was obtained from the compound of example 34 in 54 % yield as yellowish crystals. Purity: 98.4%. $^1$H NMR (ppm): 12.13 (s, 1 H), 7.60 (m, 4H), 3.46 (s, 3H).

7-chloro-3-(chlorophenyl)-1-methyl-lumazine (example 46) was obtained from the compound of example 35 in 12 % yield as yellowish crystals. Purity: 97.0%.H NMR (ppm): 12.15 (s, 1 H), 7.70 (m, 2H), 7.60 (m, 2H), 3.46 (s, 3H).

7-chloro-1-ethyl-6-phenyl-lumazine (example 47) was obtained from 7-hydroxy-1-ethyl-6-phenyl-lumazine (itself

available by reacting 5,6-diamino-1-ethyluracil and ethyl benzoylformate) in 79 % yield as white crystals. Purity: 97.5%. $^1$H NMR (ppm): 12.08 (s, 1 H), 7.74 (m, 2H), 7.54 (m, 3H), 4.12 (q, 2H), 1.23 (t, 3H).

7-chloro-6-(4-cyanophenyl)-1-methyl-lumazine (example 48) was obtained from the compound of example 36 in 77% yield as yellowish crystals. Purity: 98.5%. $^1$H NMR (ppm): 12.18 (s, 1 H), 8.05 (d, 2H), 7.95 (d, 2H), 3.46 (s, 3H).

7-bromo-1-methyl-6-phenyl-lumazine (example 49) was obtained from 7-hydroxy-1-methyl-6-phenyl-lumazine (itself available by reacting 5,6-diamino-1-methyluracil and ethyl benzoylformate) in 70 % yield as yellowish crystals. Purity: 99.2%. $^1$H NMR (ppm): 8.90 (br.s, 1 H), 7.76 (m, 2H), 7.48 (m, 2H), 3.70 (s, 3H).

7-bromo-6-(3,4-dimethoxyphenyl)-1-methyl-lumazine (example 50) was obtained from the compound of example 30 in 50% yield as yellow crystals. Purity: 100%. $^1$H NMR (ppm): 8.58 (br.s, 1 H), 7.40 (dd, 1 H), 7.30 (d, 1 H), 6.97 (d, 1 H), 3.96 (s, 3H), 3.95 (s, 3H), 3.69 (s, 3H).

7-bromo-6-(4-methoxyphenyl)-1-methyl-lumazine (example 51) was obtained from the compound of example 29 in 48 % yield as yellow crystals. Purity: 99.3%. $^1$H NMR (ppm): 8.73 (br.s, 1 H), 7.80 (m, 1 H), 7.03 (m, 2H), 3.91 (s, 3H), 3.71 (s, 3H), 3.69 (s, 3H).

7-chloro-1-methyl-6-(4-methylphenyl)-lumazine (example 52) was obtained from the compound of example 37 in 58% yield as white crystals. Purity: 97.7%. $^1$H NMR (ppm): 12.19 (s, 1 H), 7.75 (d, 2H), 7.46 (d, 2H), 3.55 (s, 3H), 2.50 (s, 3H).

7-chloro-1-benzyl-6-phenyl-lumazine (example 53) was obtained from the compound of example 38 in 48% yield as yellowish crystals. Purity: 97.4%. $^1$NMR (ppm): 12.20 (s, 1 H), 7.74 (m, 2H), 7.55 (m, 3H), 7.35 (m, 5H), 5.28 (s, 2H), 3.45 (s, 3H).

7-chloro-6-(4-chlorophenyl)-1-methyl-lumazine (example 54) was obtained from the compound of example 39 in 71 % yield as yellowish crystals. Purity: 99.7%. $^1$H NMR (ppm): 12.13 (s, 1H), 7.77 (d, 2H), 7.64 (d, 2H), 3.45 (s, 3H).

The following table 4 summarizes the meanings of substituents $R_1$, $R_2$, $R_3$ and $R_4$ for each of the 1,6-disubstituted 7-halo-lumazines of examples 40 to 54.

Table 4

| Example | $R_2$ | $R_1$ | $R_3$ | $R_4$ |
|---|---|---|---|---|
| 40 | methyl | H | 4-methoxyphenyl | Cl |
| 41 | methyl | H | 3, 4-dimethoxyphenyl | Cl |
| 42 | methyl | H | 4-fluorophenyl | Cl |
| 43 | methyl | H | 3-methoxyphenyl | Cl |
| 44 | methyl | H | 2, 6-dimethoxyphenyl | Cl |
| 45 | methyl | H | 2-chlorophenyl | Cl |
| 46 | methyl | H | 3-chlorophenyl | Cl |
| 47 | ethyl | H | phenyl | Cl |
| 48 | methyl | H | 4-cyanophenyl | Cl |
| 49 | methyl | H | phenyl | Br |
| 50 | methyl | H | 3, 4-dimethoxyphenyl | Br |
| 51 | methyl | H | 4-methoxyphenyl | Br |
| 52 | methyl | H | 4-methylphenyl | Cl |
| 53 | benzyl | H | phenyl | Cl |
| 54 | methyl | H | 4-chlorophenyl | Cl |

EXAMPLES 55 to 82 - preparation of 1,3,6-trisubstituted-7-halo-lumazines

The 7-halo-1,3,6-trisubstituted lumazines of examples 55 to 82 were prepared from the 7-halo-1,6-disubstituted lumazines of examples 40 to 54 in accordance with the method described in figure 10 for introducing a further substituent $R_1$, or $R_2$. The starting lumazine is reacted in step (a) with a reactant $R_2X$ or in step (b) with a reactant $R_1X$, wherein $R_1$ and $R_2$ may each be for instance selected from the group consisting of alkyl, cycloalkyl, alkyl carboxylic acid esters, thioesters and amides, thiocarboxylic acid esters, thioesters and amides, heterocycloalkyl, and wherein X may be for instance selected from the group consisting of chloro, bromo, iodo, tosylate and mesylate, in the presence

of a base (such as, but not limited to, potassium carbonate or sodium hydride) and a polar aprotic solvent.

More specifically, the following procedure was performed: 0.38 mmole of an alkyl halide (in which the halide is bromo or iodo) was added to a mixture of a 7-halo-1,6-disubstituted lumazine - step (a) - or a 7-halo-3,6-disubstituted lumazine - step (b) - (0.30 mmole) and $K_2CO_3$(62 mg, 0.45 mmole) in 4 ml DMF. The resulting mixture was stirred at room temperature for 4 hours. The mixture was diluted with 50 ml $CH_2Cl_2$ and washed with 40 ml brine. After drying over $MgSO_4$, filtration and concentration, the residue was purified by silica gel chromatography to yield the desired 7-halo-1,3,6-trisubstituted lumazine noted as 4 in figure 10.

The synthesis of each individual compound is now briefly described by indicating the starting compound, the yield, purity (determined by high performance liquid chromatography) and physical form, and characterized by nuclear magnetic resonance (200 MHz, in $CDCl_3$).

7-chloro-3-(ethyl butyrate)-1-methyl-6-phenyl-lumazine (example 55) was obtained from 7-chloro-1-methyl-6-phenyl-lumazine (which may itself be obtained from 7-hydroxy-1-methyl-6-phenyl-lumazine) in 79 % yield as white crystals. Purity: 99.9%. $^1$H NMR (ppm): 7.82 (m, 2H), 7.50 (m, 3H), 4.21 (t, 2H), 4.11 (q, 2H), 3.72 (s, 3H), 2.43 (t, 2H), 2.07 (m, 2H), 1.24 (t, 3H).

7-chloro-1-(ethyl butyrate)-3-methyl-6-phenyl-lumazine (example 56) was obtained from 7-chloro-3-methyl-6-phenyl-lumazine (which may itself be obtained from 7-hydroxy-3-methyl-6-phenyl-lumazine) in 62 % yield as white crystals. Purity: 98.4%. $^1$H NMR (ppm): 7.82 (m, 2H), 7.50 (m, 3H), 4.41 (t, 2H), 4.12 (q, 2H), 3.55 (s, 3H), 2.46 (t, 2H), 2.14 (m, 2H), 1.25 (t, 3H).

7-chloro-3-(2-hydroxyethyl)-1-methyl-6-phenyl-lumazine (example 57) was obtained from 7-chloro-1-methyl-6-phenyl-lumazine in 65 % yield as white crystals. Purity: 98.8%. $^1$H NMR (ppm): 7.82 (m, 2H), 7.50 (m, 3H), 4.41 (t, 2H), 3.97 (m, 2H), 3.74 (s, 3H).

7-chloro-3-(2-phenylethyl)-1-methyl-6-phenyl-lumazine (example 58) was obtained from 7-chloro-1-methyl-6-phenyl-lumazine in 65 % yield as white crystals. Purity: 98.6%. $^1$H NMR (ppm): 7.82 (m, 2H), 7.50 (m, 3H), 7.32 (m, 5H), 4.35 (m, 2H), 3.72 (s, 3H), 3.01 (m, 2H).

7-chloro-3-benzyl-1-methyl-6-phenyl-lumazine (example 59) was obtained from 7-chloro-1-methyl-6-phenyl-lumazine in 64 % yield as white crystals. Purity: 99.3%. $^1$H NMR (ppm): 7.82 (m, 2H), 7.58 (m, 2H), 7.50 (m, 3H), 7.28 (m, 3H), 5.32 (s, 2H), 3.71 (s, 3H).

7-chloro-1 -(2-phenylethyl)-3-methyl-6-phenyl-lumazine (example 60) was obtained from 7-chloro-3-methyl-6-phenyl-lumazine in 34 % yield as white crystals. Purity: 97.6%. $^1$H NMR (ppm): 7.80 (m, 2H), 7.50 (m, 3H), 7.30 (m, 5H), 4.55 (m, 2H), 3.56 (s, 3H), 3.07 (m, 2H).

1 -benzyl-7-chloro-3-methyl-6-phenyl-lumazine (example 61) was obtained from 7-chloro-3-methyl-6-phenyl-lumazine in 46 % yield as white crystals. Purity: 98.7%.$^1$H NMR (ppm): 7.82 (m, 2H), 7.58 (m, 2H), 7.50 (m, 3H), 7.32 (m, 3H), 5.51 (s, 2H), 3.56 (s, 3H).

7-chloro-1-(2-hydroxyethyl)-3-methyl-6-phenyl-lumazine (example 62) was obtained from 7-chloro-3-methyl-6-phenyl-lumazine in 42 % as white crystals. Purity: 97.5%. $^1$H NMR (ppm): 7.82 (m, 2H), 7.50 (m, 3H), 4.59 (t, 2H), 4.04 (t, 2H), 3.56 (s, 3H).

7-chloro-6-(3,4-dimethoxyphenyl)-3-(ethyl butyrate)-1-methyl-lumazine (example 63) was obtained from the compound of example 41 and ethyl 4-bromobutyrate in 56 % yield as a yellow powder. Purity: 93.7%. $^1$H NMR (ppm): 7.48 (dd, 1H), 7.38 (d, 1 H), 6.97 (d, 1 H), 4.22 (t, 2H), 4.12 (q, 2H), 3.96 (s, 6H), 3.72 (s, 3H), 2.43 (t, 2H), 2.08 (m, 2H), 1.24 (t, 3H).

7-chloro-6-(3, 4-dimethoxyphenyl)-3-(2-hydroxyethyl)-1-methyl-lumazine (example 64) was obtained from the compound of example 41 and 2-bromoethanol in 70 % yield as a white yellow powder. Purity: 98.4%.$^1$H NMR (ppm): 7.47 (dd, 1 H), 7.37 (d, 1 H), 6.97 (d, 1 H), 4.41 (m, 2H), 3.98 (m, 2H), 3.96 (s, 6H), 3.74 (s, 3H).

7-chloro-1,6-diphenyl-3-(ethyl butyrate)-lumazine (example 65) was obtained from 7-chloro-1,6-diphenyl-lumazine and ethyl 4-bromobutyrate in 67 % yield as white crystals. Purity: 99.8%. $^1$H NMR (ppm): 7.79 (m, 2H), 7.57 (m, 3H), 7.49 (m, 3H), 7.32 (m, 2H), 4.25 (t, 2H), 4.10 (q, 2H), 2.45 (t, 2H), 2.12 (m, 2H), 1.26 (t, 3H).

7-chloro-1,6-diphenyl-3-(2-hydroxyethyl)-lumazine (example 66) was obtained from 7-chloro-1,6-diphenyl-lumazine and 2-bromoethanol in 78 % yield as white crystals. Purity: 91 %. $^1$H NMR (ppm): 7.79 (m, 2H), 7.57 (m, 3H), 7.49 (m, 3H), 7.32 (m, 2H), 4.35 (t, 2H), 3.42 (t, 2H).

3-butyronitrile-7-chloro-1-methyl-6-phenyl-lumazine (example 67) was obtained from 7-chloro-1-methyl-6-phenyl-lumazine and 4-bromobutyronitrile in 89 % yield as white crystals. Purity: 99.3%. $^1$H NMR (ppm): 7.82 (m, 2H), 7.49 (m, 3H), 4.31 (t, 2H), 3.73 (s, 3H), 2.49 (t, 2H), 2.15 (m, 2H).

7-chloro-1-ethyl-3-(ethyl butyrate)-6-phenyl-lumazine (example 68) was obtained from the compound of example 47 and ethyl 4-bromobutyrate in 95 % yield as white crystals. Purity: 99.2%. $^1$H NMR (ppm): 7.82 (m, 2H), 7.50 (m, 3H), 4.38 (q, 2H), 4.21 (t, 2H), 4.10 (q, 2H), 2.43 (t, 2H), 2.07 (m, 2H), 1.35 (t, 3H), 1.24 (t, 3H).

3-acetamido-7-chloro-1-methyl-6-phenyl-lumazine (example 69) was obtained from 7-chloro-1-methyl-6-phenyl-lumazine and 2-bromoacetamide in 95 % yield as white crystals. Purity: 98.8%. $^1$H NMR (ppm): 7.82 (m, 2H), 7.50

(m, 3H), 4.82 (s, 2H), 3.74 (s, 3H).

7-bromo-3-(ethyl butyrate)-1-methyl-6-phenyl-lumazine (example 70) was obtained from the compound of example 49 and ethyl 4-bromobutyrate in 69 % yield as yellowish crystals. Purity: 98.2%. $^1$H NMR (ppm): 7.74 (m, 2H), 7.49 (m, 3H), 4.21 (t, 2H), 4.10 (q, 2H), 3.73 (s, 3H), 2.43 (t, 2H), 2.07 (m, 2H), 1.24 (t, 3H).

7-chloro-3-(ethyl acetate)-1-methyl-6-phenyl-lumazine (example 71) was obtained from 7-chloro-1-methyl-6-phenyl-lumazine and ethyl 2-bromoacetate in 97 % yield as a white powder. Purity: 99.2%. $^1$H NMR (ppm): 7.82 (m, 2H), 7.50 (m, 3H), 4.88 (s, 3H), 4.25 (q, 2H), 3.74 (s, 3H), 1.31 (t, 3H).

7-chloro-3-(ethyl pentanoate)-1-methyl-6-phenyl-lumazine (example 72) was obtained from 7-chloro-1-methyl-6-phenyl-lumazine and ethyl 5-bromopentanoate in 65 % yield as a white powder. Purity: 99.7%. $^1$H NMR (ppm): 7.82 (m, 2H), 7.50 (m, 3H), 4.13 (m, 4H), 3.72 (s, 3H), 2.37 (t, 2H), 1.75 (m, 4H), 1.25 (t, 3H).

7-chloro-3-(ethyl butyrate)-1-methyl-6-(4-methoxyphenyl)-lumazine (example 73) was obtained from the compound of example 40 and ethyl-4-bromobutyrate in 55 % yield as a yellow solid. Purity: 97.0%. $^1$H NMR (ppm): 7.82 (d, 2H), 7.01 (d, 2H), 4.21 (t, 2H), 4.11 (q, 2H), 3.88 (s, 3H), 3.71 (s, 3H), 2.43 (t, 2H), 2.08 (m, 2H), 1.24 (t, 3H).

7-chloro-1-methyl-3-(2-morpholinoethyl)-6-phenyl-lumazine (example 74) was obtained from 7-chloro-1-methyl-6-phenyl-lumazine and 4-(2-iodoethyl)morpholine in 88 % yield as white crystals. Purity: 91.2%. $^1$H NMR (ppm): 7.82 (m, 2H), 7.50 (m, 3H), 4.31 (t, 2H), 3.73 (s, 3H), 3.67 (m, 4H), 2.60 (m, 4H).

3-(2-butyloxycarbonyl-aminoethyl)-7-chloro-1-methyl-6-phenyl-lumazine (example 75) was obtained from 7-chloro-1-methyl-6-phenyl-lumazine and 2-(butyloxycarbonylamino)ethyl bromide in 61 % as white crystals. Purity: 99.5%. $^1$H NMR (ppm): 7.82 (m, 2H), 7.50 (m, 3H), 4.90 (br., 1H), 4.32 (t, 2H), 3.73 (s, 3H), 3.52 (m, 2H), 1.32 (s, 9H).

7-chloro-1,3-dimethyl-6-(4-methoxyphenyl)-lumazine (example 76) was obtained from the compound of example 40 and iodomethane in 57 % yield as a yellow solid. Purity: 99.9%. $^1$H NMR (ppm): 7.82 (d, 2H), 7.01 (d, 2H), 3.88 (s, 3H), 3.73 (s, 3H), 3.56 (s, 3H).

7-chloro-1-methyl-6-phenyl-3-(2-piperidinoethyl)-lumazine (example 77) was obtained from 7-chloro-1-methyl-6-phenyl-lumazine and 1-(2-iodoethyl)piperidine in 54 % yield as a yellow solid. Purity: 91.7%. $^1$H NMR (ppm): 7.82 (d, 2H), 7.01 (d, 2H), 4.38 (m, 2H), 3.66 (s, 3H), 2.90 (m, 4H), 1.60(m, 6H).

7-chloro-1-methyl-6-phenyl-3-(2-pyrrolidinoethyl)-lumazine (example 78) was obtained from 7-chloro-1-methyl-6-phenyl-lumazine and 1-(2-iodoethyl)pyrrolidine in 66 % yield as yellow solid. Purity: 91.9%. $^1$H NMR (ppm): 7.82 (d, 2H), 7.01 (d, 2H), 4.50 (m, 2H), 3.70 (s, 3H), 3.56 (m, 2H), 2.12 (m, 4H), 1.30 (m, 2H). 7-bromo-6-(3,4-dimethoxyphenyl)-3-(ethylbutyrate)-1-methyl-lumazine (example 79) was obtained from the compound of example 50 and ethyl 4-bromobutyrate in 54% yield as a yellow solid. Purity: 99.7%; $^1$H NMR (ppm): 7.42 (dd, 1 H), 7.31 (d, 1 H), 6.96 (d, 1 H), 4.21 (t, 2H), 4.11 (q, 2H), 3.95 (s, 6H), 3.72 (s, 3H), 2.43 (t, 2H), 2.07 (m, 2H), 1.24 (t, 3H).

7-bromo-3-(ethylbutyrate)-1-methyl-6-(4-methoxyphenyl)-lumazine (example 80) was obtained from the compound of example 51 and ethyl 4-bromobutyrate in 55 % yield as yellow solid. Purity: 99.4%. $^1$H NMR (ppm): 7.79 (d, 2H), 7.03 (d, 2H), 4.23 (t, 2H), 4.14 (q, 2H), 3.91 (s, 3H), 3.74 (s, 3H), 2.45 (t, 2H), 2.10 (m, 2H), 1.27 (t, 3H).

7-bromo-1-methyl-3-(2-morpholinoethyl)-6-phenyl-lumazine (example 81) was obtained from the compound of example 49 and 4-(2-iodoethyl)morpholine in 68 % yield as a yellowish powder. Purity: 95.5%. $^1$H NMR (ppm): 7.77 (m, 2H), 7.50 (m, 3H), 4.31 (t, 2H), 3.74 (s, 3H), 3.69 (m, 4H), 2.65 (m, 4H).

7-bromo-1 -methyl-3-(2-morpholinoethyl)-6-(3,4dimethoxyphenyl)-lumazine (example 82) was obtained from the compound of example 50 and 4-(2-iodoethyl)morpholine in 76 % yield as a yellow powder. Purity: 90.3%. $^1$H NMR (ppm): 7.42 (dd, 1 H), 7.32 (d, 1 H), 6.98 (d, 1 H), 4.32 (t, 2H), 3.96 (s, 6H), 3.73 (s, 3H), 3.72 (m, 4H), 2.70 (m, 4H).

The following table 5 summarizes the meanings of substituents $R_1$, $R_2$, $R_3$ and $R_4$ for each of the 1,3,6-trisubstituted 7-halolumazines of examples 55 to 82.

EXAMPLE 83 - preparation of 3-(ethyl butyrate)-7-fluoro-1-methyl-6-(4-methoxyphenyl)-lumazine

CsF (1.2 mmole, 200 mg) and 18-crown-6 (0.15 mmole, 40 mg) were added to a mixture of 4A° molecular sieves (1.0 g) in THF (4 ml). The resulting mixture was stirred at room temperature for 1 hour. Then the compound of example 73 (0.5 mmole) was added. The mixture was stirred at room temperature for 3 hours, and then filtered through a pad of Celite and rinsed with $CH_2Cl_2$. After concentration under reduced pressure, the residue was purified by chromatography on silica gel (2% acetone in $CH_2Cl_2$) to yield the desired 3-(ethylbutyrate)-7-fluoro-1-methyl-6-(4-methoxyphenyl)-lumazine in 81 % yield as a yellowish solid. $^1$H NMR (ppm): 8.12 (m, 2H), 7.02 (m, 3H), 4.23 (t, 2H), 4.13 (q, 2H), 3.90 (s, 3H), 3.69 (s, 3H), 2.45 (t, 2H), 2.10 (m, 2H), 1.26 (t, 3H).

EXAMPLE 84 - preparation of 7-bromo-1,3-dimethyl-6-(4-hydroxyphenyl)- lumazine

Boron tribromide (1.2 mmole) was added to a solution of the compound of example 76 (0.4 mmole) in 4 ml

$CH_2Cl_2$. The resulting mixture was stirred at room temperature for 24 hours. Reaction was then quenched with water, and the mixture extracted with $CH_2Cl_2$. After concentration under reduced pressure, the residue was purified by chromatography on silica gel (1/30 acetone /$CH_2Cl_2$) to yield 7-bromo-1, 3-dimethyl-6-(4-hydroxyphenyl)-lumazine in 90 % yield as a yellow solid. $^1H$ NMR (ppm): 7.63 (m, 2H), 6.96 (m, 2H), 6.21 (s, 1 H), 3.77 (s, 3H), 3.60 (s, 3H). Interestingly, this procedure achieves simultaneous introduction of a bromo substituent at position 7 of the pteridine ring and conversion of a methoxyphenyl to a hydroxyphenyl substituent at position 6 of the pteridine ring.

Table 5

| Example | $R_2$ | $R_1$ | $R_3$ | $R_4$ |
|---|---|---|---|---|
| 55 | methyl | ethyl butyrate | phenyl | Cl |
| 56 | ethyl butyrate | Me | phenyl | Cl |
| 57 | methyl | 2-hydroxyethyl | phenyl | Cl |
| 58 | methyl | 2-phenylethyl | phenyl | Cl |
| 59 | methyl | benzyl | phenyl | Cl |
| 60 | 2-phenylethyl | methyl | phenyl | Cl |
| 61 | benzyl | methyl | phenyl | Cl |
| 62 | 2-hydroxyethyl | methyl | phenyl | Cl |
| 63 | methyl | ethyl butyrate | 3, 4- dimethoxyphenyl | Cl |
| 64 | methyl | 2-hydroxyethyl | 3,4- dimethoxyphenyl | Cl |
| 65 | phenyl | ethyl butyrate | phenyl | Cl |
| 66 | phenyl | 2-hydroxyethyl | phenyl | Cl |
| 67 | methyl | 4-butyronitrile | phenyl | Cl |
| 68 | ethyl | ethyl butyrate | phenyl | Cl |
| 69 | methyl | 2-acetamido | phenyl | Cl |
| 70 | methyl | ethyl butyrate | phenyl | Br |
| 71 | methyl | ethyl acetate | phenyl | Cl |
| 72 | methyl | ethyl pentanoate | phenyl | Cl |
| 73 | methyl | ethyl butyrate | 4-methoxyphenyl | Cl |
| 74 | methyl | 2-morpholinoethyl | phenyl | Cl |
| 75 | methyl | 2-butyloxycarbonyl aminoethyl | phenyl | Cl |
| 76 | methyl | methyl | 4-methoxyphenyl | Cl |
| 77 | methyl | 2-piperidinoethyl | phenyl | Cl |
| 78 | methyl | 2-pyrrolidinoethyl | phenyl | Cl |
| 79 | methyl | ethyl butyrate | 3,4- dimethoxyphenyl | Br |
| 80 | methyl | ethyl butyrate | 4-methoxyphenyl | Br |
| 81 | methyl | 2-morpholinoethyl | phenyl | Br |
| 82 | methyl | 2-morpholinoethyl | 3,4- dimethoxyphenyl | Br |
| 83 | methyl | ethyl butyrate | 4-methoxyphenyl | F |
| 84 | methyl | Me | 4-hydroxyphenyl | Br |

EXAMPLES 85 to 92 - preparation of 3-carboxy-7-halo-1,6-substituted

lumazines

A 7-halo-1,6-disubstituted-3-(ethyl butyrate) lumazine is converted into the corresponding 7-halo-1,6-disubstituted-3-(*n*-butyric acid) lumazine (noted 5 in figure 11) in accordance with the method shown in figure 11. For reasons of clarity, the synthesis is only shown for the ethyl ester of n-butyric acid, although the synthetic procedure outlined hereunder is similarly applicable to other esters of other monocarboxylic acids. In step (a), the ethyl ester group of a tetra-substituted lumazine is converted into the corresponding free carboxylic acid by basic or acidic hydrolysis. Then in step (b), the 7-halo-1,6-disubstituted-3-(carboxylic acid) lumazine may be reacted with a nucleophile (e.g. an amine, alcohol, phenol, thiol or thiophenol) having the general formula $HR_5$, wherein $R_5$ may be for instance selected from the group consisting of amino, alkylamino, cycloalkylamino, arylamino, heterocyclic alkylamino, alkoxy, aryloxy, alkylaryloxy, arylalkoxy, heterocyclic alkoxy, thioalkyl, arylthio, arylalkylthio alkylarylthio and heterocyclic alkylthio) in a protic solvent or aprotic solvent in the presence of an acid catalyst. Suitable examples of such acid catalysts include, but are not limited to, acetyl chloride and trifluoroacetic anhydride. Alternatively, the monocarboxylic acid can first be converted into the corresponding monocarboxylic acid halide (e.g. by reaction with thionyl chloride or oxalyl chloride), followed by reaction with the said nucleophile $HR_5$.

More specifically, the following procedure was performed for step (a): a solution of a 7-halo-1,6-disubstituted-3-(ethyl butyrate) lumazine (0.5 mmole) in 10 ml dioxane and 10 ml 5% HCl was stirred at room temperature for 24 hours. The solvents were removed under reduced pressure to yield the corresponding 7-halo-1,6-disubstituted-3-(*n*-butyric acid) lumazine which was analyzed by high performance liquid chromatography (purity) and nuclear magnetic resonance (200 MHz, in $CDCl_3$).

7-chloro-3-(*n*-butyric acid)-1-methyl-6-phenyl-lumazine (example 85) is thus obtained from the compound of example 55 in 99 % yield as white crystals. Purity: 97.0%. $^1$H NMR (ppm): 7.80 (m, 2H), 7.49 (m, 3H), 4.23 (t, 2H), 3.72 (s, 3H), 2.47 (t, 2H), 2.08 (m, 2H).

7-chloro-3-(*n*-butyric acid)-1-ethyl-6-phenyl-lumazine (example 86) is thus obtained from the compound of example 68 in 99 % yield as white crystals. Purity: 90%. $^1$H NMR (ppm): 7.82 (m, 2H), 7.49 (m, 3H), 4.38 (q, 2H), 4.23 (t, 2H), 2.47 (t, 2H), 2.08 (m, 2H), 1.38 (t, 3H).

The following procedure was performed for making isopropyl esters (noted 6 in figure 11) according to step (b): acetylchloride (200 µl) was added to a solution of the compound of example 85 (0.4 mmole) in isopropyl alcohol (10 ml). The resulting mixture was stirred at room temperature for 24 hours and then neutralized with $NaHCO_3$ to pH 7. After filtration and concentration, the residue was purified by chromatography on silica gel to obtain 7-chloro-3-(isopropyl 4-butyrate)-1-methyl-6-phenyl-lumazine (example 87) in 92 % yield as a white powder. Purity: 97.5%. $^1$H NMR (ppm): 7.80 (m, 2H), 7.49 (m, 3H), 4.99 (m, 1 H), 4.21 (t, 2H), 3.72 (s, 3H), 2.40 (t, 2H), 2.06 (m, 2H), 1.22 (d, 6H).

Following the above procedures for steps (a) and (b), 7-bromo-6-(3,4-dimethoxyphenyl)-3-(isopropyl butyrate)-1-methyl-lumazine (example 88) was obtained from the compound of example 79 in 54% yield as a yellow powder. Purity: 92.6%. $^1$H NMR (ppm): 7.42 (dd, 1 H), 7.31 (d, 1 H), 6.96 (d, 1 H), 5.01 (m, 1 H), 4.26 (t, 2H), 3.98 (s, 6H), 3.75 (s, 3H), 2.42 (t, 2H), 2.08 (m, 2H), 1.24 (d, 6H).

The following procedure was performed for making 7-chloro-3-(t-butyl butyrate)-1-methyl-6-phenyl-lumazine (example 89) according to step (b): tertbutanol (95 µl) and trifluoroacetic anhydride (56 µl) were added to a solution of the compound of example 85 (0.2 mmole) in $CH_2Cl_2$ (4 ml). The mixture was stirred at room temperature for 4 hours. After removing the solvents under reduced pressure, the residue was purified by chromatography on silica gel to yield the desired t-butyl ester (76 %) as a white powder. Purity: 98.9%. $^1$H NMR (ppm): 7.80 (m, 2H), 7.49 (m, 3H), 4.19 (t, 2H), 3.72 (s, 3H), 2.40 (t, 2H), 2.02 (m, 2H), 1.42 (s, 9H).

The following procedure was performed for making carboxylic amides (noted 6 in figure 11) according to step (b): a solution of the compound of example 85 (0.2 mmole) in thionyl chloride (2 ml) was refluxed for 1 hour. The excess thionyl chloride was removed under reduced pressure. The residue was dissolved in $CH_2Cl_2$ (4 ml) and cooled to -78 °C, then a suitable amine (or a salt thereof) (0.3 mmole) was added and pH was adjusted above 9 by means of triethylamine. The mixture was stirred at -78 °C for 20 minutes, warmed to -20°C, then quenched with 5% HCl and extracted with $CH_2Cl_2$. After drying and filtration, the residue was purified by chromatography on silica gel to yield the desired carboxylic amide. In this way, 3-(4-butyramido)-7-chloro-1-methyl-6-phenyllumazine (example 90) was obtained from ammonia (33 % aqueous solution) in 63 % yield as a yellowish solid. Purity: 98.0%. $^1$H NMR (ppm): 7.80 (m, 2H), 7.49 (m, 3H), 6.10 (br., 1 H), 5.68 (br., 1 H), 4.23 (t, 2H), 3.73 (s, 3H), 2.34 (t, 2H), 2.10 (m, 2H).

Similarly, 7-chloro-3-(N-methyl 4-butyramido)-1-methyl-6-phenyl-lumazine (example 91) was obtained from methylamine hydrochloride in 62 % yield as a yellowish solid. Purity: 92.0%. $^1$H NMR (ppm): 7.79 (m, 2H), 7.49 (m, 3H), 6.15 (br., 1 H), 4.20 (t, 2H), 3.73 (s, 3H), 2.78 (d, 3H), 2.28 (t, 2H), 2.10 (m, 2H). 7-chloro-1-methyl-6-phenyl-3-(N-propyl 4-butyramido)-lumazine (example 92) was obtained from propylamine in 51 % yield as a white solid. Purity: 98.9%. $^1$H NMR (ppm): 7.80 (m, 2H), 7.50 (m, 3H), 6.05 (br., 1 H), 4.20 (t, 2H), 3.73 (s, 3H), 3.18 (q, 2H), 2.27 (t, 2H), 1.52 (m, 2H), 0.91 (t, 3H). Table 6 summarizes the meanings of substituents $R_2$, $R_5$, $R_3$ and $R_4$ for each of the 3-carboxy-1,6-disubstituted-7-halo-lumazines of examples 85 to 92.

Table 6

| Example | $R_2$ | $R_5$ | $R_3$ | $R_4$ |
|---|---|---|---|---|
| 85 | methyl | OH | phenyl | Cl |
| 86 | ethyl | OH | phenyl | Cl |
| 87 | methyl | $OCH(CH_3)_2$ | phenyl | Cl |
| 88 | methyl | $OC(CH_3)_3$ | 3,4- dimethoxyphenyl | Cl |
| 89 | methyl | $OCH(CH_3)_2$ | phenyl | Br |
| 90 | methyl | $NH_2$ | phenyl | Cl |
| 91 | methyl | $NHCH_3$ | phenyl | Cl |
| 92 | methyl | NH-*n*-propyl | phenyl | Cl |

EXAMPLE 93 - *in vitro* biological evaluation of polysubstituted lumazines

Table 7 below shows the $IC_{50}$ values (expressed in μM) of some of the compounds of examples 40 to 92 in the MLR test and in the TNF-α and IL-1 assays performed under the same experimental conditions as in example 26 (ND: not determined).

Table 7

| Example | MLR $IC_{50}$ (μM) | $TNF_\alpha$ $IC_{50}$ (μM) | $IL_{1\beta}$ $IC_{50}$ (μM) |
|---|---|---|---|
| 40 | 3.3 | 0.8 | 0.6 |
| 41 | 5.1 | 1.7 | 0.7 |
| 42 | 4.8 | 4.7 | 0.7 |
| 43 | 4.9 | 3.7 | 2.0 |
| 44 | 4.9 | 5.9 | 0.5 |
| 45 | 3.9 | 2.3 | 0.3 |
| 46 | 5.7 | 4.7 | ND |
| 48 | 4.3 | 2.5 | 0.4 |
| 50 | 4.0 | 1.0 | 0.6 |
| 51 | ND | 4.6 | 5.7 |
| 52 | 3.5 | 1.1 | 0.5 |
| 53 | 4.4 | 4.5 | 1.2 |
| 54 | 3.9 | 3.8 | 0.5 |
| 55 | 4.9 | 0.5 | 0.6 |
| 56 | 4.9 | 0.5 | 0.6 |
| 57 | 5.0 | 0.4 | 0.7 |
| 58 | 5.6 | 5.5 | 0.5 |
| 59 | 5.0 | 5.3 | 0.5 |
| 60 | 3.5 | 5.8 | 4.1 |
| 61 | 3.7 | 0.7 | 0.5 |

Table 7 (continued)

| Example | MLR $IC_{50}$ (μM) | $TNF_{\alpha}$ $IC_{50}$ (μM) | $IL_{1\beta}$ $IC_{50}$ (μM) |
|---|---|---|---|
| 62 | 2.5 | 0.6 | 0.6 |
| 63 | 5.7 | 0.6 | 0.9 |
| 64 | 5.7 | 0.7 | 0.5 |
| 65 | 3.8 | 0.8 | 0.5 |
| 67 | 4.8 | 0.7 | 0.6 |
| 68 | 6.8 | 3.1 | 0.6 |
| 69 | 4.5 | 5.8 | 3.4 |
| 70 | 5.0 | 5.5 | 3.5 |
| 71 | 2.8 | 3.4 | 0.6 |
| 72 | 3.5 | 3.4 | 0.6 |
| 73 | 4.9 | 0.6 | 0.5 |
| 74 | 4.2 | 2.6 | 0.7 |
| 75 | 5.1 | 3.5 | 0.7 |
| 76 | 4.5 | 0.6 | 0.6 |
| 77 | 3.4 | 3.7 | 0.7 |
| 78 | 4.4 | 7.4 | 0.8 |
| 79 | 4.7 | 0.7 | 0.2 |
| 80 | ND | 2.1 | 6.4 |
| 81 | ND | 1.7 | 5.4 |
| 82 | ND | 10.0 | 5.7 |
| 83 | ND | 4.9 | 6.4 |
| 84 | ND | 0.9 | 3.9 |
| 85 | 5.0 | 6.1 | 3.9 |
| 86 | 4.6 | 8.4 | 2.5 |
| 87 | 5.4 | 6.1 | 2.8 |
| 88 | ND | 0.7 | 7.5 |
| 89 | 4.2 | 5.6 | 2.1 |
| 90 | 3.9 | 4.9 | 1.6 |
| 92 | 3.4 | 5.4 | 1.1 |

EXAMPLE 94 - model of rheumatoid arthritis

Collagen type II (hereinafter referred as CII) induced experimental model of rheumatoid arthritis (hereinafter referred as RA) in DBA mice is widely accepted as the most relevant and predictive preclinical model for RA. In this model, DBA mice are immunized with CII, the collagen type mainly present in the joint structures, together with complete Freund Adjuvant in their tail. 2 to 3 weeks later, several of the immunized mice start to develop arthritis in the four footpaths. In order to further worsen the disease, mice are given a second CII boost at three weeks after the first immunisation, this time however in a footpath. Because the immune system is already immunised in these mice, this rapidly provokes a severe swelling of the injected footpath (named Delayed Type Hypersensitivity or DTH) which can be used as a measurement for T-cell activation. Within a few days after the booster, almost all untreated animals start developing symptoms of arthritis. RA development is scored from 0 to 16 (16 being severe clinical arthritis in all four footpaths). At the end of the study (3 weeks after the CII boost) antibody formation was determined against CII and

histology performed on the footpaths.

The efficiency of the polysubstituted lumazine of example 41, administered in an amount of 20 mg/kg/day, started one day before the CII boost) was explored in this CII model. All such treated animals developed significanly less severe rheumatoid arthritis (clinical scores ranging from 0 to 5) as compared to untreated control mice (clinical scores from 6 to 12) and also compared to mice treated with methotrexate (clinical scores ranging from 2 to 7), the most effective compound for the treatment of rheumatoid arthritis to date.

EXAMPLE 95 - model of multiple sclerosis

The polysubstituted lumazine of example 42 showed significant protection in a murine multiple sclerosis standard model (experimental allergic encephalomyelitis) such as described by Kuschnaroff et al. in *J. Neuroimmunol.* (1999) 99:157-168. Animals that were treated with this lumazine (administered in an amount of 20 mg/kg/day) developed less severe multiple sclerosis (clinical scores from 0 to 1) when compared to untreated control animals (clinical scores ranging from 3 to 4). Animals treated with mitoxantrone (the only drug approved for multiple sclerosis) gave similar results as the polysubstituted lumazine of example 42. However, mitoxantrone induces heart and kidney toxicity, whereas our lumazine did not show any sign of toxicity.

## Claims

1. A poly-substituted pteridinedione (lumazine), or a mono- or polysubstituted 2-thiolumazine, 4-thiolumazine or 2,4-dithiolumazine being represented by the

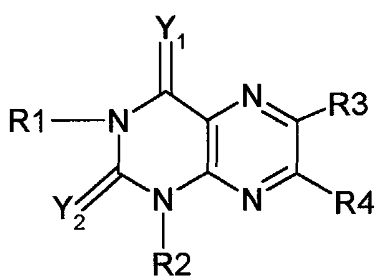

general formula (I),
wherein:

a) if $Y_1$ and $Y_2$ are both oxygen and $R_4$ is hydrogen, then:

- $R_1$ is a radical selected from the group consisting of hydrogen; $C_{1-5}$ alkyl; $C_{2-7}$ alkenyl; aryl; alkylaryl; ω-hydroxy $C_{1-7}$ alkyl; ω-epoxy $C_{1-7}$ alkyl; ω-carboxy $C_{1-7}$ alkyl (wherein the carboxy group may be acid, ester, thioester, acid halide or amide); ω-cyano $C_{1-7}$ alkyl; arylalkyl; arylalkenyl; heterocyclic-substituted alkyl; heterocyclic-substituted alkenyl; groups having the formula -S-R (i.e. wherein a sulfur atom is attached to the nitrogen atom of the pteridine ring) wherein R is a monovalent group selected from the group consisting of $C_{1-7}$ alkyl, aryl and $C_{3-10}$ cycloalkyl and wherein the said monovalent group is optionally substituted with one or more substituents selected from the group consisting of amino, amino-acid, alkylamino, arylamino, cycloalkylamino, carboxylic acid, carboxylic ester, sulfonic acid and phosphonic acid; and optionally substituted heterocyclic radicals;
- $R_2$ is a radical selected from the group consisting of hydrogen; $C_{1-5}$ alkyl; $C_{2-7}$ alkenyl; aryl; alkylaryl; ω-hydroxy $C_{1-7}$ alkyl; ω-epoxy $C_{1-7}$ alkyl; ω-carboxy $C_{1-7}$ alkyl (wherein the carboxy group may be acid, ester, thioester, acid halide or amide); ω-cyano $C_{1-7}$ alkyl; arylalkyl; arylalkenyl; heterocyclic-substituted alkyl; heterocyclic-substituted alkenyl; groups having the formula -S-R (i.e.
wherein a sulfur atom is attached to the nitrogen atom of the pteridine ring)
wherein R is a monovalent group selected from the group consisting of $C_{1-7}$ alkyl, aryl and $C_{3-10}$ cycloalkyl and wherein the said monovalent group is optionally substituted with one or more substituents selected from the group consisting of amino, amino-acid, alkylamino, arylamino, cycloalkylamino, carboxylic acid, carboxylic ester, sulfonic acid and phosphonic acid; and optionally substituted heterocyclic radicals;
- at most one of $R_1$ and $R_2$ is hydrogen; and

- $R_3$ is an atom or radical selected from the group consisting of fluoro; iodo; $C_{3-4}$ alkyl; $C_{2-7}$ alkenyl; $C_{2-7}$ alkynyl; $C_{3-4}$ haloalkyl; $C_{1-2}$ haloalkyl wherein halo is fluoro or chloro; $C_{1-4}$ alkoxy; $C_{3-10}$ cycloalkoxy; aryloxy; arylalkyloxy; oxyheterocyclic; heterocyclic-substituted alkyloxy; thio $C_{1-7}$ alkyl; thio $C_{3-10}$ cycloalkyl; thioaryl; thioheterocyclic; arylalkylthio; heterocyclic-substituted alkylthio; hydroxylamino; acetal; carboxylic acid esters, thioesters and amides; thiocarboxylic acid; thiocarboxylic acid esters, thioesters and amides; sulfhydryl; $C_{2-7}$ alkylamino; cycloalkylamino; alkenylamino; cycloalkenylamino; alkynylamino; arylamino; arylalkylamino; hydroxyalkylamino; mercaptoalkyl-amino; heterocyclic amino; heterocyclic-substituted alkylamino; oximino; alkyloximino; hydrazino; alkylhydrazino; phenylhydrazino; cysteinyl acid, esters or amides; aryl substituted with one or more substituents selected from the group consisting of halogen, $C_{1-4}$ alkyl, $C_{3-7}$ alkenyl, $C_{2-7}$ alkynyl, halo $C_{1-7}$ alkyl, $C_{2-4}$ alkoxy, hydroxyl, sulfhydryl, amino, $C_{3-10}$ cycloalkoxy, aryloxy, arylalkyloxy, oxyheterocyclic, heterocyclic-substituted alkyloxy, thio $C_{1-7}$ alkyl, thio $C_{3-10}$ cycloalkyl, thioaryl, thioheterocyclic, arylalkylthio, heterocyclic-substituted alkylthio, formyl, hydroxylamino, cyano, carboxylic acid or esters or thioesters or amides thereof, thiocarboxylic acid or esters or thioesters or amides thereof, $C_{1-7}$ alkylamino, cycloalkylamino, alkenylamino, cycloalkenylamino, alkynylamino, arylamino, arylalkylamino, hydroxyalkylamino, mercaptoalkylamino, heterocyclic amino, hydrazino, alkylhydrazino and phenylhydrazino; optionally substituted heterocyclic radicals; aryl or heterocyclic radicals substituted with an aliphatic spacer between the pteridine ring and the aryl or heterocyclic radical, whereby said aliphatic spacer is a branched or straight, saturated or unsaturated aliphatic chain of 2 to 4 carbon atoms which may contain one or more functions, atoms or radicals selected from the group consisting of carbonyl (oxo), alcohol (hydroxyl), ether, acetal, amino, imino, oximino, alkyloximino, amino-acid, cyano, carboxylic acid or ester or thioester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxyalkyl-amino, mercaptoalkylamino, heterocyclic amino, hydrazino, alkylhydrazino, phenylhydrazino, sulfonyl, sulfonamido and halogen, or whereby said aliphatic spacer is a methylene group containing a function, atom or radical chosen from the group consisting of carbonyl (oxo), alcohol (hydroxyl), ether, acetal, amino, imino, oximino, alkyloximino, amino-acid, cyano, carboxylic acid or ester or thioester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxylalkylamino, mercaptoalkylamino, heterocyclic amino, hydrazino, alkylhydrazino, phenylhydrazino, sulfonyl, sulfonamido and halogen; branched or straight, saturated or unsaturated aliphatic chain of 3 to 7 carbon atoms containing one or more functions selected from the group consisting of carbonyl (oxo), alcohol (hydroxyl), ether, acetal, amino, imino, oximino, alkyloximino, amino-acid, cyano, carboxylic acid ester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxyalkylamino, mercaptoalkylamino, heterocyclic amino, hydrazino, alkylhydrazino, phenylhydrazino, sulfonyl, sulfonamido and halogen; hydroxyethyl; oximinoethyl; alkyloximinoethyl; and methyl or ethyl or ethenyl containing one or more functions or radicals selected from the group consisting of ether, acetal, amino, imino, amino-acid, cyano, carboxylic acid or ester or thioester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylalkyl-amino, hydroxyalkylamino, mercaptoalkylamino, heterocyclic amino, hydrazino, alkylhydrazino, phenylhydrazino, sulfonyl, sulfonamido, fluoro and chloro;

b) if $Y_1$ and $Y_2$ are both oxygen and $R_3$ is hydrogen, then:

- $R_1$ is a radical selected from the group consisting of hydrogen; $C_{1-5}$ alkyl; $C_{2-7}$ alkenyl; aryl; alkylaryl; ω-hydroxy $C_{1-5}$ alkyl; ω-epoxy $C_{1-5}$ alkyl; ω-carboxy $C_{1-5}$ alkyl (wherein the carboxy group may be acid, ester, thioester, acid halide or amide); ω-cyano $C_{1-7}$ alkyl; arylalkyl; arylalkenyl; heterocyclic-substituted alkyl; heterocyclic-substituted alkenyl; groups having the formula -S-R (i.e.
  wherein a sulfur atom is attached to the nitrogen atom of the pteridine ring)
  wherein R is a monovalent group selected from the group consisting of $C_{1-7}$ alkyl, aryl and $C_{3-10}$ cycloalkyl and wherein the said monovalent group is optionally substituted with one or more substituents selected from the group consisting of amino, amino-acid, alkylamino, arylamino, cycloalkylamino, carboxylic acid, carboxylic ester, sulfonic acid and phosphonic acid; and optionally substituted heterocyclic radicals;
- $R_2$ is a radical selected from the group consisting of hydrogen; $C_{1-5}$ alkyl; $C_{2-7}$ alkenyl; aryl; alkylaryl; ω-hydroxy $C_{1-7}$ alkyl; ω-epoxy $C_{1-7}$ alkyl; ω-carboxy $C_{1-7}$ alkyl (wherein the carboxy group may be acid, ester, thioester, acid halide or amide); ω-cyano $C_{1-7}$ alkyl; arylalkyl; arylalkenyl; heterocyclic-substituted alkyl; heterocyclic-substituted alkenyl; groups having the formula -S-R (i.e. wherein a sulfur atom is attached to the nitrogen atom of the pteridine ring) wherein R is a monovalent group selected from the group consisting of $C_{1-7}$ alkyl, aryl and $C_{3-10}$ cycloalkyl and wherein the said monovalent group is optionally

substituted with one or more substituents selected from the group consisting of amino, amino-acid, alkylamino, arylamino, cycloalkylamino, carboxylic acid, carboxylic ester, sulfonic acid and phosphonic acid; and optionally substituted heterocyclic radicals;

- at most one of $R_1$ and $R_2$ is hydrogen; and
- R4 is an atom or radical selected from the group consisting of fluoro; iodo; $C_{3-4}$ alkyl; $C_{2-7}$ alkenyl; $C_{2-7}$ alkynyl; halo $C_{3-4}$ alkyl; halo $C_{1-2}$ alkyl wherein halo is fluoro or chloro; $C_{1-4}$ alkoxy; $C_{3-10}$ cycloalkoxy; aryloxy; arylalkyloxy; oxyheterocyclic; heterocyclic-substituted alkyloxy; thio $C_{1-7}$ alkyl; thio $C_{3-10}$ cycloalkyl; thioaryl; thioheterocyclic; arylalkylthio; heterocyclic-substituted alkylthio; hydroxylamino; acetal; carboxylic acid esters, thioesters and amides; thiocarboxylic acid; thiocarboxylic acid esters, thioesters and amides; sulfhydryl; $C_{2-7}$ alkylamino; cycloalkylamino; alkenylamino; cycloalkenylamino; alkynylamino; arylamino; arylalkylamino; hydroxyalkylamino; mercaptoalkyl-amino; heterocyclic amino; heterocyclic-substituted alkylamino; oximino; alkyloximino; hydrazino; alkylhydrazino; phenylhydrazino; cysteinyl acid, esters or amides; aryl substituted with one or more substituents selected from the group consisting of halogen, nitro, $C_{1-4}$ alkyl, $C_{2-7}$ alkenyl, $C_{2-7}$ alkynyl, halo $C_{1-4}$ alkyl, $C_{2-4}$ alkoxy, hydroxyl, sulfhydryl, amino, $C_{3-10}$ cycloalkoxy, aryloxy, arylalkyloxy, oxyheterocyclic, heterocyclic-substituted alkyloxy, thio $C_{1-7}$ alkyl, thio $C_{3-10}$ cycloalkyl, thioaryl, thioheterocyclic, arylalkylthio, heterocyclic-substituted alkylthio, formyl, hydroxylamino, cyano, carboxylic acid or esters or thioesters or amides thereof, thiocarboxylic acid or esters or thioesters or amides thereof, $C_{1-7}$ alkylamino, cycloalkylamino, alkenylamino, cycloalkenylamino, alkynylamino, arylamino, arylalkylamino, hydroxylalkylamino, mercaptoalkylamino, heterocyclic amino, hydrazino, alkylhydrazino and phenylhydrazino; optionally substituted heterocyclic radicals; aryl or heterocyclic radicals substituted with an aliphatic spacer between the pteridine ring and the aryl or heterocyclic radical, whereby said aliphatic spacer is a branched or straight, saturated or unsaturated aliphatic chain of 2 to 4 carbon atoms which may contain one or more functions, atoms or radicals selected from the group consisting of carbonyl (oxo), alcohol (hydroxyl), ether, acetal, amino, imino, oximino, alkyloximino, amino-acid, cyano, carboxylic acid or ester or thioester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxyalkyl-amino, mercaptoalkylamino, heterocyclic amino, hydrazino, alkylhydrazino, phenylhydrazino, sulfonyl, sulfonamido and halogen, or whereby said aliphatic spacer is a methylene group containing a function, atom or radical chosen from the group consisting of carbonyl (oxo), alcohol (hydroxyl), ether, acetal, amino, imino, oximino, alkyloximino, amino-acid, cyano, carboxylic acid or ester or thioester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxylalkylamino, mercaptoalkylamino, heterocyclic amino, hydrazino, alkylhydrazino, phenylhydrazino, sulfonyl, sulfonamido, fluoro and chloro; branched or straight, saturated or unsaturated aliphatic chain of 3 to 7 carbon atoms containing one or more functions selected from the group consisting of carbonyl (oxo), alcohol (hydroxyl), ether, acetal, amino, imino, oximino, alkyloximino, amino-acid, cyano, carboxylic acid ester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxyalkylamino, mercaptoalkyl-amino, heterocyclic amino, hydrazino, alkylhydrazino, phenylhydrazino, sulfonyl, sulfonamido and halogen; hydroxyethyl; oximinoethyl; alkyloximinoethyl; and methyl or ethyl or ethenyl containing one or more functions, atoms or radicals selected from the group consisting of ether, acetal, amino, imino, amino-acid, cyano, carboxylic acid or ester or thioester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylalkyl-amino, hydroxyalkylamino, mercaptoalkylamino, heterocyclic amino, hydrazino, alkylhydrazino, phenylhydrazino, sulfonyl, sulfonamido, fluoro and chloro;

c) if one or more of $Y_1$ and $Y_2$ is sulfur and at most one of $Y_1$ and $Y_2$ is oxygen, then:

- each of $R_1$ and $R_2$ is a radical independently selected from the group consisting of hydrogen; $C_{1-7}$ alkyl; $C_{2-7}$ alkenyl; aryl; alkylaryl; ω-hydroxy $C_{1-7}$ alkyl; ω-epoxy $C_{1-7}$ alkyl; ω-carboxy $C_{1-7}$ alkyl (wherein the carboxy group may be acid, ester, thioester, acid halide or amide); ω-cyano $C_{1-7}$ alkyl; arylalkyl; arylalkenyl; heterocyclic-substituted alkyl; heterocyclic-substituted alkenyl; groups having the formula -S-R (i.e. wherein a sulfur atom is attached to the nitrogen atom of the pteridine ring) wherein R is a monovalent group selected from the group consisting of $C_{1-7}$ alkyl, aryl and $C_{3-10}$ cycloalkyl and wherein the said monovalent group is optionally substituted with one or more substituents selected from the group consisting of amino, amino-acid, alkylamino, arylamino, cycloalkylamino, carboxylic acid, carboxylic ester, sulfonic acid and phosphonic acid; and optionally substituted heterocyclic radicals;
- each of $R_3$ and $R_4$ is an atom or radical independently selected from the group consisting of hydrogen; halogen; $C_{2-4}$ alkyl; $C_{2-7}$ alkenyl; $C_{2-7}$ alkynyl; halo $C_{1-4}$ alkyl; $C_{2-4}$ alkoxy; $C_{3-10}$ cycloalkoxy; aryloxy;

arylalkyloxy; oxyheterocyclic; heterocyclic-substituted alkyloxy; thio $C_{2-7}$ alkyl; thio $C_{3-10}$ cycloalkyl; thioaryl; thioheterocyclic; arylalkylthio; heterocyclic-substituted alkylthio; hydroxylamino; acetal; formyl; cyano; carboxylic acid; carboxylic acid esters, thioesters and amides; thiocarboxylic acid; thiocarboxylic acid esters, thioesters and amides; amino; alkylamino; cycloalkylamino; alkenylamino; cycloalkenylamino; alkynylamino; arylamino; arylalkylamino; hydroxyalkylamino; mercaptoalkylamino; heterocyclic amino; heterocyclic-substituted alkylamino; oximino; alkyloximino; hydrazino; alkylhydrazino; phenylhydrazino; cysteinyl acid, esters or amides; aryl substituted with one or more substituents selected from the group consisting of halogen, $C_{1-4}$ alkyl, $C_{2-7}$ alkenyl, $C_{2-7}$ alkynyl, halo $C_{1-4}$ alkyl, $C_{1-4}$ alkoxy, hydroxyl, sulfhydryl, amino, $C_{3-10}$ cycloalkoxy, aryloxy, arylalkyloxy, oxyheterocyclic, heterocyclic-substituted alkyloxy, thio $C_{1-7}$ alkyl, thio $C_{3-10}$ cycloalkyl, thioaryl, thioheterocyclic, arylalkylthio, heterocyclic-substituted alkylthio, formyl, hydroxylamino, cyano, carboxylic acid or esters or thioesters or amides thereof, thiocarboxylic acid or esters or thioesters or amides thereof, $C_{1-7}$ alkylamino, cycloalkylamino, alkenylamino, cycloalkenylamino, alkynylamino, arylamino, arylalkylamino, hydroxyalkylamino, mercaptoalkylamino, heterocyclic amino, hydrazino, alkylhydrazino and phenylhydrazino; optionally substituted heterocyclic radicals; aryl or heterocyclic radicals substituted with an aliphatic spacer between the pteridine ring and the aryl or heterocyclic radical, whereby said aliphatic spacer is a branched or straight, saturated or unsaturated aliphatic chain of 1 to 4 carbon atoms which may contain one or more functions, atoms or radicals selected from the group consisting of carbonyl (oxo), alcohol (hydroxyl), ether, acetal, amino, imino, oximino, alkyloximino, amino-acid, cyano, carboxylic acid or ester or thioester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxyalkyl-amino, mercaptoalkylamino, heterocyclic amino, hydrazino, alkylhydrazino, phenylhydrazino, sulfonyl, sulfonamido and halogen; branched or straight, saturated or unsaturated aliphatic chain of 1 to 7 carbon atoms containing one or more functions selected from the group consisting of carbonyl (oxo), alcohol (hydroxyl), ether, acetal, amino, imino, oximino, alkyloximino, amino-acid, cyano, carboxylic acid ester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxylalkylamino, mercaptoalkyl-amino, heterocyclic amino, hydrazino, alkylhydrazino, phenylhydrazino, sulfonyl, sulfonamido and halogen; or $R_4$ and $R_3$ together form an aryl radical being optionally substituted with one or more substituents $R_a$ each independently selected from the group consisting of amino, alkylamino, cycloalkylamino, alkenylamino, cycloalkenylamino, alkynylamino, arylamino, arylalkylamino, hydroxyalkylamino, mercaptoalkylamino, heterocyclic amino and heterocyclic-substituted alkylamino, wherein each substituent $R_a$ may further comprise one or more functions selected from the group consisting of carbonyl, amino and carboxyl, and wherein two adjacent substituents $R_a$ may together form an heterocyclic radical; and

- at most one of $R_1$, $R_2$, $R_3$ and $R_4$ is hydrogen;

d) if $Y_1$ and $Y_2$ are both oxygen and none of $R_3$ and $R_4$ is hydrogen, then:

- $R_2$ is a radical selected from the group consisting of $C_{1-7}$ alkyl; $C_{2-7}$ alkenyl; aryl; alkylaryl; ω-hydroxy $C_{1-7}$ alkyl; ω-epoxy $C_{1-7}$ alkyl; ω-carboxy $C_{1-7}$ alkyl (wherein the carboxy group may be acid, ester, thioester, acid halide or amide); ω-cyano $C_{1-7}$ alkyl; arylalkyl; arylalkenyl; heterocyclic-substituted alkyl; heterocyclic-substituted alkenyl; groups having the formula -S-R (i.e.
wherein a sulfur atom is attached to the nitrogen atom of the pteridine ring)
wherein R is a monovalent group selected from the group consisting of $C_{1-7}$ alkyl, aryl and $C_{3-10}$ cycloalkyl and wherein the said monovalent group is optionally substituted with one or more substituents selected from the group consisting of amino, amino-acid, alkylamino, arylamino, cycloalkylamino, carboxylic acid, carboxylic ester, sulfonic acid and phosphonic acid; and optionally substituted heterocyclic radicals;
- $R_1$ is an atom or radical independently defined as $R_2$, or is hydrogen;
- $R_4$ is an atom or radical selected from the group consisting of halogen; $C_{2-7}$ alkenyl; $C_{2-7}$ alkynyl; $C_{2-7}$ haloalkyl; fluoromethyl; $C_{2-4}$ alkoxy; $C_{3-10}$ cycloalkoxy; aryloxy; arylalkyloxy; oxyheterocyclic; heterocyclic-substituted alkyloxy; thio $C_{1-7}$ alkyl; thio $C_{3-10}$ cycloalkyl; thioaryl; thioheterocyclic; arylalkylthio; heterocyclic-substituted alkylthio; hydroxylamino; acetal; carboxylic acid thioesters and amides; thiocarboxylic acid; thiocarboxylic acid esters, thioesters and amides; sulfhydryl; $C_{2-7}$ alkylamino; cycloalkylamino; alkenylamino; cycloalkenylamino; alkynylamino; arylamino; arylalkylamino; hydroxyalkylamino; mercaptoalkyl-amino; heterocyclic amino; heterocyclic-substituted alkylamino; hydrazino; alkylhydrazino; phenylhydrazino; cysteinyl acid, esters or amides; aryl optionally substituted with one or more substituents selected from the group consisting of halogen, $C_{1-4}$ alkyl, $C_{2-7}$ alkenyl, $C_{2-7}$ alkynyl, $C_{1-4}$ haloalkyl, $C_{1-4}$ alkoxy, hydroxyl, sulfhydryl, amino, $C_{3-10}$ cycloalkoxy, aryloxy, arylalkyloxy, oxyheterocyclic, heterocyclic-substituted alkyloxy, thio $C_{1-7}$ alkyl, thio $C_{3-10}$ cycloalkyl, thioaryl, thioheterocyclic, arylalkylthio, heterocyclic-substituted alkylthio, formyl, hydroxylamino, cyano, carboxylic acid or esters or thioesters or amides thereof, thiocarboxylic acid or esters or thioesters or amides thereof, $C_{1-7}$ alkylamino, cycloalkylamino, alkenylamino, cycloalkenylamino, alkynylamino, arylamino, arylalkylamino, hydroxyalkylamino, mercaptoalkylamino, heterocyclic amino, hydrazino, alkylhydrazino and phenylhydrazino; optionally substituted heterocyclic radicals; aryl or heterocyclic radicals substituted with an aliphatic spacer between the pteridine ring and the aryl or heterocyclic radical, whereby said aliphatic spacer is a branched or straight, saturated or unsaturated aliphatic chain of 1 to 4 carbon atoms which may contain one or more functions, atoms or radicals selected from the group consisting of carbonyl (oxo), alcohol (hydroxyl), ether, acetal, amino, imino, oximino, alkyloximino, amino-acid, cyano, carboxylic acid or ester or thioester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxyalkyl-amino, mercaptoalkylamino, heterocyclic amino, hydrazino, alkylhydrazino, phenylhydrazino, sulfonyl, sulfonamido and halogen; branched or straight, saturated or unsaturated aliphatic chain of 2 to 7 carbon atoms containing one or more functions selected from the group consisting of carbonyl (oxo), alcohol (hydroxyl), ether, acetal, amino, imino, oximino, alkyloximino, amino-acid, cyano, carboxylic acid ester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxylalkylamino, mercaptoalkyl-amino, heterocyclic amino, hydrazino, alkylhydrazino, phenylhydrazino, sulfonyl, sulfonamido and halogen; hydroxyethyl; and

- $R_3$ is an atom or radical independently defined as $R_4$, or is amino or methoxy, or $R_4$ and $R_3$ together form an aryl radical being optionally substituted with one or more substituents $R_a$ each independently selected from the group consisting of amino, alkylamino, cycloalkylamino, alkenylamino, cycloalkenylamino, alkynylamino, arylamino, arylalkylamino, hydroxyalkylamino, mercaptoalkylamino, heterocyclic amino and heterocyclic-substituted alkylamino, wherein each substituent $R_a$ may further comprise one or more functions selected from the group consisting of carbonyl, amino and carboxyl, and wherein two adjacent substituents $R_a$ may together form an heterocyclic radical;

e) if $Y_1$ and $Y_2$ are both oxygen and none of $R_3$ and $R_4$ is hydrogen, then:

- $R_1$ is a radical selected from the group consisting of $C_{2-7}$alkyl; $C_{2-7}$alkenyl; aryl; alkylaryl; ω-hydroxy $C_{1-7}$ alkyl; ω-epoxy $C_{1-7}$ alkyl; ω-carboxy $C_{1-7}$ alkyl (wherein the carboxy group may be acid, ester, thioester, acid halide or amide); ω-cyano $C_{1-7}$ alkyl; arylalkyl; arylalkenyl; heterocyclic-substituted alkyl; heterocyclic-substituted alkenyl; groups having the formula -S-R (i.e.
  wherein a sulfur atom is attached to the nitrogen atom of the pteridine ring)
  wherein R is a monovalent group selected from the group consisting of $C_{1-7}$ alkyl, aryl and $C_{3-10}$ cycloalkyl and wherein the said monovalent group is optionally substituted with one or more substituents selected from the group consisting of amino, amino-acid, alkylamino, arylamino, cycloalkylamino, carboxylic acid, carboxylic ester, sulfonic acid and phosphonic acid; and optionally substituted heterocyclic radicals;
- $R_2$ is hydrogen;
- $R_3$ is an atom or radical selected from the group consisting of fluorine; bromine; iodine; $C_{2-7}$ alkyl; $C_{2-7}$ alkenyl; $C_{2-7}$ alkynyl; $C_{1-7}$ haloalkyl; $C_{1-4}$ alkoxy; $C_{3-10}$ cycloalkoxy; aryloxy; arylalkyloxy; oxyheterocyclic; heterocyclic-substituted alkyloxy; thio $C_{1-7}$ alkyl; thio $C_{3-10}$ cycloalkyl; thioaryl; thioheterocyclic; arylalkylthio; heterocyclic-substituted alkylthio; hydroxylamino; acetal; carboxylic acid esters, thioesters and amides; thiocarboxylic acid; thiocarboxylic acid esters, thioesters and amides; sulfhydryl; amino; alkylamino; cycloalkylamino; alkenylamino; cycloalkenylamino; alkynylamino; arylamino; arylalkylamino; hydroxyalkylamino; mercaptoalkylamino; heterocyclic amino; heterocyclic-substituted alkylamino; hydrazino; alkylhydrazino; phenylhydrazino; cysteinyl acid, esters or amides; aryl substituted with one or more substituents selected from the group consisting of halogen, $C_{1-4}$ alkyl, $C_{2-7}$ alkenyl, $C_{2-7}$ alkynyl, $C_{1-4}$ haloalkyl, $C_{1-4}$ alkoxy, hydroxyl, sulfhydryl, amino, $C_{3-10}$ cycloalkoxy, aryloxy, arylalkyloxy, oxyheterocyclic, heterocyclic-substituted alkyloxy, thio $C_{1-7}$ alkyl, thio $C_{3-10}$ cycloalkyl, thioaryl, thioheterocyclic, arylalkylthio, heterocyclic-substituted alkylthio, formyl, hydroxylamino, cyano, carboxylic acid or esters or thioesters or amides thereof, thiocarboxylic acid or esters or thioesters or amides thereof, $C_{1-7}$ alkylamino, cycloalkylamino, alkenylamino, cycloalkenylamino, alkynylamino, arylamino, arylalkylamino, hydroxyalkylamino, mercaptoalkylamino, heterocyclic amino, hydrazino, alkylhydrazino and phenylhydrazino; optionally substituted heterocyclic radicals; aryl or heterocyclic radicals substituted with an aliphatic spacer between the pteridine ring and the aryl or heterocyclic radical, whereby said aliphatic spacer is a branched or straight, saturated or unsaturated aliphatic chain of 1 to 4 carbon atoms which may contain one or more functions, atoms or radicals selected from the group consisting of carbonyl (oxo), alcohol (hydroxyl), ether, acetal, amino, imino, oximino, alkyloximino, amino-acid, cyano, carboxylic acid or ester or thioester or

amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxyalkylamino, mercaptoalkylamino, heterocyclic amino, hydrazino, alkylhydrazino, phenylhydrazino, sulfonyl, sulfonamido and halogen; branched or straight, saturated or unsaturated aliphatic chain of 1 to 7 carbon atoms containing one or more functions selected from the group consisting of carbonyl (oxo), alcohol (hydroxyl), ether, acetal, amino, imino, oximino, alkyloximino, amino-acid, cyano, carboxylic acid ester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxyalkylamino, mercaptoalkyl-amino, heterocyclic amino, hydrazino, alkylhydrazino, phenylhydrazino, sulfonyl, sulfonamido and halogen; hydroxyethyl; and

- $R_4$ is an atom or radical independently defined as $R_3$, or is chloro, or $R_4$ and $R_3$ together form an aryl radical being optionally substituted with one or more substituents $R_a$ each independently selected from the group consisting of amino, alkylamino, cycloalkylamino, alkenylamino, cycloalkenylamino, alkynylamino, arylamino, arylalkylamino, hydroxyalkylamino, mercaptoalkylamino, heterocyclic amino and heterocyclic-substituted alkylamino, wherein each substituent $R_a$ may further comprise one or more functions selected from the group consisting of carbonyl, amino and carboxyl, and wherein two adjacent substituents $R_a$ may together form an heterocyclic radical;

f) if $Y_1$ and $Y_2$ are both oxygen and $R_2$ and $R_3$ are both hydrogen, then:

- $R_1$ is a radical selected from the group consisting of $C_{1-7}$ alkyl; $C_{2-7}$ alkenyl; aryl; alkylaryl; ω-hydroxy $C_{1-7}$ alkyl; ω-epoxy $C_{1-7}$ alkyl; ω-carboxy $C_{1-7}$ alkyl (wherein the carboxy group may be acid, ester, thioester, acid halide or amide); ω-cyano $C_{1-7}$ alkyl; arylalkyl; arylalkenyl; heterocyclic-substituted alkyl; heterocyclic-substituted alkenyl; groups having the formula -S-R (i.e.
  wherein a sulfur atom is attached to the nitrogen atom of the pteridine ring)
  wherein R is a monovalent group selected from the group consisting of $C_{1-7}$ alkyl, aryl and $C_{3-10}$ cycloalkyl and wherein the said monovalent group is optionally substituted with one or more substituents selected from the group consisting of amino, amino-acid, alkylamino, arylamino, cycloalkylamino, carboxylic acid, carboxylic ester, sulfonic acid and phosphonic acid; and optionally substituted heterocyclic radicals;
- $R_4$ is an atom or a radical selected from the group consisting of halogen, cyano, amino, alkylamino, cycloalkylamino, alkenylamino, cycloalkenylamino, alkynylamino, arylamino, arylalkylamino, hydroxyalkylamino, mercaptoalkylamino, heterocyclic amino and heterocyclic-substituted alkylamino; or a pharmaceutically acceptable salt or an enantiomer thereof.

**2.** A poly-substituted pteridinedione (lumazine), or a mono- or polysubstituted 2-thiolumazine, 4-thiolumazine or 2,4-dithiolumazine according to claim 1, wherein $R_1$ and $R_2$ are independently benzyl, phenyl, 2-phenylethyl, butyric acid or ester, butyronitrile, 2-hydroxyethyl, ethyl or methyl.

**3.** A poly-substituted pteridinedione (lumazine), or a mono- or polysubstituted 2-thiolumazine, 4-thiolumazine or 2,4-dithiolumazine according to claim 1, wherein $R_4$ is chloro, hydroxy or phenoxy.

**4.** A poly-substituted pteridinedione (lumazine), or a mono- or polysubstituted 2-thiolumazine, 4-thiolumazine or 2,4-dithiolumazine according to claim 1, wherein $R_3$ is substituted phenyl.

**5.** A pharmaceutically acceptable salt according to claim 1, being obtained from a salt-forming acid or base selected from the group consisting of inorganic acids, organic monocarboxylic or dicarboxylic acids, inorganic bases and organic bases.

**6.** A pharmaceutical composition comprising one or more pharmaceutically acceptable carriers and a poly-substituted pteridinedione (lumazine), or a mono-or polysubstituted 2-thiolumazine, 4-thiolumazine or 2,4-dithiolumazine according to claim 1.

**7.** A pharmaceutical composition comprising one or more pharmaceutically acceptable carriers and a poly-substituted pteridinedione (lumazine), or a mono-or polysubstituted 2-thiolumazine, 4-thiolumazine or 2,4-dithiolumazine represented by the general formula (IV)

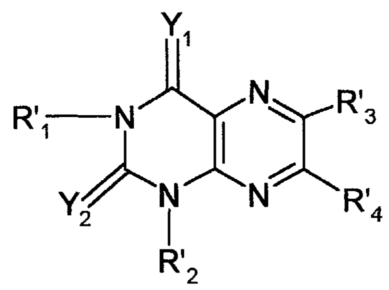

wherein:

- each of $Y_1$ and $Y_2$ is independently selected from sulfur and oxygen;
- each of $R'_1$ and $R'_2$ is a radical independently selected from the group consisting of hydrogen; $C_{1-7}$ alkyl; $C_{2-7}$ alkenyl; aryl; alkylaryl; ω-hydroxy $C_{1-7}$ alkyl; ω-epoxy $C_{1-7}$ alkyl; ω-carboxy $C_{1-7}$ alkyl (wherein the carboxy group may be acid, ester, thioester, acid halide or amide); ω-cyano $C_{1-7}$ alkyl; arylalkyl; arylalkenyl; heterocyclic-substituted alkyl; heterocyclic-substituted alkenyl; groups having the formula -S-R (i.e. wherein a sulfur atom is attached to the nitrogen atom of the pteridine ring) wherein R is a monovalent group selected from the group consisting of $C_{1-7}$ alkyl, aryl and $C_{3-10}$ cycloalkyl and wherein the said monovalent group is optionally substituted with one or more substituents selected from the group consisting of amino, amino-acid, alkylamino, arylamino, cycloalkylamino, carboxylic acid, carboxylic ester, sulfonic acid and phosphonic acid; and optionally substituted heterocyclic radicals;
- at most one of $R'_1$, $R'_2$, $R'_3$ and $R'_4$ is hydrogen when one or more of $Y_1$ and $Y_2$ is sulfur;
- at most one of $R'_1$ and $R'_2$ is hydrogen when both $Y_1$ and $Y_2$ are oxygen;
- each of $R'_3$ and $R'_4$ is an atom or radical independently selected from the group consisting of hydrogen; halogen; $C_{1-4}$ alkyl; $C_{2-7}$ alkenyl; $C_{2-7}$ alkynyl; halo $C_{1-4}$ alkyl; $C_{1-4}$ alkoxy; $C_{3-10}$ cycloalkoxy; aryloxy; arylalkyloxy; oxyheterocyclic; heterocyclic-substituted alkyloxy; thio $C_{1-7}$ alkyl; thio $C_{3-10}$ cycloalkyl; thioaryl; thioheterocyclic; arylalkylthio; heterocyclic-substituted alkylthio; hydroxylamino; acetal; formyl; cyano; carboxylic acid; carboxylic acid esters, thioesters and amides; thiocarboxylic acid; thiocarboxylic acid esters, thioesters and amides; amino; $C_{2-7}$ alkylamino; cycloalkylamino; alkenylamino; cycloalkenylamino; alkynylamino; arylamino; arylalkylamino; hydroxyalkylamino; mercaptoalkylamino; heterocyclic amino; heterocyclic-substituted alkylamino; oximino; alkyloximino; hydrazino; alkylhydrazino; phenylhydrazino; cysteinyl acid, esters or amides; aryl optionally substituted with one or more substituents selected from the group consisting of halogen, $C_{1-4}$ alkyl, $C_{2-7}$ alkenyl, $C_{2-7}$ alkynyl, halo $C_{1-4}$ alkyl, $C_{1-4}$ alkoxy, hydroxyl, sulfhydryl, amino, $C_{3-10}$ cycloalkoxy, aryloxy, arylalkyloxy, oxyheterocyclic, heterocyclic-substituted alkyloxy, thio $C_{1-7}$ alkyl, thio $C_{3-10}$ cycloalkyl, thioaryl, thioheterocyclic, arylalkylthio, heterocyclic-substituted alkylthio, formyl, hydroxylamino, cyano, carboxylic acid or esters or thioesters or amides thereof, thiocarboxylic acid or esters or thioesters or amides thereof, $C_{1-7}$ alkylamino, cycloalkylamino, alkenylamino, cycloalkenylamino, alkynylamino, arylamino, arylalkylamino, hydroxyalkylamino, mercaptoalkylamino, heterocyclic amino, hydrazino, alkylhydrazino and phenylhydrazino; optionally substituted heterocyclic radicals; aryl or heterocyclic radicals substituted with an aliphatic spacer between the pteridine ring and the aryl or heterocyclic radical, whereby said aliphatic spacer is a branched or straight, saturated or unsaturated aliphatic chain of 1 to 4 carbon atoms which may contain one or more functions, atoms or radicals selected from the group consisting of carbonyl (oxo), alcohol (hydroxyl), ether, acetal, amino, imino, oximino, alkyloximino, amino-acid, cyano, carboxylic acid or ester or thioester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxyalkylamino, mercaptoalkylamino, heterocyclic amino, hydrazino, alkylhydrazino, phenylhydrazino, sulfonyl, sulfonamido and halogen; branched or straight, saturated or unsaturated aliphatic chain of 1 to 7 carbon atoms containing one or more functions selected from the group consisting of carbonyl (oxo), alcohol (hydroxyl), ether, acetal, amino, imino, oximino, alkyloximino, amino-acid, cyano, carboxylic acid ester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxylalkylamino, mercaptoalkyl-amino, heterocyclic amino, hydrazino, alkylhydrazino, phenylhydrazino, sulfonyl, sulfonamido and halogen, and
- $R'_4$ and $R'_3$ may together form an aryl radical being optionally substituted with one or more substituents $R_a$ each independently selected from the group consisting of amino, alkylamino, cycloalkylamino, alkenylamino, cycloalkenylamino, alkynylamino, arylamino, arylalkylamino, hydroxyalkylamino, mercaptoalkylamino, heterocyclic amino and heterocyclic-substituted alkylamino, wherein each substituent $R_a$ may further comprise one or more functions selected from the group consisting of carbonyl, amino and carboxyl, and wherein two adjacent substituents $R_a$ may together form an heterocyclic radical.

**8.** A pharmaceutical composition according to claim 6, wherein $R_1$ and $R_2$ are independently benzyl, phenyl, 2-phenylethyl, butyric acid or ester, butyronitrile, 2-hydroxyethyl, ethyl or methyl.

**9.** A pharmaceutical composition according to claim 6, wherein $R_4$ is chloro, hydroxy or phenoxy.

**10.** A pharmaceutical composition according to claim 6, wherein $R_3$ is substituted phenyl.

FIGURE 1

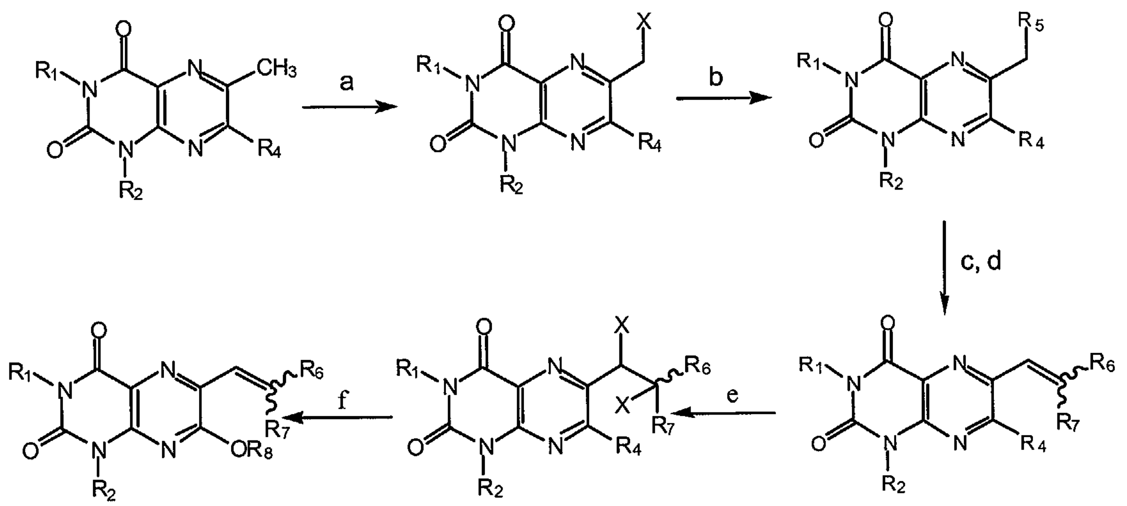


FIGURE 2

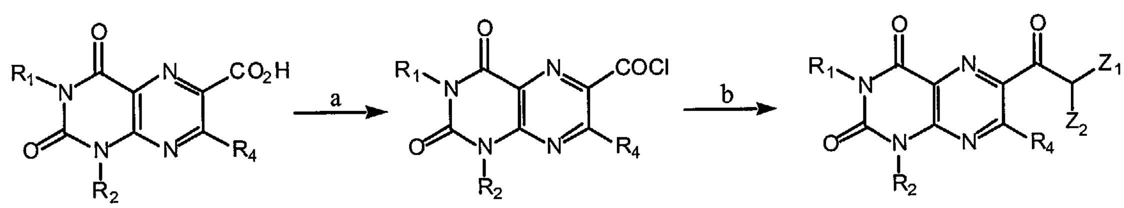


FIGURE 3

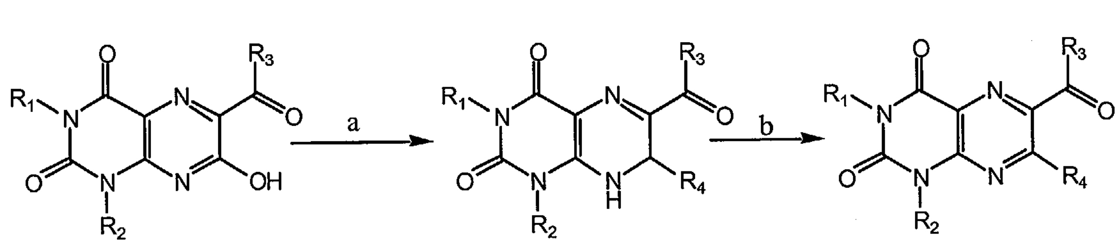

FIGURE 4

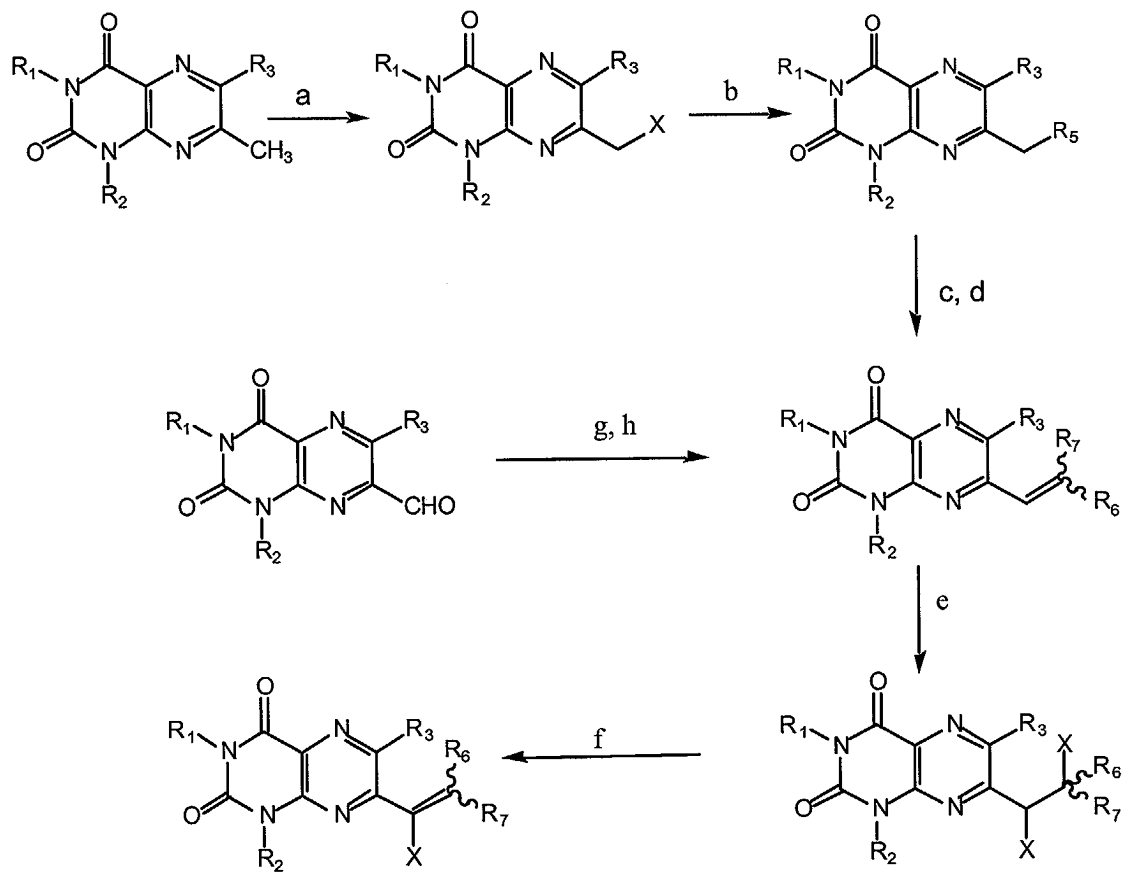

FIGURE 5

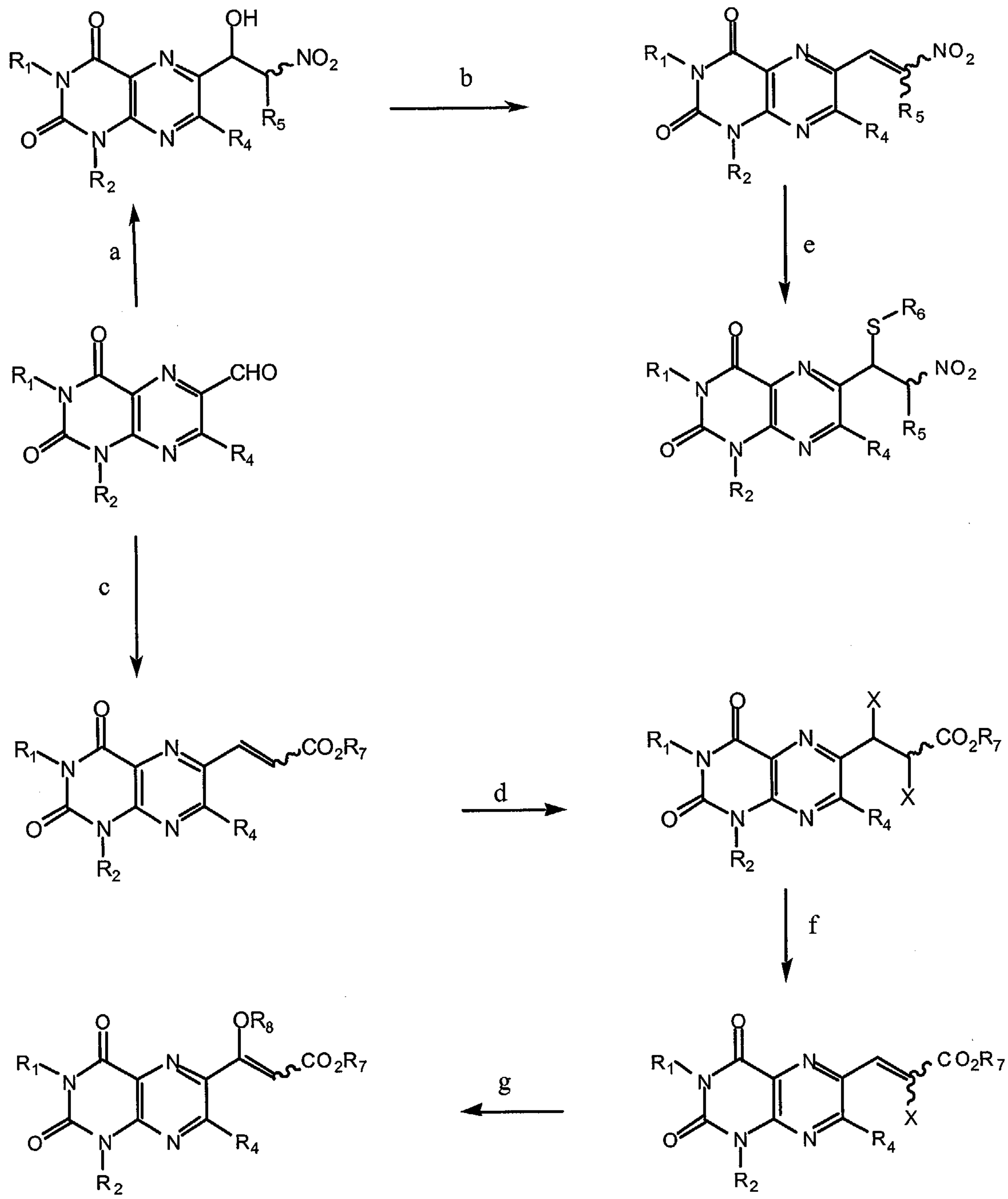

FIGURE 6

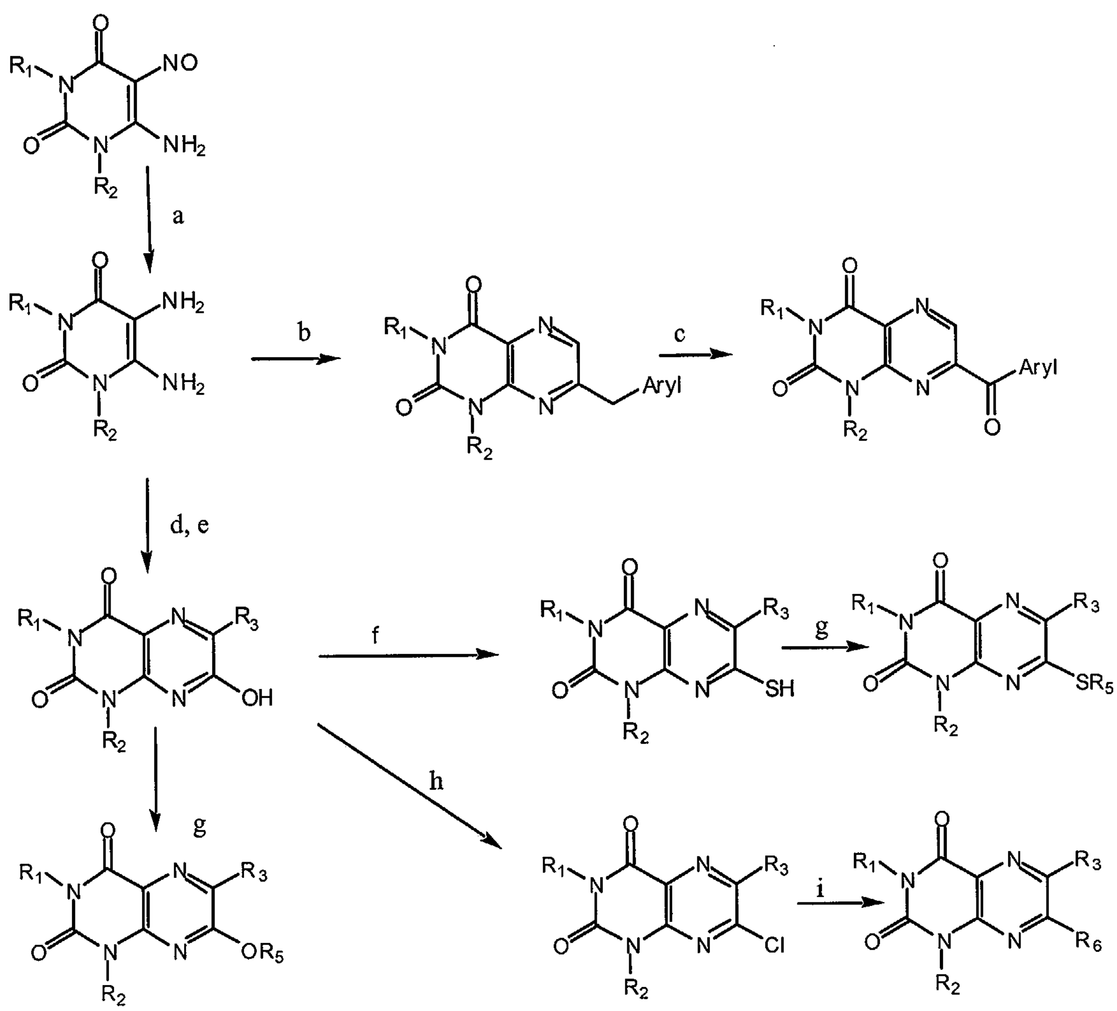

FIGURE 7

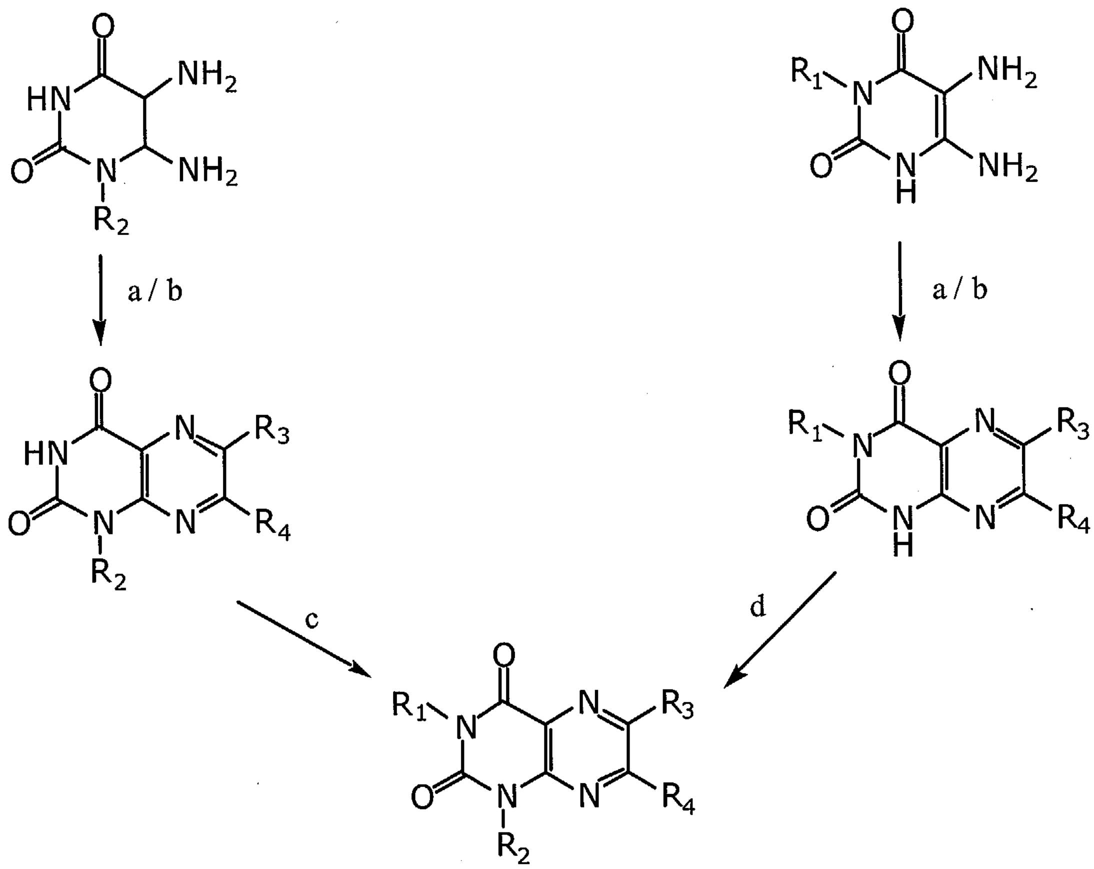

FIGURE 8

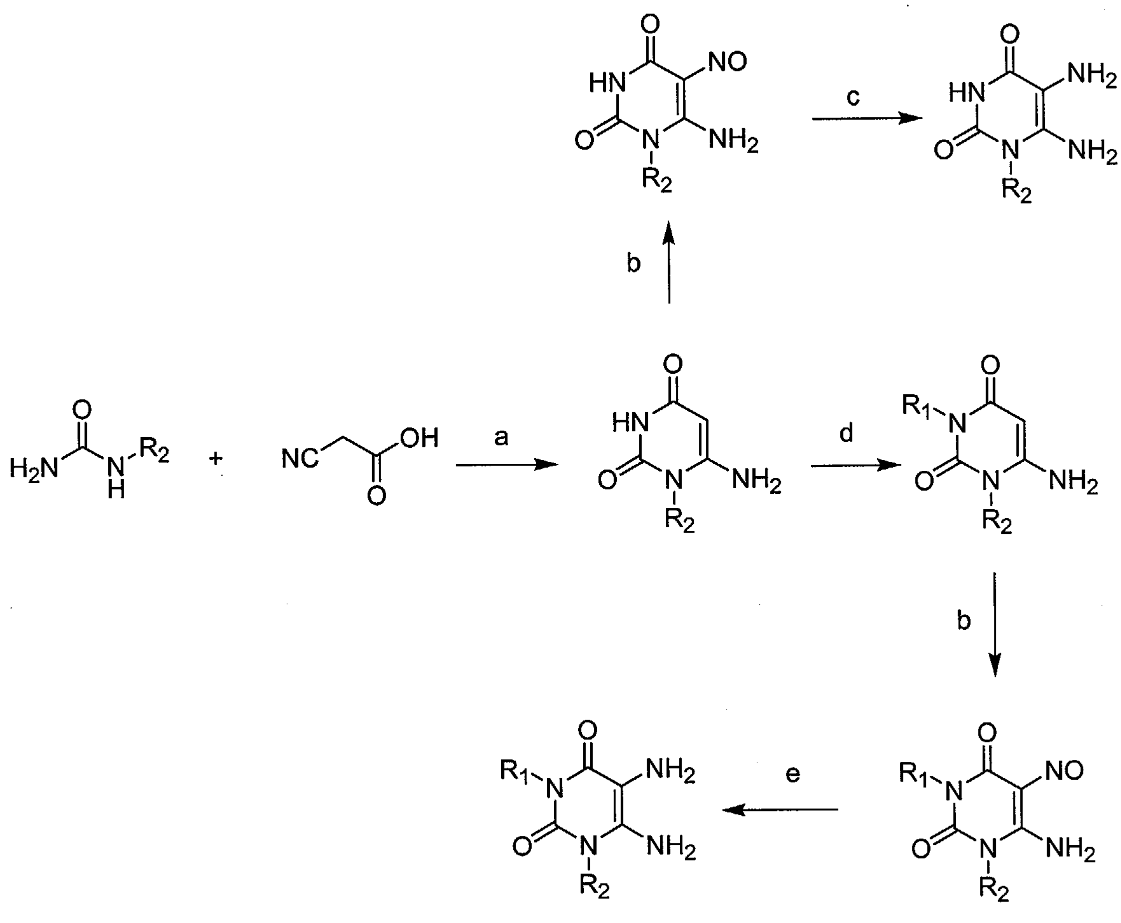

FIGURE 9

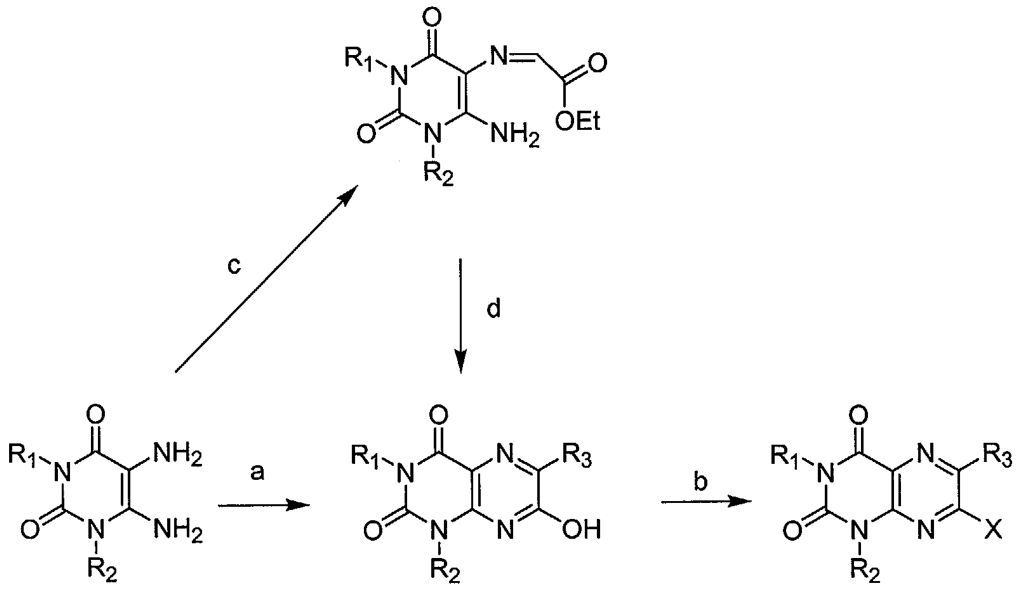


1 2 3

FIGURE 10

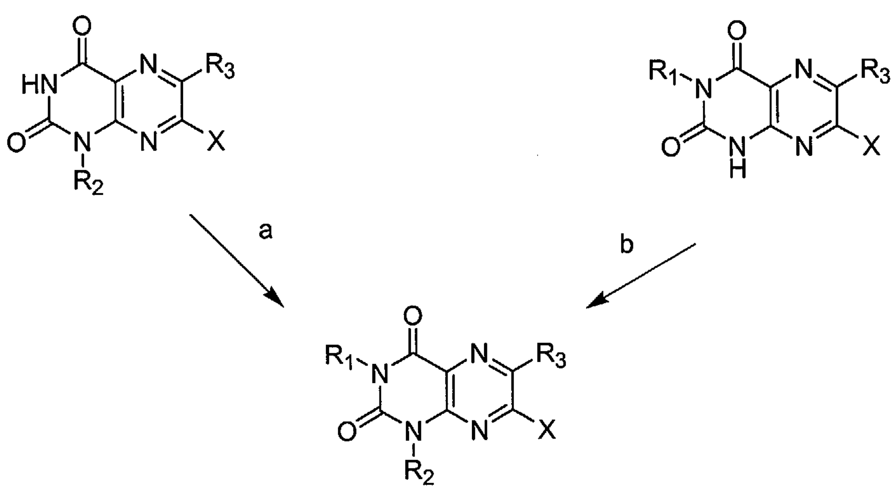


4

FIGURE 11

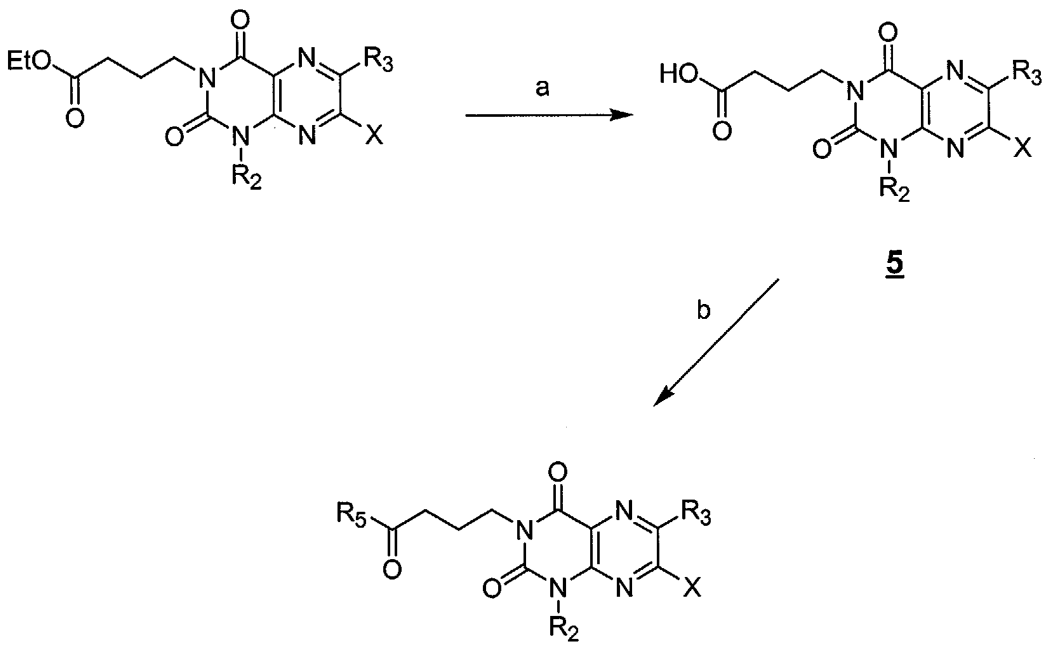


5

6

European Patent Office

# PARTIAL EUROPEAN SEARCH REPORT

which under Rule 45 of the European Patent Convention shall be considered, for the purposes of subsequent proceedings, as the European search report

Application Number EP 03 07 9183

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,X | US 3 071 587 A (W. V. CURRAN, R. B. ANGIER) 1 January 1963 (1963-01-01)<br>* claims 1-5 * | 1-4 | C07D475/00<br>C07D475/02<br>A61K31/519 |
| E | US 2003/236255 A1 (M. J. A. WAER ET AL.) 25 December 2003 (2003-12-25)<br>* claims 1-19 * | 1-10 | |
| D,X | WO 94/06431 A (CELL THERAPEUTICS, INC.) 31 March 1994 (1994-03-31)<br>* claims 1-10 * | 1-10 | |
| D,X | WO 00/45800 A (K. U. LEUVEN RESEARCH & DEVELOPMENT) 10 August 2000 (2000-08-10)<br>* claims 1-15 * | 1-10 | |
| X | EP 0 290 819 A (BIORESEARCH SPA) 17 November 1988 (1988-11-17)<br>* claims 1-14 * | 1-10 | |
| D,X | WO 94/11001 A (CELL THERAPEUTICS, INC.) 26 May 1994 (1994-05-26)<br>* claims 1-12 *<br>-/-- | 1-10 | TECHNICAL FIELDS SEARCHED (Int.Cl.7)<br>C07D<br>A61K |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 20 September 2004 | Herz, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

**INCOMPLETE SEARCH SHEET C**

Application Number
EP 03 07 9183

Claim(s) searched incompletely:
1-10

Claim(s) not searched:
-

Reason for the limitation of the search:

Present claims 1 to 10 relate to an extremely large number of possible compounds. Support within the meaning of Article 84 EPC and/or disclosure within the meaning of Article 83 EPC is to be found, however, for only a very small proportion of the compounds claimed. In the present case, the claims so lack support, and the application so lacks disclosure, that a meaningful search over the whole of the claimed scope is impossible.

Furthermore, the initial phase of the search revealed a very large number of documents relevant to the issue of novelty. So many documents were retrieved that it is impossible to determine which parts of the claim(s) may be said to define subject-matter for which protection might legitimately be sought (Article 84 EPC). For these reasons, a meaningful search over the whole breadth of the claims is impossible. Consequently, the search has been restricted to the examples.

European Patent Office

# PARTIAL EUROPEAN SEARCH REPORT

Application Number
EP 03 07 9183

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 5 843 943 A (D. A. CARSON ET AL.) 1 December 1998 (1998-12-01) * claims 1-9 * | 1-10 | |
| D,X | WO 98/52948 A (THE REGENTS OF THE UNIVERSITY OF CALIFORNIA) 26 November 1998 (1998-11-26) * claims 1-25 * | 1-10 | |
| X | DATABASE BEILSTEIN BEILSTEIN INSTITUTE FOR ORGANIC CHEMISTRY, FRANKFURT-MAIN, DE; XP002296933 Database accession no. 6337777,6373242 * abstract * & A. V. GULEVSKAYA ET AL.: HETEROCYC. COMPD., vol. 28, no. 9, 1992, pages 1015-1020, | 1-5 | TECHNICAL FIELDS SEARCHED (Int.Cl.7) |
| X | DATABASE BEILSTEIN BEILSTEIN INSTITUTE FOR ORGANIC CHEMISTRY, FRANKFURT-MAIN, DE; XP002296934 Database accession no. 285496,252276,250719 * abstract * & PFLEIDERER: CHEM. BER., vol. 90, 1957, page 2631, | 1-5 | |
| X | DATABASE BEILSTEIN BEILSTEIN INSTITUTE FOR ORGANIC CHEMISTRY, FRANKFURT-MAIN, DE; XP002296935 Database accession no. 533693,540145 * abstract * & HENSEKE, MUELLER: CHEM. BER., vol. 93, 1960, page 2668, | 1-5 | |

-/--

EPO FORM 1503 03.82 (P04C10)

European Patent Office

# PARTIAL EUROPEAN SEARCH REPORT

Application Number
EP 03 07 9183

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | DATABASE BEILSTEIN<br>BEILSTEIN INSTITUTE FOR ORGANIC CHEMISTRY, FRANKFURT-MAIN, DE;<br>XP002296936<br>Database accession no. 9571456,9570157<br>* abstract *<br>& A. V. GULEVSKAYA ET AL.: RUSS. CHEM. BL.,<br>vol. 52, no. 6, 2003, pages 1403-1410,<br>----- | 1-5 | |
| X | DATABASE BEILSTEIN<br>BEILSTEIN INSTITUTE FOR ORGANIC CHEMISTRY, FRANKFURT-MAIN, DE;<br>XP002296937<br>Database accession no. 7216143<br>* abstract *<br>& T. SUGIMOTO, W. PFLEIDERER: HETEROCYCLES,<br>vol. 41, no. 4, 1995, page 781-788,<br>----- | 1-5 | TECHNICAL FIELDS SEARCHED (Int.Cl.7) |
| X | DATABASE BEILSTEIN<br>BEILSTEIN INSTITUTE FOR ORGANIC CHEMISTRY, FRANKFURT-MAIN, DE;<br>XP002296938<br>Database accession no. 7928670<br>* abstract *<br>& S. MURATA ET AL..: HETEROCYCLES,<br>vol. 48, no. 6, 1998, page 1255-1274,<br>-----<br>-/-- | 1-5 | |

EPO FORM 1503 03.82 (P04C10)

European Patent Office

## PARTIAL EUROPEAN SEARCH REPORT

Application Number
EP 03 07 9183

| Category | Citation of document with indication, where appropriate, of relevant passages | Relevant to claim | CLASSIFICATION OF THE APPLICATION (Int.Cl.7) |
|---|---|---|---|
| D,X | H. B. COTTAM ET AL.: "Substituted Xanthines, Pteridinediones and Related Compounds as Potential Antiinflammatory Agents. Synthesis and Biological Evaluation of Inhibitors of Tumor Necrosis Factor .alpha." J. MED. CHEM., vol. 39, no. 1, 1996, pages 2-9, XP002296929 * table 1 * | 1-10 | |
| X | N. SATO, S. FUKUYA: "Studies on pyrazines. Part 37. Synthesis of 6-propionylpteridine-2,4(1H,3H)-dione and its 1- and/or 3-methyl derivatives from marine natural products" J. CHEM. SOC., PERKIN TRANS. 1, 2000, pages 89-95, XP002296930 Compounds of formula 9 and 11 | 1-5 | TECHNICAL FIELDS SEARCHED (Int.Cl.7) |
| X | S. MURATA ET AL.: "A facile method for regioselective 6,7-disubstitution of pteridine" HETEROCYCLES, vol. 53, no. 6, 2000, pages 1259-1262, XP002296931 * page 1260 * | 1-5 | |
| X | P. GIORI ET AL.: "Reactivity of 3H-Pyrimido[5,4-c][1,2,5]oxadiazin-3-one towards Carbanions: Synthesis of Pteridine-2,4-diones" J. HETEROCYCLIC CHEMISTRY, vol. 23, 1986, pages 1661-1665, XP002296932 * table 1 * | 1-5 | |

EPO FORM 1503 03.82 (P04C10)

**ANNEX TO THE EUROPEAN SEARCH REPORT ON EUROPEAN PATENT APPLICATION NO.** EP 03 07 9183

This annex lists the patent family members relating to the patent documents cited in the above-mentioned European search report.
The members are as contained in the European Patent Office EDP file on
The European Patent Office is in no way liable for these particulars which are merely given for the purpose of information.

20-09-2004

| Patent document cited in search report | Publication date | Patent family member(s) | Publication date |
|---|---|---|---|
| US 3071587 A | 01-01-1963 | NONE | |
| US 2003236255 A1 | 25-12-2003 | AU 2441800 A | 25-08-2000 |
| | | CA 2361561 A1 | 10-08-2000 |
| | | WO 0045800 A2 | 10-08-2000 |
| | | EP 1187615 A2 | 20-03-2002 |
| | | JP 2002536320 T | 29-10-2002 |
| WO 9406431 A | 31-03-1994 | US 5288721 A | 22-02-1994 |
| | | AU 5138493 A | 12-04-1994 |
| | | CA 2145192 A1 | 31-03-1994 |
| | | EP 0662834 A1 | 19-07-1995 |
| | | JP 8501564 T | 20-02-1996 |
| | | WO 9406431 A1 | 31-03-1994 |
| | | US 6121270 A | 19-09-2000 |
| | | US 5866576 A | 02-02-1999 |
| WO 0045800 A | 10-08-2000 | AU 2441800 A | 25-08-2000 |
| | | CA 2361561 A1 | 10-08-2000 |
| | | WO 0045800 A2 | 10-08-2000 |
| | | EP 1187615 A2 | 20-03-2002 |
| | | JP 2002536320 T | 29-10-2002 |
| | | US 2003236255 A1 | 25-12-2003 |
| EP 0290819 A | 17-11-1988 | IT 1204612 B | 10-03-1989 |
| | | AT 97808 T | 15-12-1993 |
| | | DE 3885933 D1 | 13-01-1994 |
| | | DE 3885933 T2 | 14-04-1994 |
| | | EP 0290819 A2 | 17-11-1988 |
| | | ES 2061543 T3 | 16-12-1994 |
| | | JP 2621925 B2 | 18-06-1997 |
| | | JP 63297326 A | 05-12-1988 |
| | | US 5047405 A | 10-09-1991 |
| WO 9411001 A | 26-05-1994 | US 5473070 A | 05-12-1995 |
| | | AU 5671294 A | 08-06-1994 |
| | | CA 2151617 A1 | 26-05-1994 |
| | | EP 0669825 A1 | 06-09-1995 |
| | | WO 9411001 A1 | 26-05-1994 |
| | | US 6693105 B1 | 17-02-2004 |
| | | US 5804584 A | 08-09-1998 |
| | | US 5780476 A | 14-07-1998 |
| | | US 6133274 A | 17-10-2000 |
| US 5843943 A | 01-12-1998 | AT 220549 T | 15-08-2002 |
| | | AU 717243 B2 | 23-03-2000 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 03 07 9183

This annex lists the patent family members relating to the patent documents cited in the above-mentioned European search report.
The members are as contained in the European Patent Office EDP file on
The European Patent Office is in no way liable for these particulars which are merely given for the purpose of information.

20-09-2004

| Patent document cited in search report | | Publication date | Patent family member(s) | | | Publication date |
|---|---|---|---|---|---|---|
| US 5843943 | A | | AU | 4601996 | A | 24-07-1996 |
| | | | CA | 2208580 | A1 | 11-07-1996 |
| | | | DE | 69527438 | D1 | 22-08-2002 |
| | | | DE | 69527438 | T2 | 06-03-2003 |
| | | | EP | 1193257 | A2 | 03-04-2002 |
| | | | EP | 0801568 | A1 | 22-10-1997 |
| | | | JP | 11502193 | T | 23-02-1999 |
| | | | WO | 9620710 | A1 | 11-07-1996 |
| | | | US | 6323201 | B1 | 27-11-2001 |
| | | | US | 2002165202 | A1 | 07-11-2002 |
| WO 9852948 | A | 26-11-1998 | US | 6323201 | B1 | 27-11-2001 |
| | | | AU | 747159 | B2 | 09-05-2002 |
| | | | AU | 7489698 | A | 11-12-1998 |
| | | | CA | 2287484 | A1 | 26-11-1998 |
| | | | EP | 0986561 | A1 | 22-03-2000 |
| | | | JP | 2001526684 | T | 18-12-2001 |
| | | | WO | 9852948 | A1 | 26-11-1998 |
| | | | US | 2002165202 | A1 | 07-11-2002 |

EPO FORM P0459

For more details about this annex : see Official Journal of the European Patent Office, No. 12/82